# EUROPEAN PATENT APPLICATION

(11) **EP 4 714 960 A1**
(43) Date of publication of application: **25.03.2026**
(21) Application number: 24807059.1
(22) Date of filing: 01.05.2024
(51) Int. Cl.: C07F 7/18, C07C 211/07, C07C 271/16, C07C 323/12, C09K 3/18

(54) **HYDROCARBON TERMINAL GROUP-CONTAINING COMPOUND, SURFACE TREATMENT AGENT, AND ARTICLE**

(30) Priority: 16.05.2023 JP 2023080539
(71) Applicant: SHIN-ETSU CHEMICAL CO., LTD., Tokyo 100-0005 (JP)
(72) Inventor: SAKOH Ryusuke, Annaka-shi, Gunma 379-0224 (JP); MORI Seiya, Annaka-shi, Gunma 379-0224 (JP)
(74) Representative: Ter Meer Steinmeister & Partner
(86) International application number: PCT/JP2024/016780
(87) International publication number: WO 2024/237105

(57) **Abstract**

A hydrocarbon terminal group-containing compound represented by formula (1) is capable of forming a cured film that is excellent in water repellency, slipperiness, ease of cleaning off dirt, wear resistance, and chemical resistance. 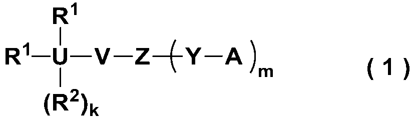 (R¹ is a C3 to C32 monovalent hydrocarbon group that may contain O, S, N, or Si; R² is H, a halogen atom, a hydroxyl group, a siloxy group, an amino group, a thiol group, a monovalent hydrocarbon group, R¹, or -Y-A; U is C, Si, N, or a trivalent or tetravalent organic group; V is a single bond or a divalent hydrocarbon group that may contain O, N, or S; Z is a single bond, C, Si, N, S, or a trivalent to octavalent organic group; Y is a divalent hydrocarbon group that may contain O, N, S, or Si; A is a monovalent reactive group; k is a value of 0 or 1; and m is a value of 1-7.)

## Description

### TECHNICAL FIELD

This invention relates to a hydrocarbon end group-containing compound; and more particularly, to a hydrocarbon end group-containing compound capable of forming a film having improved water repellency, wear resistance, fingerprint low visibility, and chemical resistance, specifically alkyl end group-containing compound; a surface treating agent comprising the compound; and an article surface-treated with the surface treating agent.

### BACKGROUND ART

Recently, the conversion of displays including smartphones and vehicle-mounted displays to touch panels is accelerated. Since touch panels having their screen exposed encounter many chances of coming into direct contact with fingers, cheeks or other parts, there arises the problem of susceptibility to stains like sebum. To improve the outer appearance and visibility, the demand for a technique of rendering the display surface unreceptive to fingerprints and a technique of rendering the display surface easy to remove stains is increasing every year. It is desired to develop the material capable of meeting these requirements. Especially, the surface of a touch panel display is receptive to fingerprint stains, it is desired to provide a water/oil repellent layer. Although the prior art water/oil repellent layer has high water/oil repellency and effective stain wipe-off, there are problems that wear resistance is insufficient and once fingerprints are deposited, the fingerprints look rather conspicuous.

In general, fluoropolyether-containing compounds have water/oil repellency, chemical resistance, lubricity, parting properties and antifouling properties because of extremely low surface free energy. For taking advantage of these properties, they are widely used as water/oil repellent antifouling agents for paper and textile, lubricants for magnetic recording media, oil-proof agents for precision machines, parting agents, cosmetics, and protective films. The same properties mean non-stickiness and non-adhesion to other substrates at the same time. Although they can be coated to the substrate surface, it is difficult to bond the coating tightly to the substrate.

Silane coupling agents are well known as the means for bonding organic compounds to the surface of substrates of glass, fabric and the like. They are widely utilized as the coating agent to the surface of various substrates. The silane coupling agent has an organic functional group and a reactive silyl group (typically, hydrolyzable silyl group such as alkoxysilyl group) in the molecule. The hydrolyzable silyl group undergoes self-condensation reaction by airborne moisture, to form a coating. The coating becomes a tough coating with durability through chemical and physical bonding of the hydrolyzable silyl group to the surface of glass or metal.

Then, Patent Documents 1 to 6 (JP-A 2008-534696, JP-A 2008-537557,, JP-A 2012-072272, JP-A 2012-157856, JP-A 2013-136833, and JP-A 2015-199906) disclose compositions comprising a fluoropolyether-containing polymer obtained by introducing a hydrolyzable silyl group into the fluoropolyether-containing compound, the compositions being able to form a coating which is tightly bonded to the substrate surface and has water/oil repellency, chemical resistance, lubricity, parting properties, and antifouling properties on the substrate surface.

In particular, the compound described in Patent Document 7 (WO 2017/212850) allegedly has excellent wear resistance. However, the drawback that fingerprints, if deposited, look conspicuous because of high water/oil repellency is pointed out.

Then Patent Document 8 (WO 2019/082583) proposes a fluorine group-free surface treating agent having restrained repellency of fingerprints. However, its wear resistance is insufficient to withstand a rigorous service environment.

Furthermore, since fluorine compounds are characterized by the difficulty of decomposition in the natural world and tend to accumulate in the natural world, it is demanded to develop a surface protective agent of non-fluorine base material.

### PRIOR ART DOCUMENTS

### PATENT DOCUMENTS

Patent Document 1: JP-A 2008-534696
Patent Document 2: JP-A 2008-537557
Patent Document 3: JP-A 2012-072272
Patent Document 4: JP-A 2012-157856
Patent Document 5: JP-A 2013-136833
Patent Document 6: JP-A 2015-199906
Patent Document 7: WO 2017/212850
Patent Document 8: WO 2019/082583

### SUMMARY OF INVENTION

### TECHNICAL PROBLEM

An object of the invention, which has been made under the above-mentioned circumstances, is to provide a non-fluorine based (i.e., not containing fluorine in the molecule) hydrocarbon end group-containing compound capable of forming a cured film having improved water repellency, slipperiness, stain wipe-off, wear resistance, and chemical resistance; a substantially non-fluorine based surface treating agent comprising the compound; and an article surface-treated with the surface treating agent.

### SOLUTION TO PROBLEM

Making extensive investigations to attain the above object, the inventors have found that when a hydrocarbon end group-containing compound having the general formula (1) is used, a surface treating agent comprising the compound can form a cured film having water repellency, slipperiness, stain wipe-off, wear resistance, especially steel wool wear resistance, and chemical resistance. The invention is predicated on this finding.

Accordingly, the invention provides a hydrocarbon end group-containing compound, surface treating agent, and an article as defined below.
[1] A hydrocarbon end group-containing compound having the general formula (1): 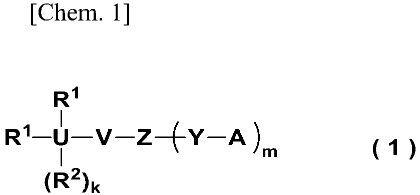 wherein R¹ is independently a straight, branched and/or cyclic C₃-C₃₂ monovalent hydrocarbon group which may contain at least one element selected from oxygen, sulfur, nitrogen and silicon, R² is hydrogen, halogen, hydroxy, siloxy, amino, thiol, monovalent hydrocarbon group of 1 or 2 carbon atoms, R¹ or -Y-A, U is carbon, silicon, nitrogen or tri- or tetravalent organic group, V is a single bond or a divalent hydrocarbon group which may contain at least one element selected from oxygen, nitrogen and sulfur, Z is a single bond, carbon, silicon, nitrogen, sulfur or a trivalent to octavalent organic group, Y is independently a divalent hydrocarbon group which may contain at least one element selected from oxygen, nitrogen, sulfur and silicon, A is independently a monovalent reactive group, k is 0 or 1, and m is an integer of 1 to 7.
[2] The hydrocarbon end group-containing compound of [1] wherein A in formula (1) is a group having the general formula (2): 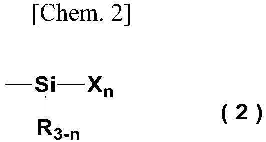
   wherein R is independently a C₁-C₄ alkyl group or phenyl group, X is independently a hydroxy or hydrolyzable group, and n is an integer of 1 to 3, or the general formula (3): 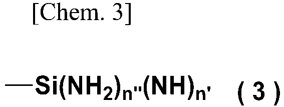
   wherein n" is a number of 0 to 3, and n' is (3-n")/2.
[3] The hydrocarbon end group-containing compound of [1] or [2] wherein R¹ in formula (1) is a group having any one of the following formulae: 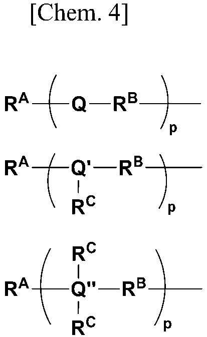 wherein R^{A} is independently a straight, branched and/or cyclic C₃-C₃₂ monovalent hydrocarbon group,
   Q is independently a divalent group selected from the class consisting of oxygen, sulfur, C₆-C₈ divalent cyclic hydrocarbon group, diorganosilylene group, silalkylene structure or silarylene structure, a straight divalent organopolysiloxane residue of 2 to 10 silicon atoms, branched or cyclic divalent organopolysiloxane residue of 3 to 10 silicon atoms, carbonyl (or ketone) group, ester group, carbonate group, sulfinyl group, sulfonyl group, thioester group, thiocarbonate group, thiocarbamate group, amino group, amide group, carbamate group, urea group, and divalent nitrogen-containing heterocyclic group,
   Q' is independently a trivalent group selected from the class consisting of nitrogen, C₆-C₈ trivalent cyclic hydrocarbon group, straight trivalent organopolysiloxane residue of 2 to 10 silicon atoms, branched or cyclic trivalent organopolysiloxane residue of 3 to 10 silicon atoms, trivalent amide group, and trivalent nitrogen-containing heterocyclic group,
   Q" is independently a tetravalent group selected from the class consisting of carbon, silicon, C₆-C₈ tetravalent cyclic hydrocarbon group, straight tetravalent organopolysiloxane residue of 2 to 10 silicon atoms, and branched or cyclic tetravalent organopolysiloxane residue of 3 to 10 silicon atoms,
   R⁸ is independently a single bond or a C₁-C₃₂ straight, branched or cyclic divalent hydrocarbon group,
   R^{C} is independently R^{A} or hydrogen,
   p is an integer of 0 to 10, and
   the total number of carbon atoms in the structures is up to 32.
[4] The hydrocarbon end group-containing compound of any one of [1] to [3] wherein Y in formula (1) is a group selected from the class consisting of a C₁-C₂₀ alkylene group which may contain at least one element selected from oxygen, nitrogen and sulfur, a C₁-C₁₀ alkylene group containing a C₆-C₈ arylene moiety, a divalent group having C₁-C₈ alkylene groups bonded via a diorganosilylene moiety, silalkylene structure, silarylene structure or nitrogen-containing heterocyclic moiety, and a divalent group having a C₁-C₁₀ alkylene moiety bonded to the valence bond of a straight organopolysiloxane residue of 2 to 10 silicon atoms, or branched or cyclic organopolysiloxane residue of 3 to 10 silicon atoms.
[5] The hydrocarbon end group-containing compound of any one of [1] to [4] wherein Z in formula (1) is a single bond, or a trivalent to octavalent group selected from the class consisting of carbon, silicon, nitrogen, C₆-C₈ tri- or tetravalent cyclic hydrocarbon group, a trivalent group: -SiR³= wherein R³ is hydroxy, C₁-C₃ alkyl moiety or C₁-C₃ alkoxy moiety, a trivalent group: -CR⁴= wherein R⁴ is hydrogen, hydroxy, or C₁-C₃ alkyl moiety, a straight trivalent to octavalent organopolysiloxane residue of 2 to 10 silicon atoms, branched or cyclic trivalent to octavalent organopolysiloxane residue of 3 to 10 silicon atoms, trivalent amide group, trivalent carbamate group, tri- or tetravalent urea group, and trivalent to octavalent nitrogen-containing heterocyclic group.
[6] The hydrocarbon end group-containing compound of any one of [1] to [5] wherein U in formula (1) is a tri- or tetravalent group selected from the class consisting of carbon, silicon, nitrogen, a C₆-C₈ tri- or tetravalent cyclic hydrocarbon group, straight tri- or tetravalent organopolysiloxane residue of 2 to 10 silicon atoms, branched or cyclic tri- or tetravalent organopolysiloxane residue of 3 to 10 silicon atoms, trivalent amide group, trivalent carbamate group, tri- or tetravalent urea group, and tri- or tetravalent nitrogen-containing heterocyclic group.
[7] The hydrocarbon end group-containing compound of any one of [2] to [6] wherein X in formula (2) is selected from the class consisting of hydroxy, C₁-C₁₀ alkoxy group, C₂-C₁₀ alkoxyalkoxy group, C₁-C₁₀ acyloxy group, C₂-C₁₀ alkenyloxy group, halogen, and C₂-C₁₀ dialkylamino group.
[8] A surface treating agent comprising the hydrocarbon end group-containing compound of any one of [1] to [7].
[9] An article treated on its surface with the surface treating agent of [8].

### ADVANTAGEOUS EFFECTS OF INVENTION

The hydrocarbon end group-containing compound has at least two hydrocarbon chains of a predetermined number of carbon atoms at the end of the molecular chain so that the mobility of the molecular chain is improved. Then an article treated on its surface with a surface treating agent comprising the compound has improved water repellency, slipperiness, stain wipe-off, and wear resistance as well as chemical resistance.

### DESCRIPTION OF EMBODIMENTS

The invention provides a hydrocarbon end group-containing compound having the general formula (1).

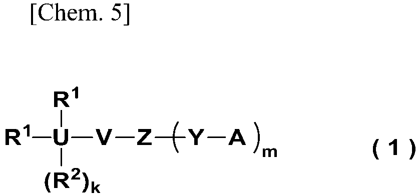

Herein R¹ is independently a straight, branched and/or cyclic C₃-C₃₂ monovalent hydrocarbon group which may contain at least one element selected from oxygen, sulfur, nitrogen and silicon, R² is hydrogen, halogen, hydroxy, siloxy, amino, thiol, monovalent hydrocarbon group of 1 or 2 carbon atoms, R¹ or -Y-A, U is carbon, silicon, nitrogen or tri- or tetravalent organic group, V is a single bond or a divalent hydrocarbon group which may contain at least one element selected from oxygen, nitrogen and sulfur, Z is a single bond, carbon, silicon, nitrogen, sulfur or a trivalent to octavalent organic group, Y is independently a divalent hydrocarbon group which may contain at least one element selected from oxygen, nitrogen, sulfur and silicon, A is independently a monovalent reactive group, k is 0 or 1, and m is an integer of 1 to 7.

The hydrocarbon end group-containing compound has at least two hydrocarbon end groups of a predetermined number of carbon atoms, specifically at least two groups selected from C₃-C₃₂, preferably C₆-C₂₈ alkyl groups, and C₆-C₃₂ aryl groups at one end, and a reactive group capable of providing substrate adhesion at the other end, which are linked via a linking group. Since at least two hydrocarbon end groups of a predetermined number of carbon atoms, specifically at least two groups selected from C₃-C₃₂, preferably C₆-C₂₈ alkyl groups, and C₆-C₃₂ aryl groups are at one end, the compound has enhanced mobility at the molecular chain end. A cured film of a surface treating agent comprising the compound exhibits excellent water repellency, slipperiness, stain wipe-off, wear resistance, and chemical resistance.

In formula (1), R¹ is independently a straight, branched and/or cyclic C₃-C₃₂, preferably C₆-C₂₈, more preferably C₈-C₂₈ monovalent hydrocarbon group which may contain at least one element selected from oxygen, sulfur, nitrogen and silicon.

When at least one element selected from oxygen, sulfur, nitrogen and silicon is contained, it preferably takes the form of an ether, carbonyl (or ketone), ester, carbonate, thioether, sulfinyl, sulfonyl, thioester, thiocarbonate, thiocarbamate, amino, amide, carbamate, urea, oxazole, imidazole, triazole, cyanurate, isocyanurate, diorganosilylene, organopolysiloxane, silalkylene, and silarylene group.

Groups having the following formulae are preferred as R¹.

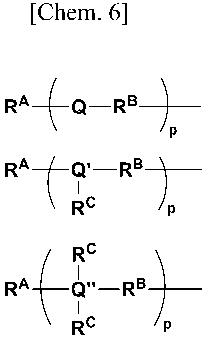

Herein R^{A} is independently a straight, branched and/or cyclic C₃-C₃₂ monovalent hydrocarbon group. Q is independently a divalent group selected from the class consisting of oxygen, sulfur, C₆-C₈ divalent cyclic hydrocarbon group, diorganosilylene group, silalkylene structure or silarylene structure, straight divalent organopolysiloxane residue of 2 to 10 silicon atoms, branched or cyclic divalent organopolysiloxane residue of 3 to 10 silicon atoms, carbonyl (or ketone) group, ester group, carbonate group, sulfinyl group, sulfonyl group, thioester group, thiocarbonate group, thiocarbamate group, amino group, amide group, carbamate group, urea group, and divalent nitrogen-containing heterocyclic group (e.g., divalent oxazole, divalent imidazole or divalent triazole group). Q' is independently a trivalent group selected from the class consisting of nitrogen, C₆-C₈ trivalent cyclic hydrocarbon group, straight trivalent organopolysiloxane residue of 2 to 10 silicon atoms, branched or cyclic trivalent organopolysiloxane residue of 3 to 10 silicon atoms, trivalent amide group, and trivalent nitrogen-containing heterocyclic group (e.g., trivalent cyanurate, trivalent isocyanurate or trivalent triazole group). Q" is independently a tetravalent group selected from the class consisting of carbon, silicon, C₆-C₈ tetravalent cyclic hydrocarbon group, straight tetravalent organopolysiloxane residue of 2 to 10 silicon atoms, and branched or cyclic tetravalent organopolysiloxane residue of 3 to 10 silicon atoms. R⁸ is independently a single bond or a C₁-C₃₂ divalent hydrocarbon group which may be straight, branched or cyclic. R^{C} is independently R^{A} or hydrogen, and p is an integer of 0 to 10. The total number of carbon atoms in the structures is up to 32.

In the above formulae, R^{A} is independently a straight, branched and/or cyclic C₃-C₃₂, preferably C₆-C₂₈, more preferably C₈-C₂₈ monovalent hydrocarbon group. Exemplary of the group R^{A} are those shown below.

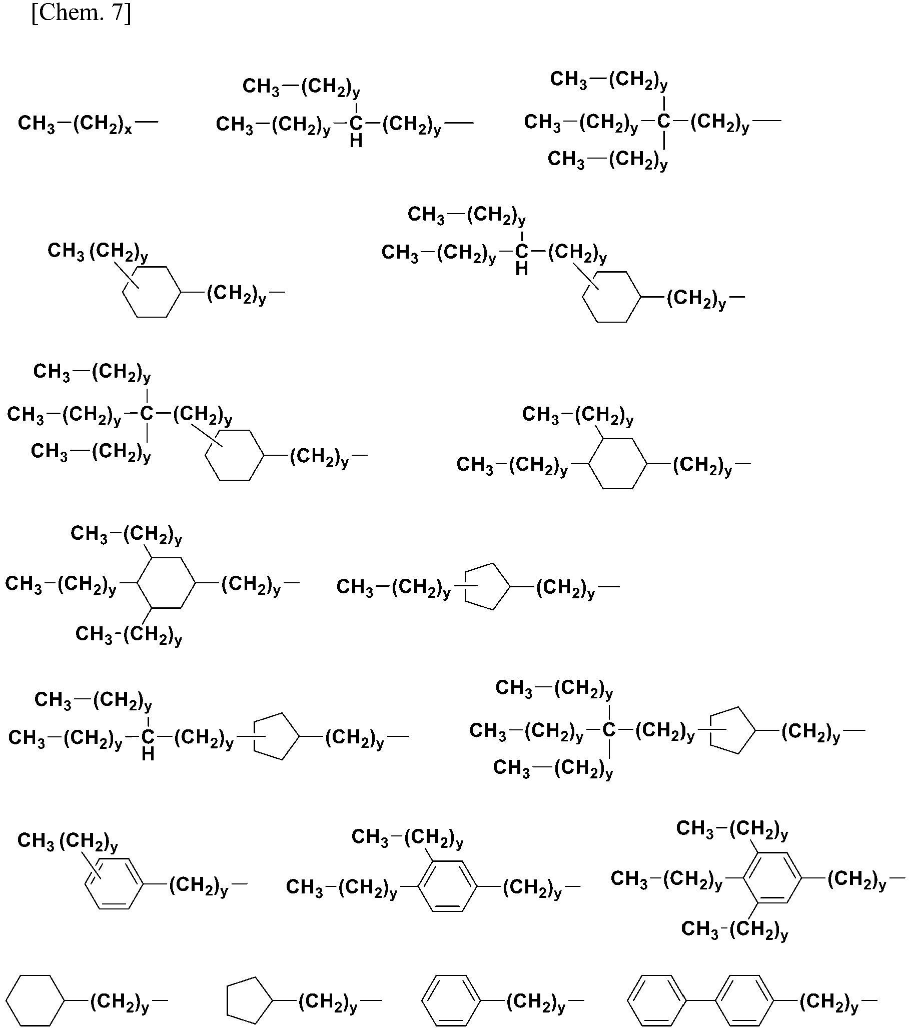

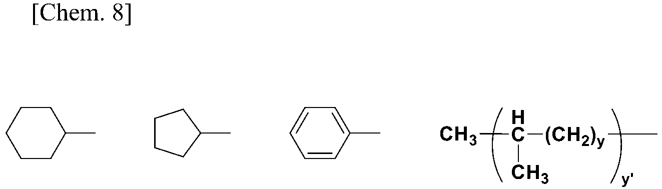

Herein x is an integer of 2 to 31, preferably 5 to 27, more preferably 7 to 27, y and y' each are an integer of at least 1, such that the total number of carbon atoms in the structures is up to 32.

In the above formulae, Q is independently a divalent group selected from the class consisting of oxygen, sulfur, C₆-C₈ divalent cyclic hydrocarbon group, diorganosilylene group, silalkylene structure or silarylene structure, straight divalent organopolysiloxane residue of 2 to 10, especially 2 to 8 silicon atoms, branched or cyclic divalent organopolysiloxane residue of 3 to 10, especially 3 to 8 silicon atoms, carbonyl (or ketone) group, ester group, carbonate group, sulfinyl group, sulfonyl group, thioester group, thiocarbonate group, thiocarbamate group, amino group, amide group, carbamate group, urea group, and divalent nitrogen-containing heterocyclic group (e.g., divalent oxazole, divalent imidazole or divalent triazole group).

In the diorganosilylene group, silalkylene structure, silarylene structure, and organopolysiloxane residue, the silicon-bonded group is preferably a C₁-C₈, more preferably C₁-C₄ alkyl group such as methyl, ethyl, propyl or butyl, or phenyl group. The alkylene moiety in the silalkylene structure is preferably a C₂-C₆, more preferably C₂-C₄ alkylene moiety such as ethylene, propylene (i.e., trimethylene or methylethylene), or butylene (i.e., tetramethylene or methylpropylene). The organopolysiloxane residue may contain a silalkylene structure having two silicon atoms linked by an alkylene moiety such as ethylene or propylene.

Exemplary of Q are those shown below. In the structure shown below, the valence bond on the left is attached to R^{A} or R^{B} and the valence bond on the right is attached to R^{B}.

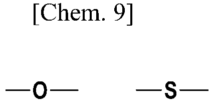

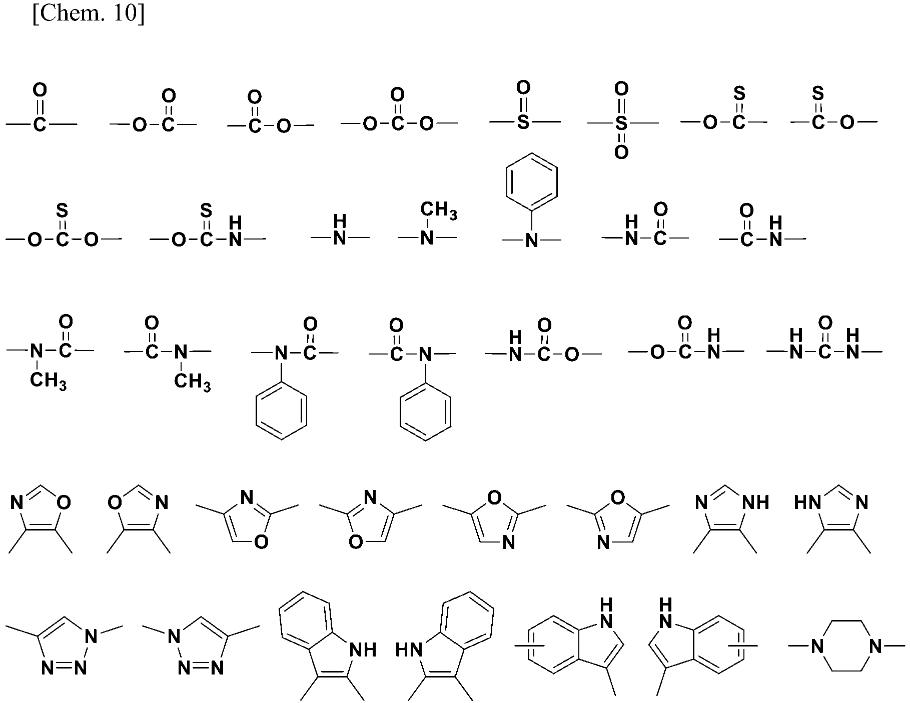

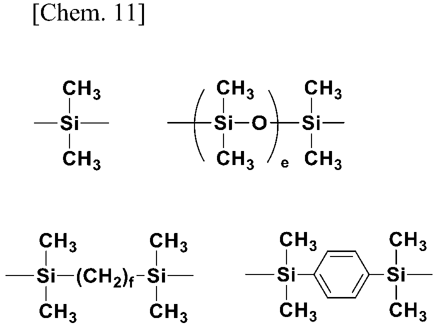

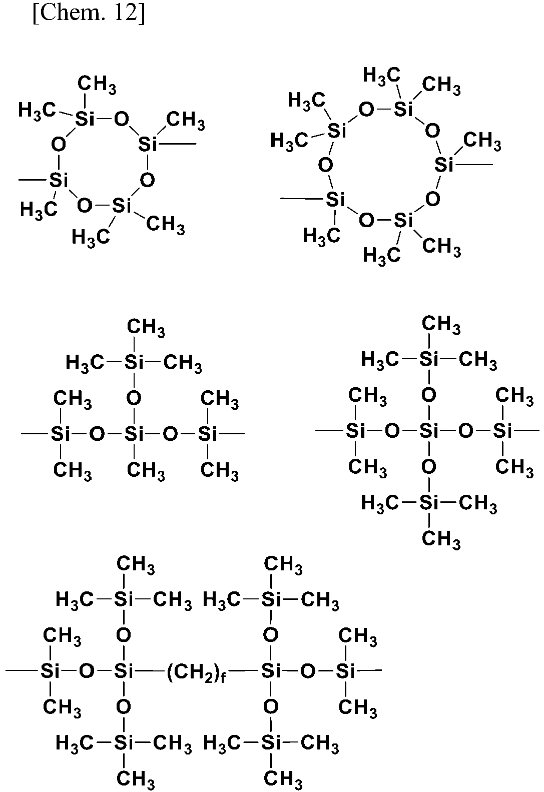

Herein f is an integer of 2 to 4, and e is an integer of 1 to 9.

In the above formulae, Q' is independently a trivalent group selected from the class consisting of nitrogen, C₆-C₈ trivalent cyclic hydrocarbon group, straight trivalent organopolysiloxane residue of 2 to 10, especially 2 to 8 silicon atoms, branched or cyclic trivalent organopolysiloxane residue of 3 to 10, especially 3 to 8 silicon atoms, trivalent amide group, and trivalent nitrogen-containing heterocyclic group (e.g., trivalent cyanurate, trivalent isocyanurate or trivalent triazole group).

The organopolysiloxane residue preferably contains a C₁-C₈, more preferably C₁-C₄ alkyl group such as methyl, ethyl, propyl or butyl, or phenyl group. The organopolysiloxane residue may contain a silalkylene structure having two silicon atoms linked by an alkylene moiety such as ethylene or propylene.

Exemplary of Q' are those shown below. In the structure shown below, the valence bond on the left is attached to R^{A} or R^{B}, the valence bond on the right is attached to R^{B}, and another valence bond is attached to R^{C}.

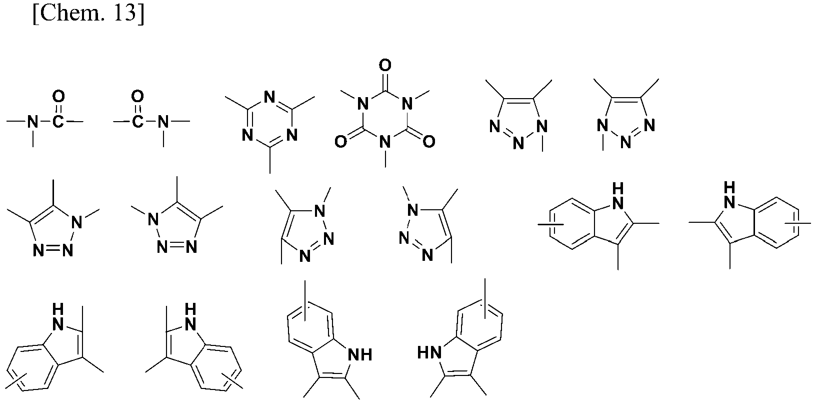

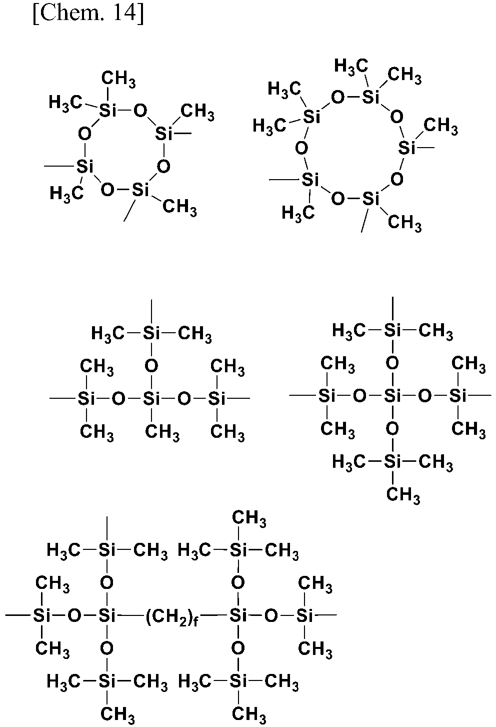

Herein f is an integer of 2 to 4.

In the above formulae, Q" is independently a tetravalent group selected from the class consisting of carbon, silicon, C₆-C₈ tetravalent cyclic hydrocarbon group, straight tetravalent organopolysiloxane residue of 2 to 10, especially 2 to 8 silicon atoms, and branched or cyclic tetravalent organopolysiloxane residue of 3 to 10, especially 3 to 8 silicon atoms.

The organopolysiloxane residue preferably contains a C₁-C₈, more preferably C₁-C₄ alkyl group such as methyl, ethyl, propyl or butyl, or phenyl group. The organopolysiloxane residue may contain a silalkylene structure having two silicon atoms linked by an alkylene moiety such as ethylene or propylene.

Exemplary of Q" are those shown below. In the structure shown below, the valence bond on the left is attached to R^{A} or R^{B}, the valence bond on the right is attached to R^{B}, and another valence bond is attached to R^{C}.

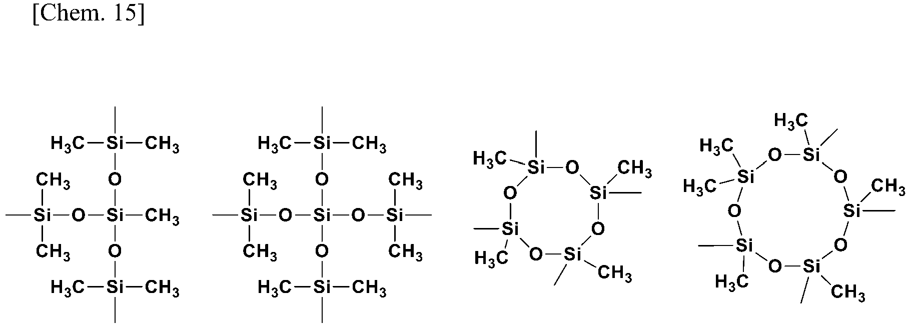

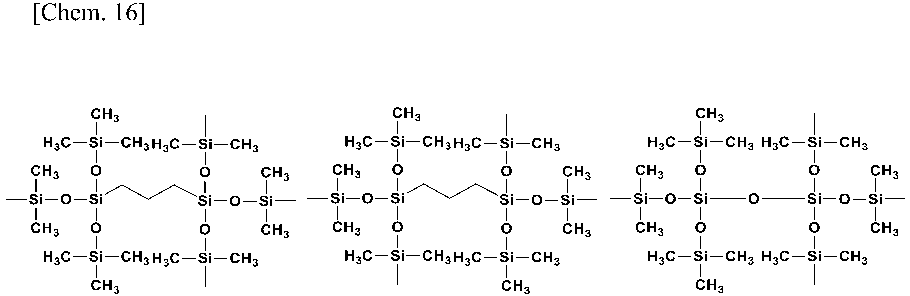

In the above formulae, R⁸ is independently a single bond or a C₁-C₃₂ divalent hydrocarbon group which may be straight, branched or cyclic. Exemplary of the hydrocarbon group are groups of the following formula.

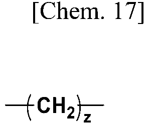

Herein z is an integer of 1 to 10.

In the above formulae, R^{C} is independently R^{A} or hydrogen. When R^{C} is R^{A}, R^{C} may be identical with or different from R^{A}.

In the above formulae, p is an integer of 0 to 10, preferably 0, 1 or 2. The total number of carbon atoms in the structures in R¹ is up to 32.

The following groups are preferred as R¹.

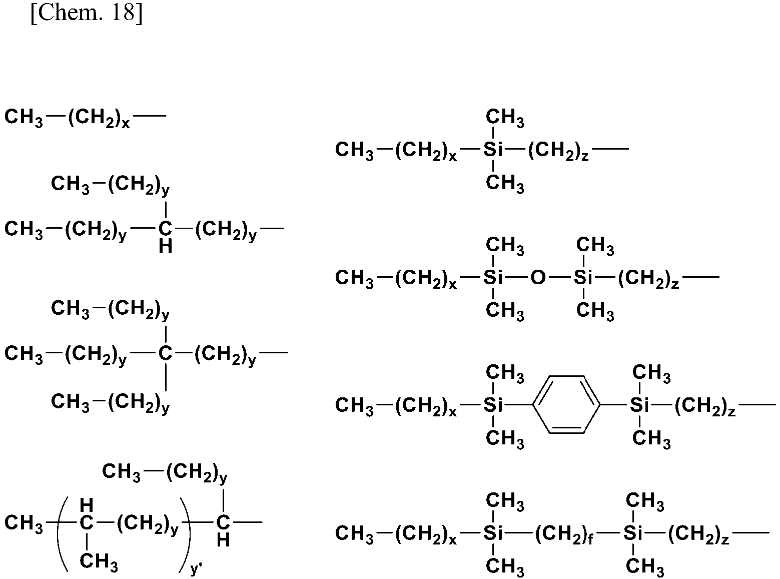

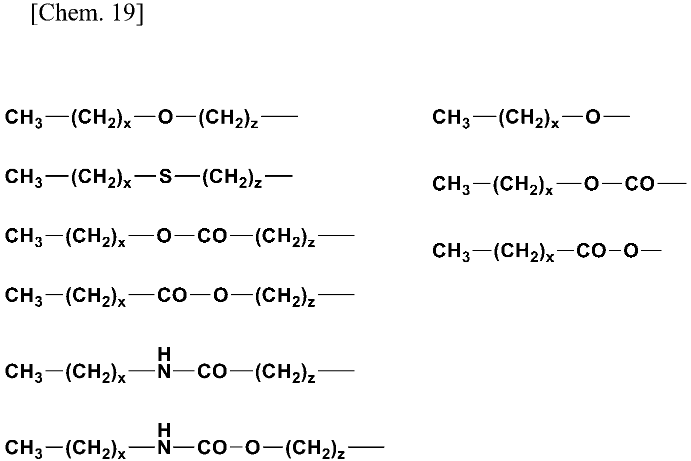

Herein x, y and z are as defined above, with the proviso that the total number of carbon atoms in the structures is 3 to 32.

In formula (1), R² is hydrogen, halogen, hydroxy, siloxy, amino, thiol, monovalent hydrocarbon group of 1 or 2 carbon atoms (i.e., methyl or ethyl), R¹ or -Y-A. R² is preferably hydrogen, chlorine, hydroxy, methyl, ethyl, R¹ or -Y-A.

In formula (1), U is carbon, silicon, nitrogen or tri- or tetravalent organic group. The tri- or tetravalent organic group is preferably a tri- or tetravalent group selected from C₆-C₈ tri- or tetravalent cyclic hydrocarbon groups, straight tri- or tetravalent organopolysiloxane residues of 2 to 10, especially 2 to 8 silicon atoms, and branched or cyclic tri- or tetravalent organopolysiloxane residues of 3 to 10, especially 3 to 8 silicon atoms, trivalent amide groups, trivalent carbamate groups, tri- or tetravalent urea groups, and tri- or tetravalent nitrogen-containing heterocyclic groups (e.g., trivalent cyanurate groups, trivalent isocyanurate groups, and trivalent triazine ring-containing groups).

The organopolysiloxane residue preferably contains a C₁-C₈, more preferably C₁-C₄ alkyl group such as methyl, ethyl, propyl or butyl, or phenyl group. The organopolysiloxane residue may contain a silalkylene structure having two silicon atoms linked by an alkylene moiety such as ethylene or propylene.

Exemplary of U are those shown below. In the structure shown below, the valence bond on the right is preferably attached to V.

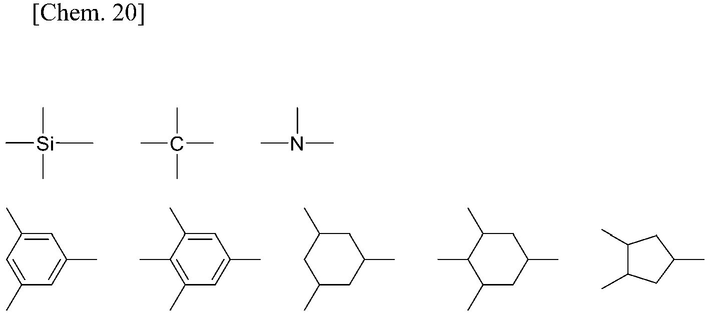

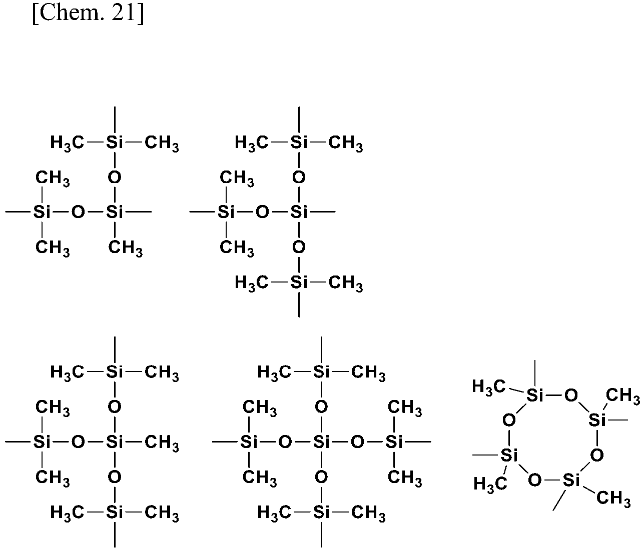

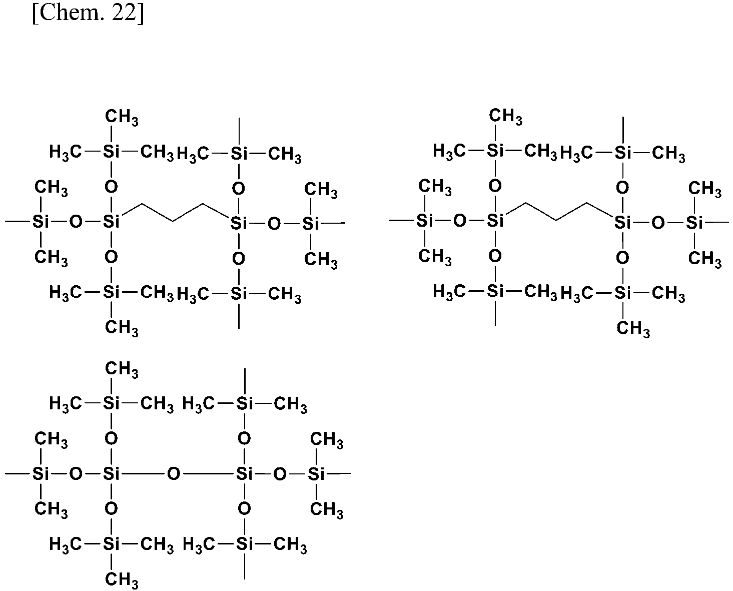

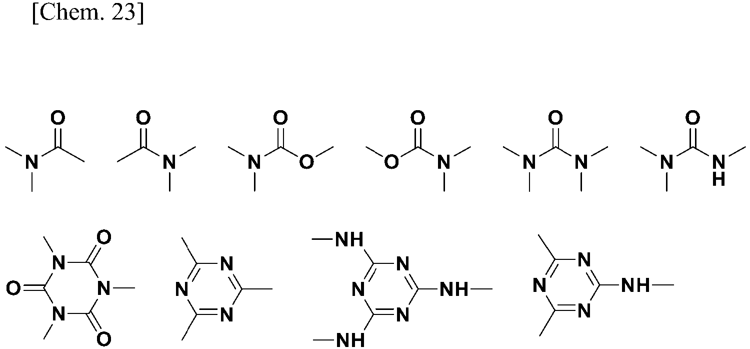

In formula (1), V is a single bond or a divalent hydrocarbon group, preferably of 1 to 20 carbon atoms, which may contain at least one element selected from oxygen, nitrogen and sulfur. It is a group for linking U and Z groups. When Z is a single bond, V is preferably a single bond.

Examples of the divalent hydrocarbon group include C₁-C₁₀ alkylene groups which may contain at least one element selected from oxygen, nitrogen and sulfur, and C₁-C₁₀ alkylene groups containing a C₆-C₈ arylene group, specifically C₇-C₁₈ alkylene-arylene groups.

Besides a single bond, those shown below are exemplary of V. In the structure shown below, the valence bond on the left is attached to U and the valence bond on the right is attached to Z.

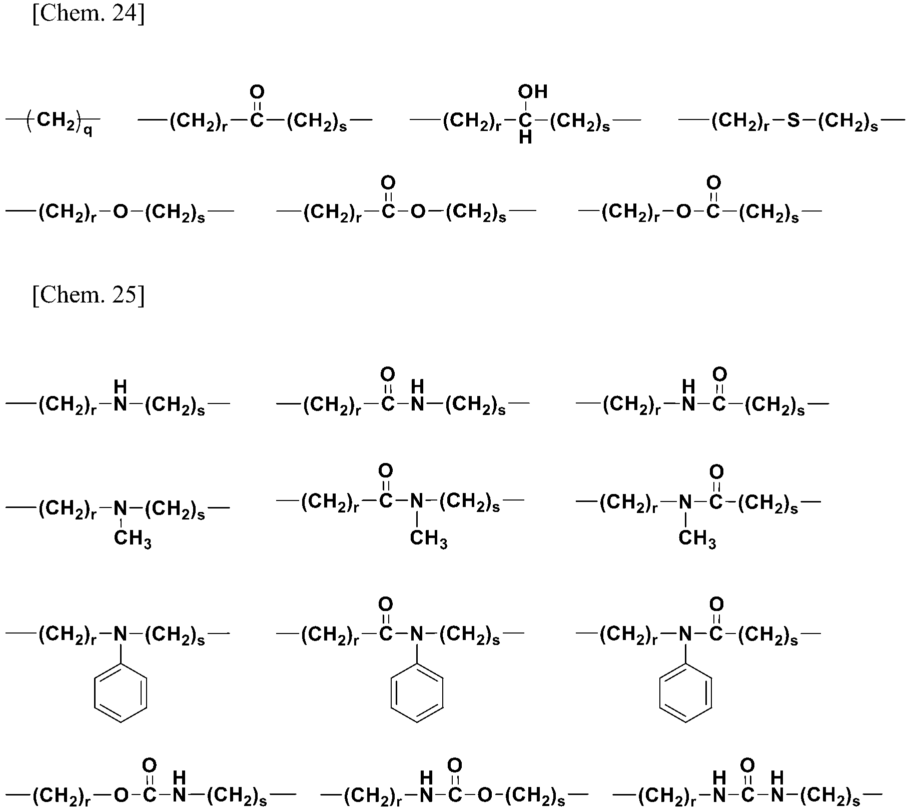

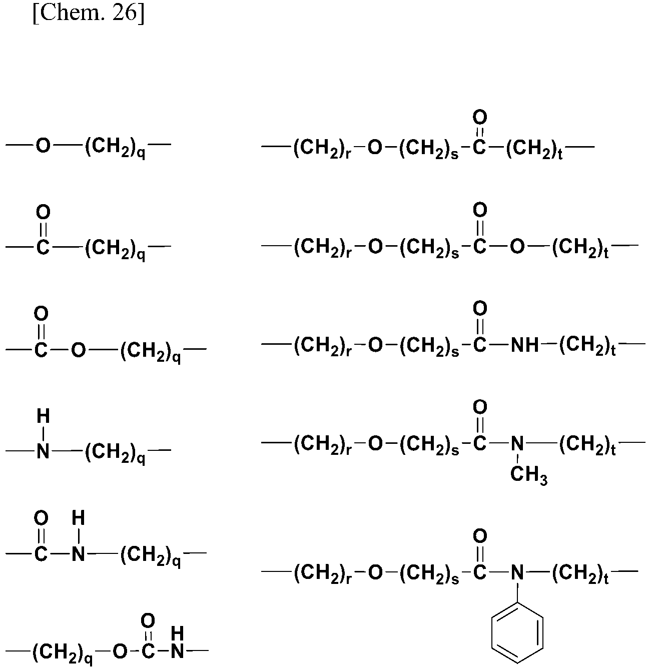

Herein q is an integer of 1 to 10, r, s and t each are an integer of 1 to 8, the sum of r and s is an integer of 2 to 10, and the sum of r, s and t is an integer of 3 to 10.

In formula (1), Z is a single bond, carbon, silicon, nitrogen, sulfur or a trivalent to octavalent organic group. Examples of the trivalent to octavalent organic group include C₆-C₈ tri- or tetravalent cyclic hydrocarbon groups, trivalent groups of the formula: -SiR³= wherein R³ is hydroxy, C₁-C₃ alkyl moiety or C₁-C₃ alkoxy moiety, trivalent groups of the formula: -CR⁴= wherein R⁴ is hydrogen, hydroxy, or C₁-C₃ alkyl moiety, straight trivalent to octavalent organopolysiloxane residues of 2 to 10, especially 2 to 8 silicon atoms, branched or cyclic trivalent to octavalent organopolysiloxane residues of 3 to 10, especially 3 to 8 silicon atoms, trivalent amide groups, trivalent carbamate groups, tri- or tetravalent urea groups, and trivalent to octavalent nitrogen-containing heterocyclic groups (e.g., trivalent cyanurate groups, trivalent isocyanurate groups and tri- or tetravalent triazine ring-containing groups).

The organopolysiloxane residue preferably contains a C₁-C₈, more preferably C₁-C₄ alkyl group such as methyl, ethyl, propyl or butyl, or phenyl group. The organopolysiloxane residue may contain a silalkylene structure having two silicon atoms linked by an alkylene moiety such as ethylene or propylene.

Besides a single bond, those shown below are exemplary of Z. In the structure shown below, the valence bond on the left is attached to V and another valence bond(s) is attached to Y.

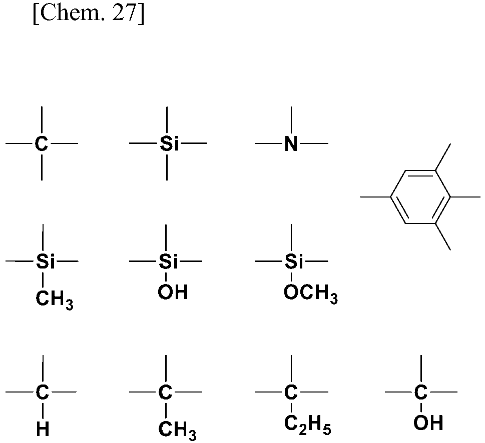

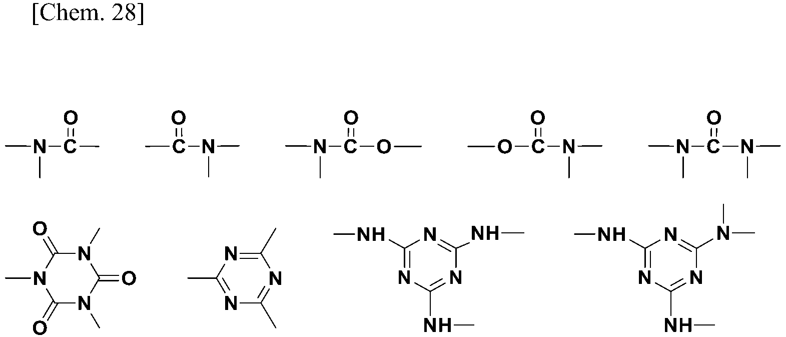

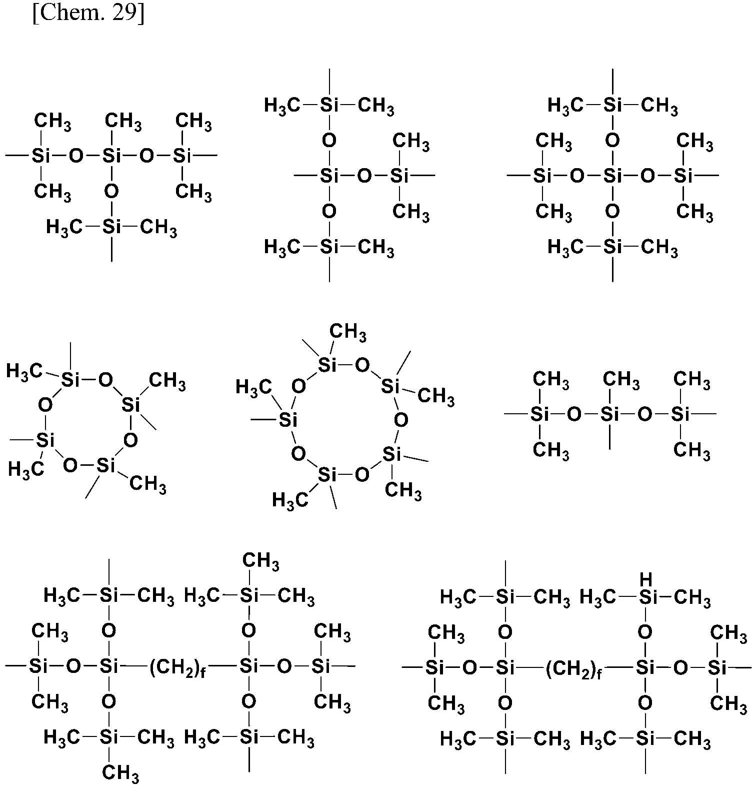

Herein f is an integer of 2 to 4.

In formula (1), Y is independently a divalent hydrocarbon group, preferably of 1 to 20 carbon atoms, which may contain at least one element selected from oxygen, nitrogen, sulfur and silicon. It is a group for linking groups Z and A. Examples of the divalent hydrocarbon group include a C₁-C₂₀, preferably C₁-C₁₀ alkylene group which may contain at least one element selected from oxygen, nitrogen and sulfur, a C₁-C₁₀ alkylene group containing a C₆-C₈ arylene moiety (specifically C₇-C₁₈ alkylene-arylene group), a divalent group having C₁-C₈ alkylene groups bonded via a diorganosilylene moiety, silalkylene structure, silarylene structure or nitrogen-containing heterocyclic moiety, and a divalent group having a C₁-C₁₀ alkylene group bonded to the valence bond of a straight organopolysiloxane residue of 2 to 10, especially 2 to 8 silicon atoms, or branched or cyclic organopolysiloxane residue of 3 to 10, especially 3 to 8 silicon atoms.

In the diorganosilylene group, silalkylene structure, silarylene structure, and organopolysiloxane residue, the silicon-bonded group is preferably a C₁-C₈, more preferably C₁-C₄ alkyl group such as methyl, ethyl, propyl or butyl, or phenyl group. The alkylene moiety in the silalkylene structure is preferably a C₂-C₆, more preferably C₂-C₄ alkylene moiety such as ethylene, propylene (i.e., trimethylene or methylethylene), or butylene (i.e., tetramethylene or methylpropylene). The organopolysiloxane residue may contain a silalkylene structure having two silicon atoms linked by an alkylene moiety such as ethylene or propylene.

Exemplary of Y are those shown below. In the structure shown below, the valence bond on the left is attached to Z and the valence bond on the right is attached to A.

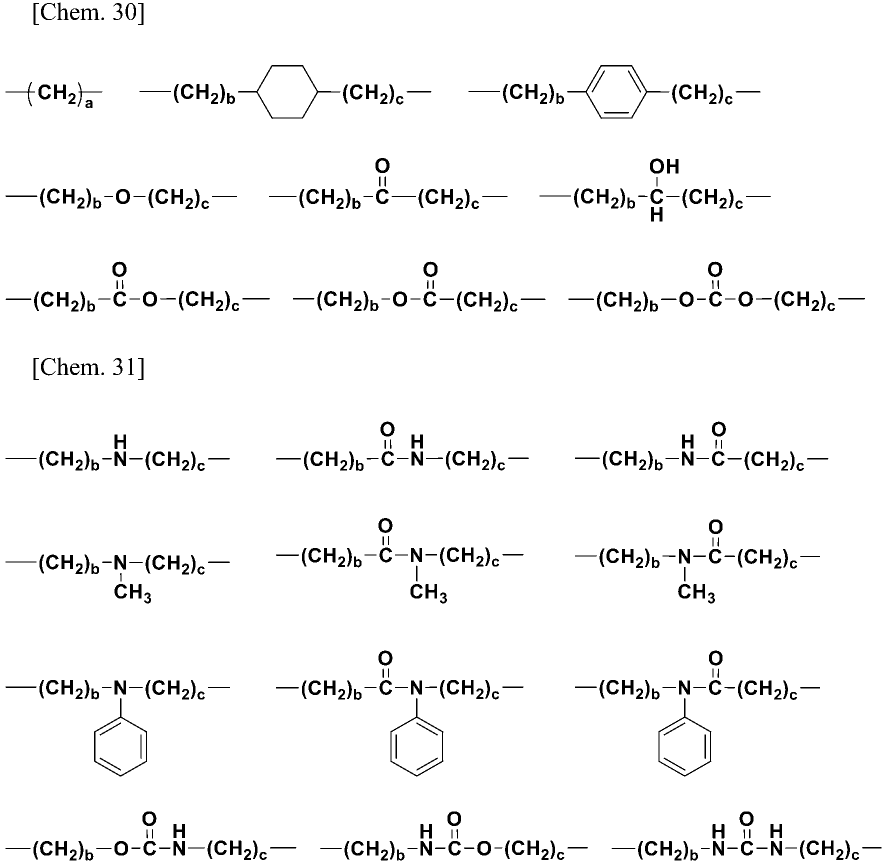

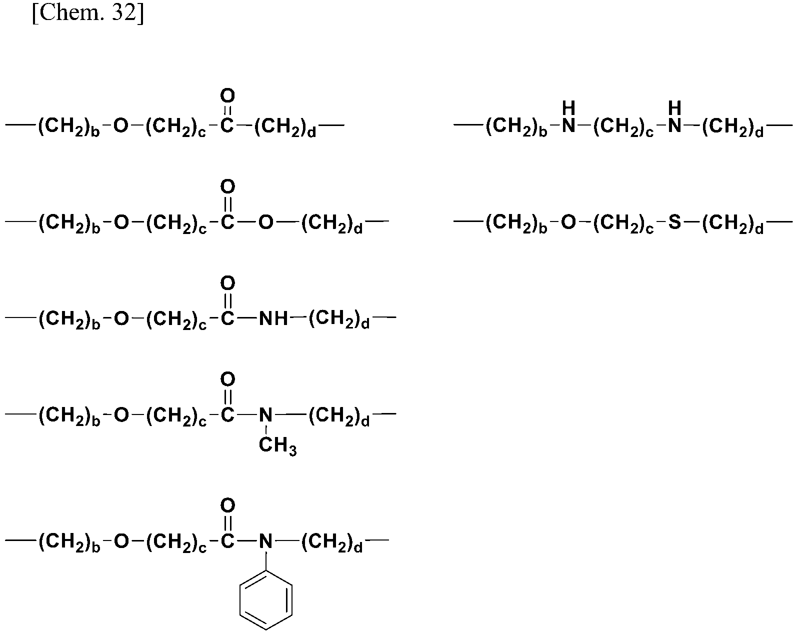

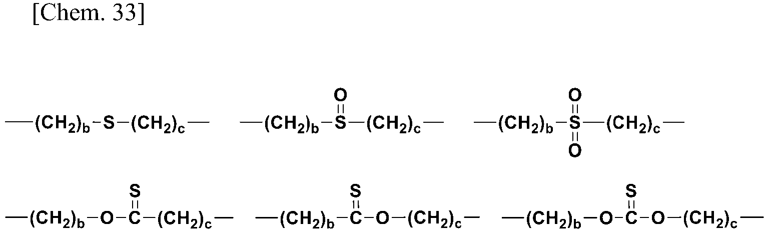

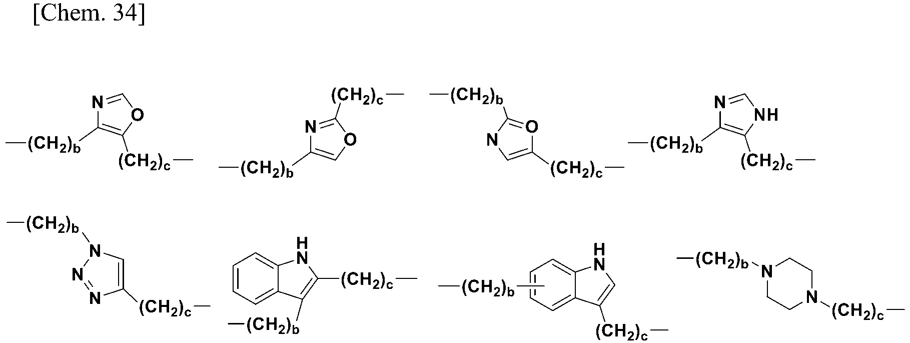

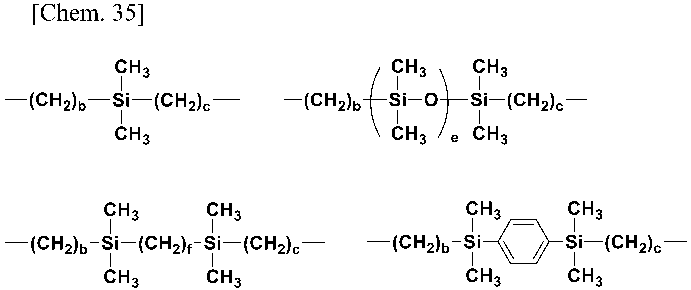

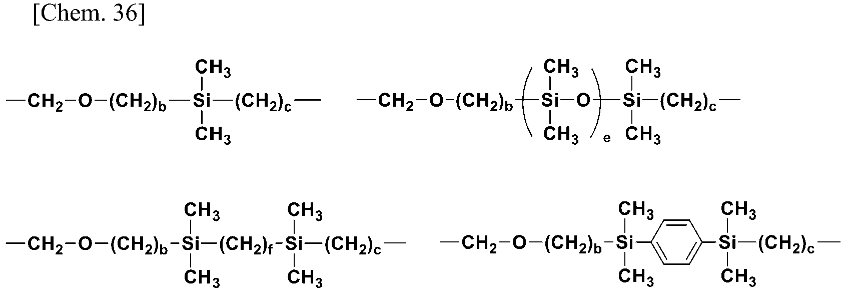

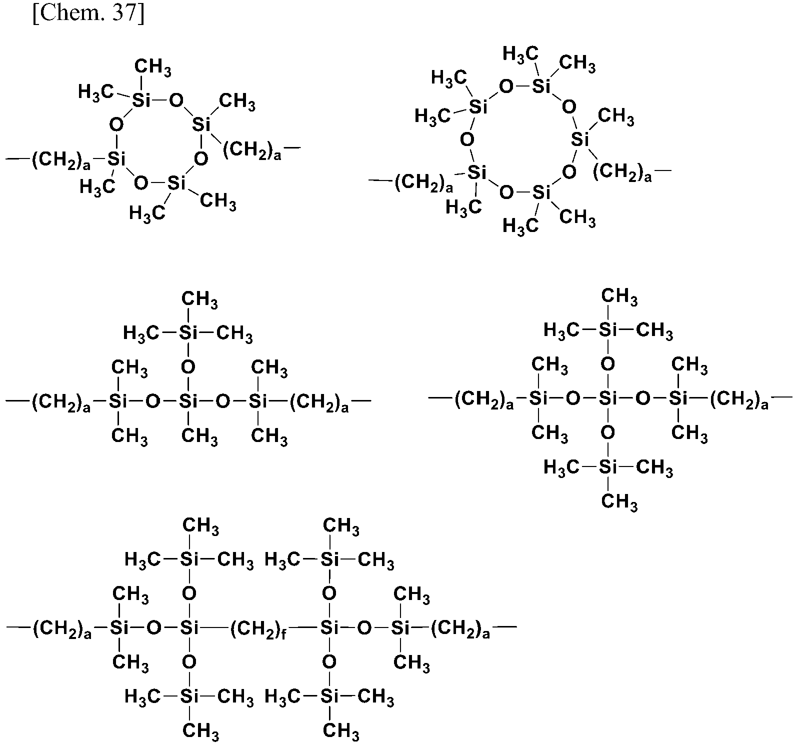

Herein a is independently an integer of 1 to 10, b, c and d each are an integer of 1 to 8, the sum of b and c is an integer of 2 to 10, the sum of b, c and d is an integer of 3 to 10, e is an integer of 1 to 9, and f is an integer of 2 to 4.

In formula (1), A is independently a monovalent reactive group, preferably a functional group which is adhesion-reactive (or bond-reactive) to the surface of substrates (to be surface-treated) of various materials including paper, fabric, metals and oxides thereof, glass, plastics or resins, ceramics and quartz. Examples of the monovalent reactive group include carbon-carbon double bond-containing groups (exclusive of alkenyl groups, i.e., groups capable of hydrosilation addition polymerization, limited to groups pertaining to ionic addition polymerization or curing reaction with the aid of actinic energy radiation or light), carbon-carbon triple bond-containing groups, cyclic ether groups, hydroxy-containing groups (exclusive of hydroxy alone), thiol groups, amino groups, azide groups, nitrogen-containing heterocyclic groups, phosphate-containing groups, hydroxy-containing silyl groups (silanol groups), and hydrolyzable silyl groups.

Exemplary of A are monovalent groups, which include carbon-carbon double bond-containing groups, for example, cinnamate, sorbate, acryloyl, methacryloyl, acryloyloxy, methacryloyloxy, acrylamide, methacrylamide, and vinyl ether; carbon-carbon triple bond-containing groups, for example, C₂-C₂₀ alkynyl groups such as ethynyl, propargyl, 2-methyl-2-propynyl, 3-butynyl, 4-pentynyl and 5-hexynyl and C₂-C₂₀ alkynyloxy groups such as propargyloxy, 2-methyl-2-propynyloxy, 3-butynyloxy, 4-pentynyloxy and 5-hexynyloxy; cyclic ether groups, for example, epoxy, glycidyl, alicyclic epoxy and oxetanyl; hydroxy-containing groups, for example, carboxyl and catechol; thiol groups; amino, alkylamino, dialkylamino groups; azide groups; nitrogen-containing heterocyclic groups, for example, imidazolyl, triazolyl, benzotriazolyl, tetrazolyl, isocyanate; phosphate-containing groups, for example, phosphoric acid and phosphoric ester groups; hydroxy-containing silyl groups (silanol groups) and hydrolyzable silyl groups. Of these, hydroxy-containing silyl groups (silanol groups) and hydrolyzable silyl groups are preferred.

Of the hydroxy-containing silyl groups and hydrolyzable silyl groups, groups having the general formula (2): 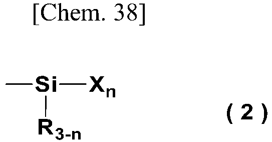
wherein R is independently a C₁-C₄ alkyl group or phenyl group, X is independently a hydroxy or hydrolyzable group, and n is an integer of 1 to 3, or the general formula (3): 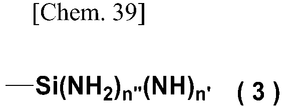
wherein n" is a number of 0 to 3, and n' is (3-n")/2 are preferred.

In formula (2), R is independently a C₁-C₄ alkyl group such as methyl, ethyl, propyl or butyl or phenyl group. Inter alia, methyl is preferred.

In formula (2), X is independently a hydroxy or hydrolyzable group. Examples of the group X include hydroxy; C₁-C₁₀ alkoxy groups such as methoxy, ethoxy, propoxy, isopropoxy and butoxy; C₂-C₁₀ alkoxyalkoxy groups such as methoxymethoxy and methoxyethoxy; C₁-C₁₀ acyloxy groups such as acetoxy; C₂-C₁₀ alkenyloxy groups such as isopropenoxy and cyclopentenyloxy; halogens such as chloro, bromo and iodo; and C₂-C₁₀ dialkylamino groups such as dimethylamino and diethylamino. Inter alia, methoxy, ethoxy, isopropenoxy and chloro are preferred. X may be the same or different.

In formula (3), n" is a number of 0 to 3 (0 or a positive number of up to 3), preferably n" < 3, most preferably n"=0. When n"=3 in formula (3), formula (1) represents the molecular formula or structural formula of a hydrocarbon end group-containing compound (monomer). When n" < 3 in formula (3), formula (1) represents the compositional formula of a polymer (polysilazane compound) of a hydrocarbon end group-containing compound.

In formula (3), n' is (3-n")/2, preferably 1.5.

In formula (1), k is 0 or 1. When U is trivalent, k is 0. When U is tetravalent, k is 1.

Also, m is an integer of 1 to 7, preferably 1 to 3.

Of the hydrocarbon end group-containing compounds having formula (1), hydrocarbon end group-containing compounds having the following formula (Z) are preferred because cured films having chemical resistance are formed.

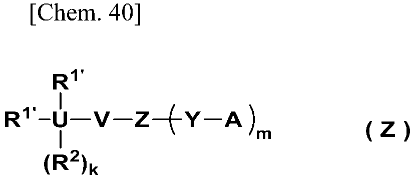

Herein R^{1'} is independently a straight, branched and/or cyclic, C₁₃-C₃₂ monovalent hydrocarbon group. R² is hydrogen, halogen, hydroxy, siloxy, amino, thiol, monovalent hydrocarbon group of 1 or 2 carbon atoms, R¹ or -Y-A. U is carbon, silicon, nitrogen or tri- or tetravalent organic group. V is a single bond or a divalent hydrocarbon group which may contain at least one element selected from oxygen, nitrogen and sulfur. Z is a single bond, carbon, silicon, nitrogen, sulfur or a trivalent to octavalent organic group.

Y is independently a divalent hydrocarbon group which may contain at least one element selected from oxygen, nitrogen, sulfur and silicon. "A" is independently a monovalent reactive group, k is 0 or 1, and m is an integer of 1 to 7.

In formula (Z), R^{1'} is independently a straight, branched and/or cyclic, C₁₃-C₃₂ monovalent hydrocarbon group, preferably C₁₅-C₂₈ monovalent hydrocarbon group. With the carbon count of R^{1'} being 13 or more, an effect of preventing chemicals from penetrating into the substrate-bonding portion is exerted. Since R^{1'} does not include a structure containing at least one of oxygen, sulfur, nitrogen and silicon atoms (especially, ester structure, urethane structure or -C(=O)N= structure), the effect of preventing penetration of chemicals is more exerted, leading to greater chemical resistance.

R², U, V and Z in formula (Z) are as defined in formula (1).

Exemplary structures of the hydrocarbon end group-containing compound having formula (1) are shown below. By changing the combination of R¹, R², U, V, Z, Y, A, k and m in formula (1), a number of hydrocarbon end group-containing compounds are obtained.

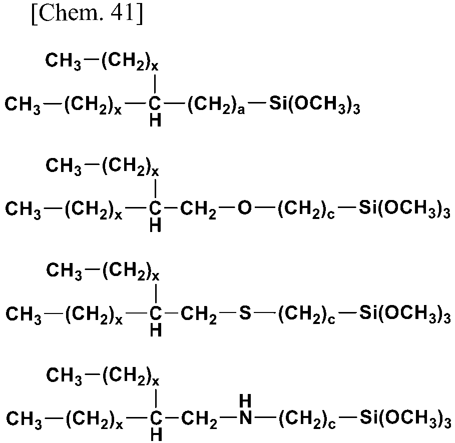

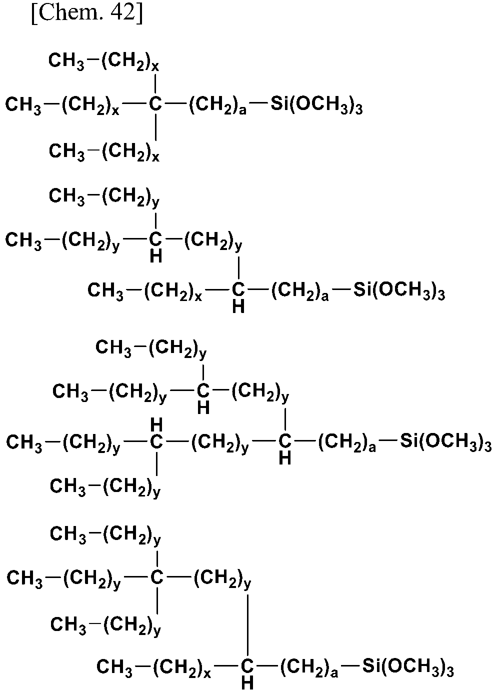

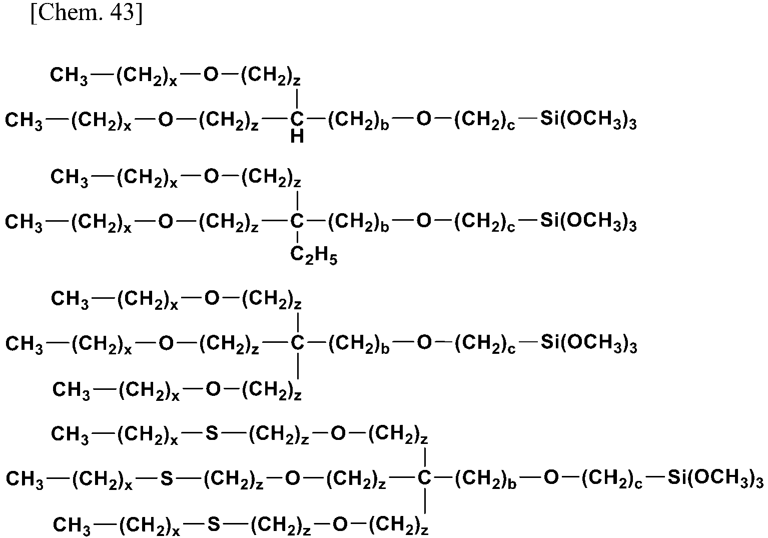

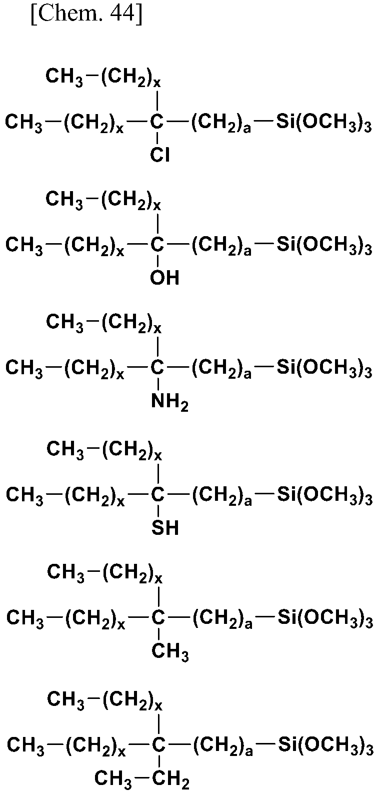

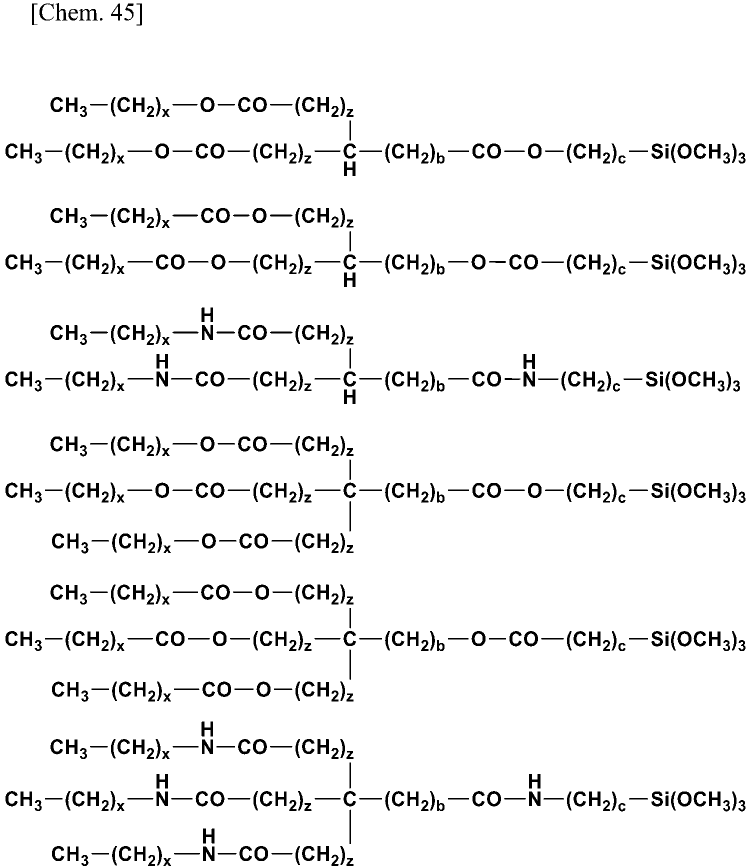

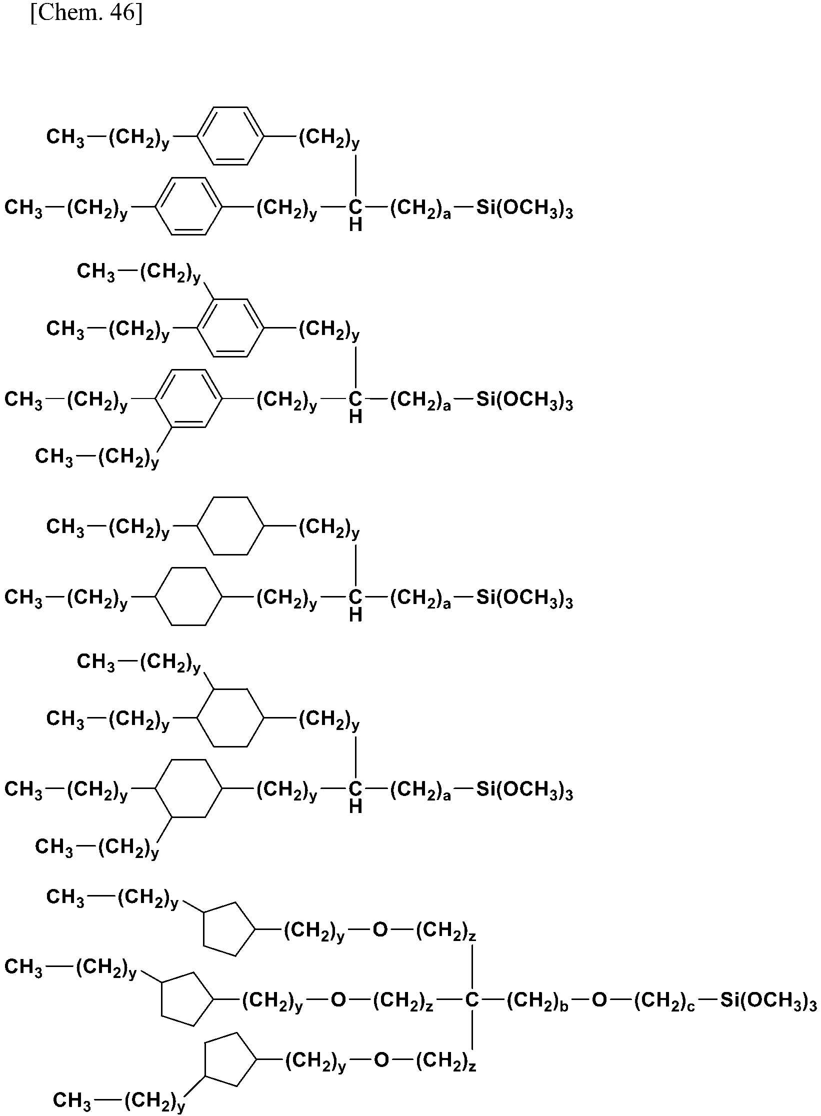

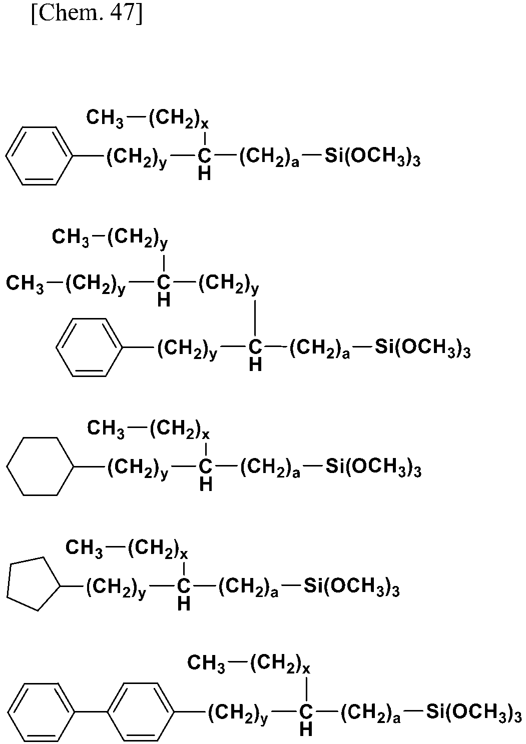

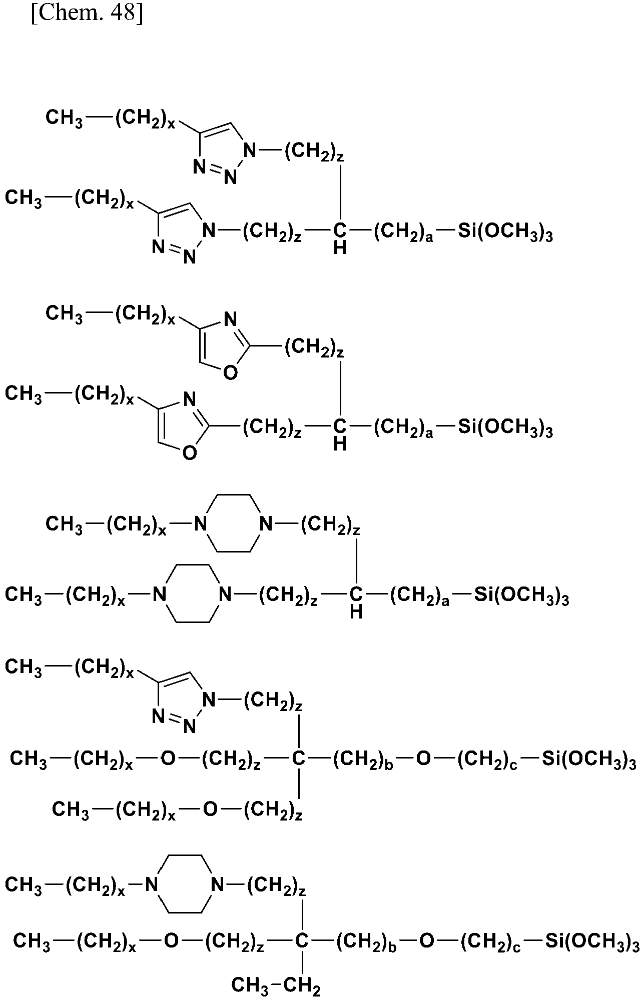

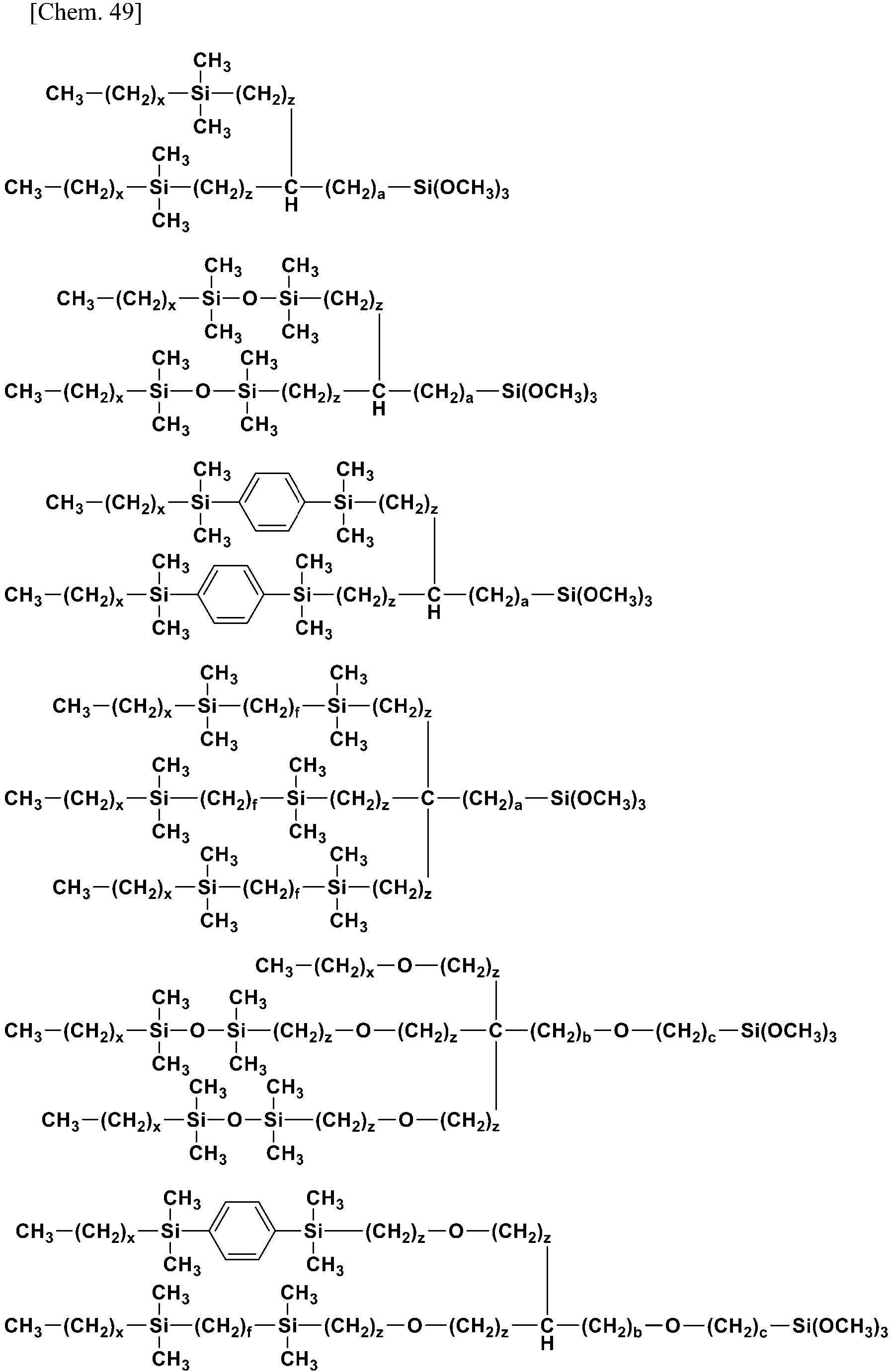

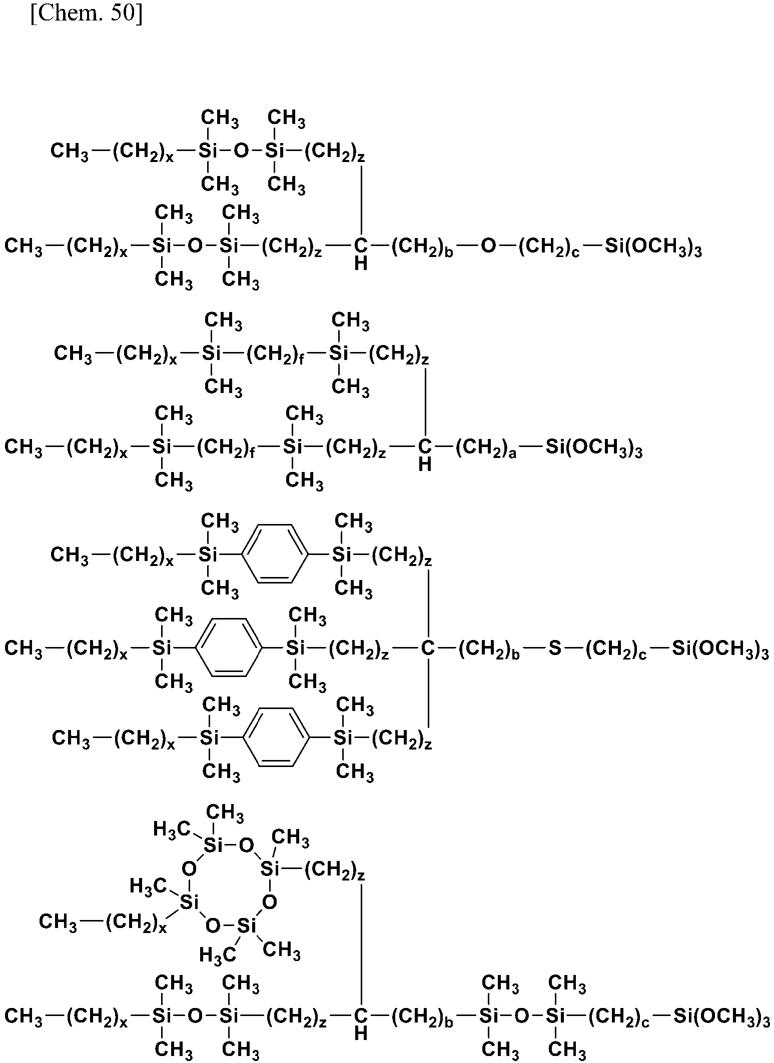

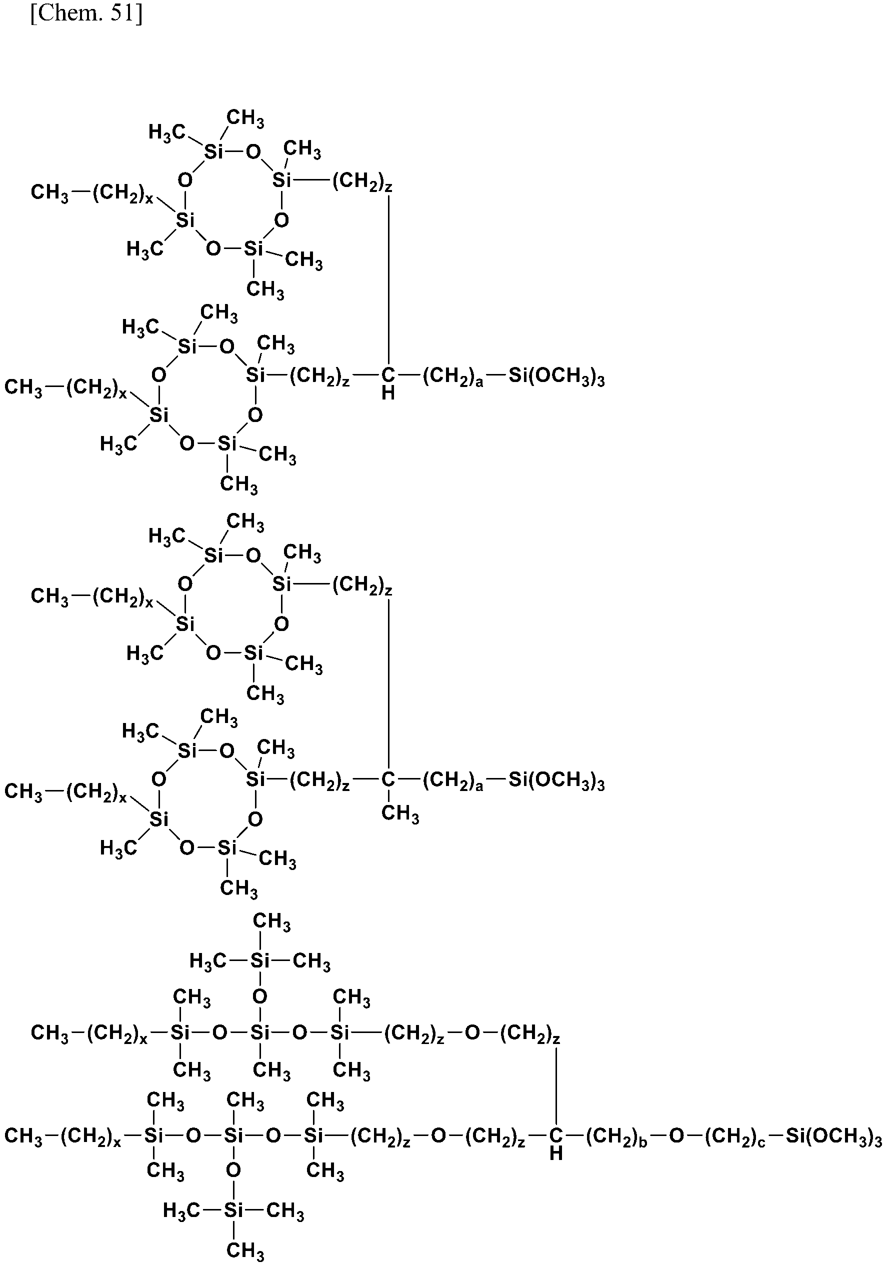

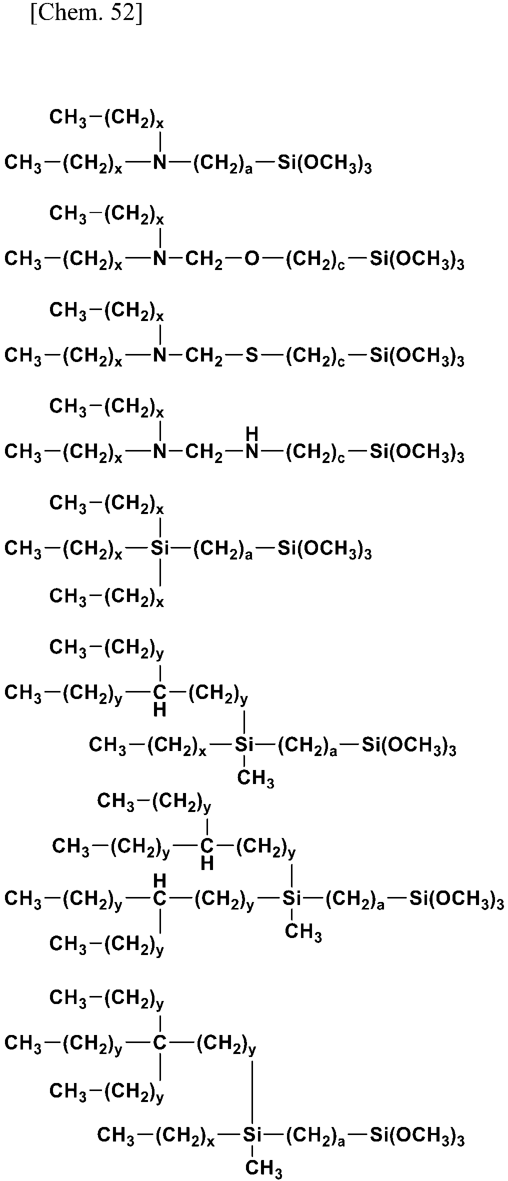

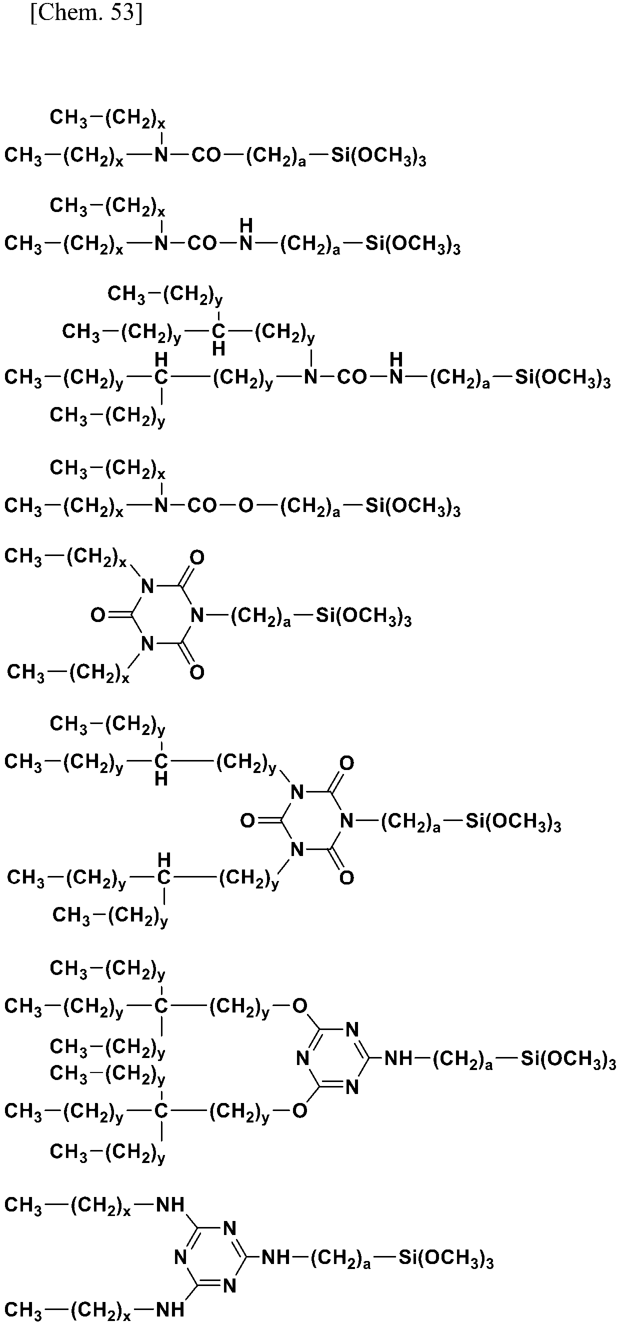

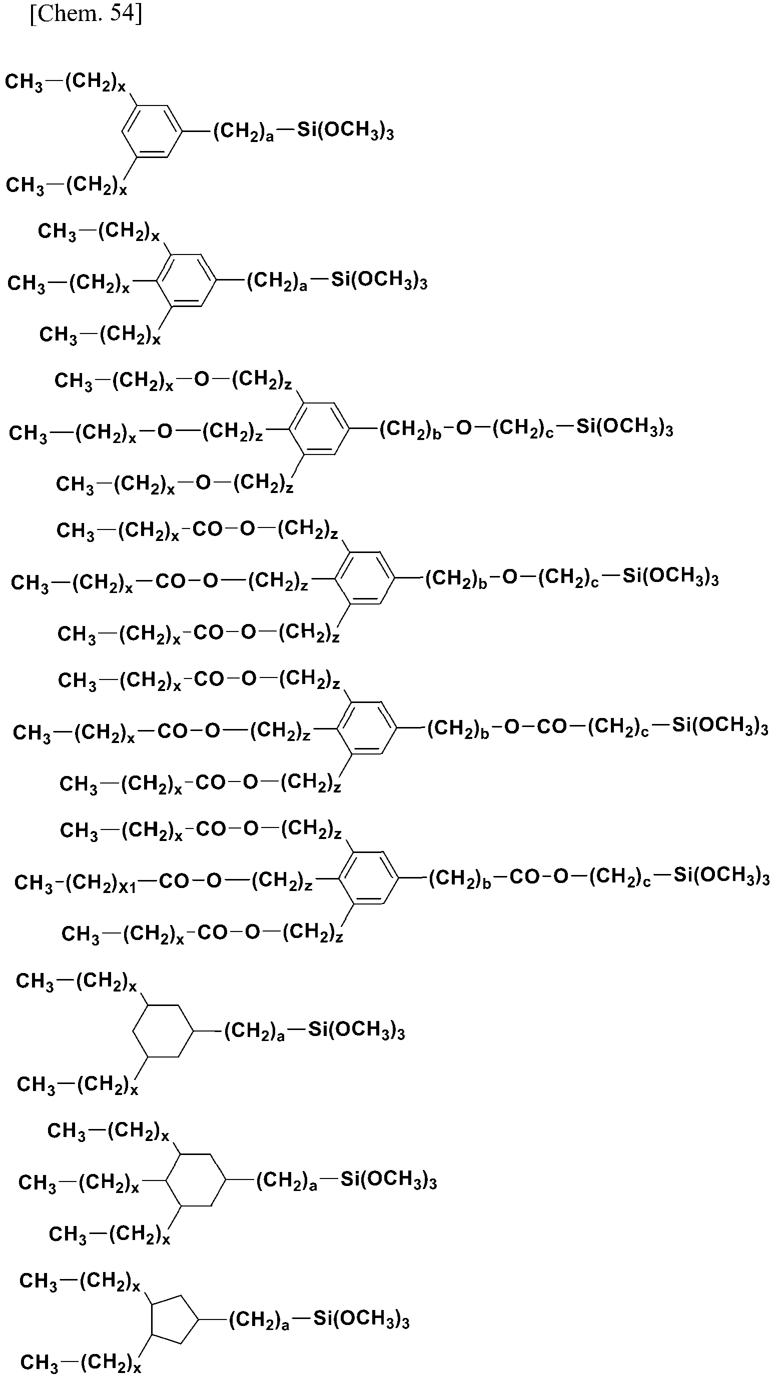

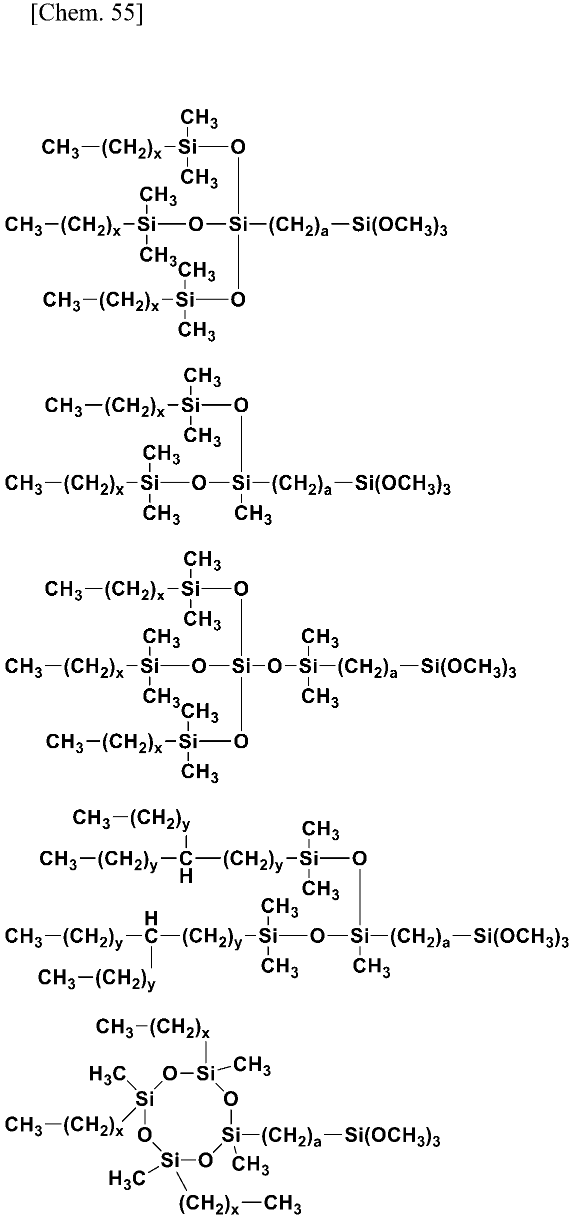

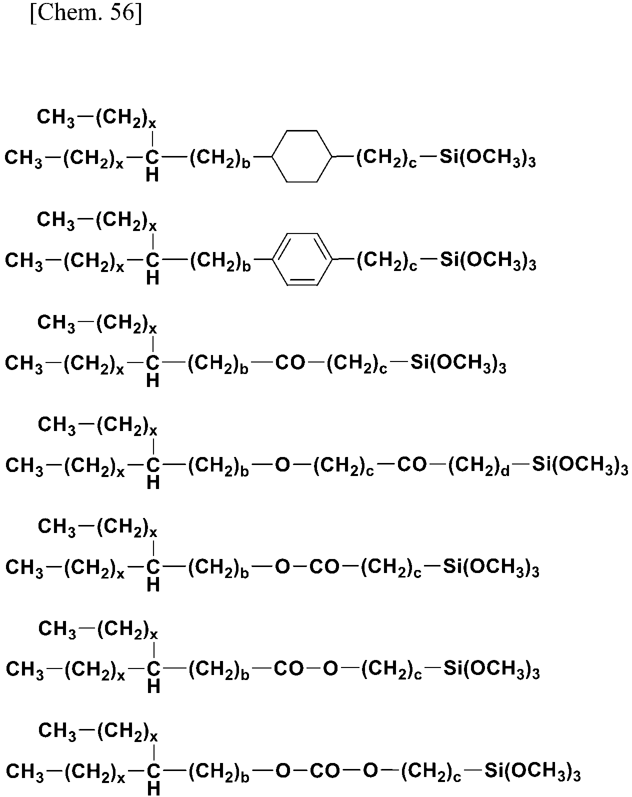

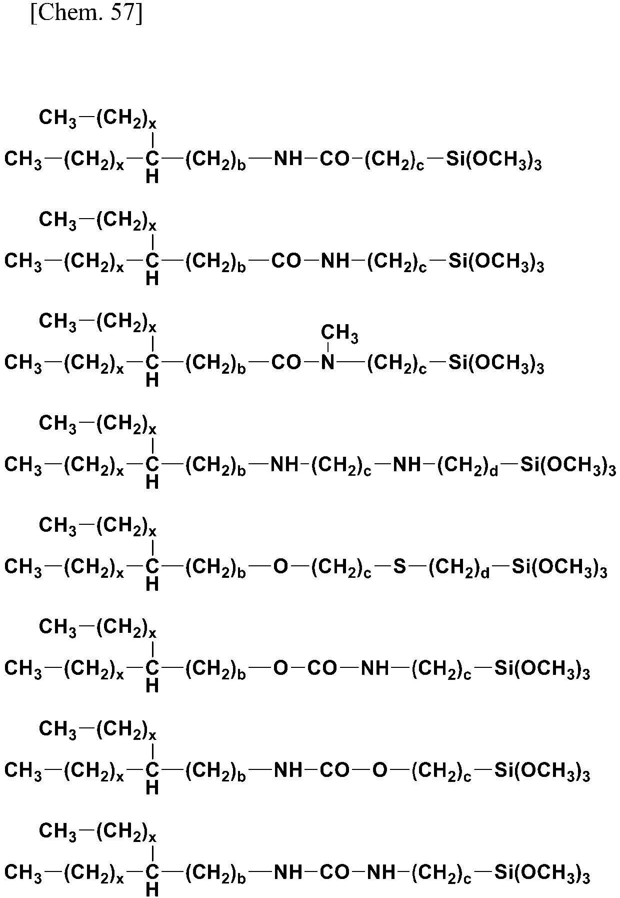

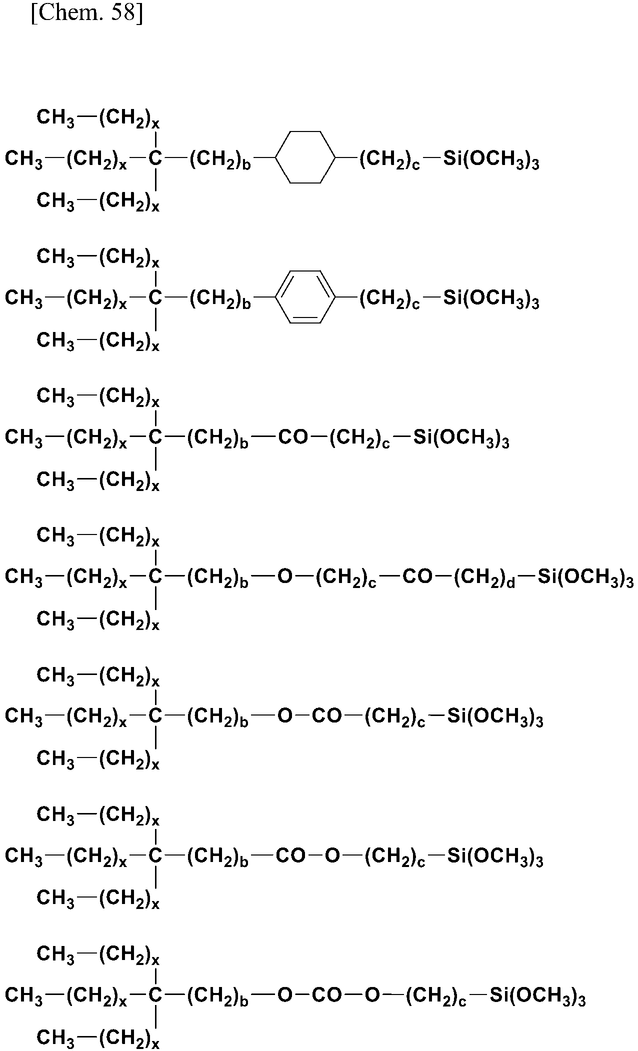

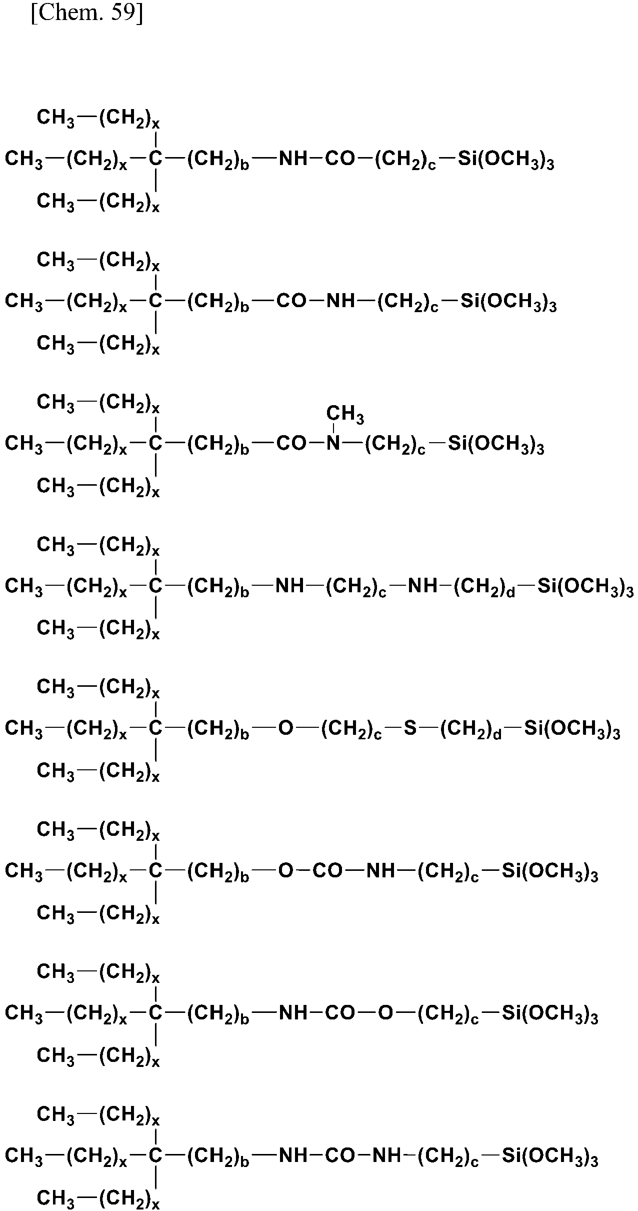

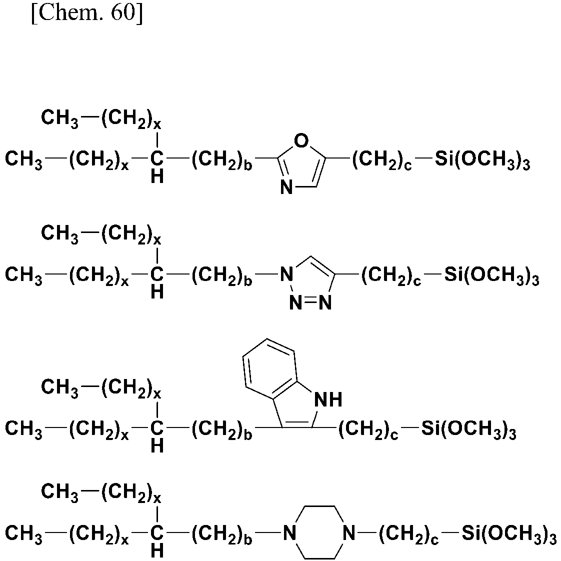

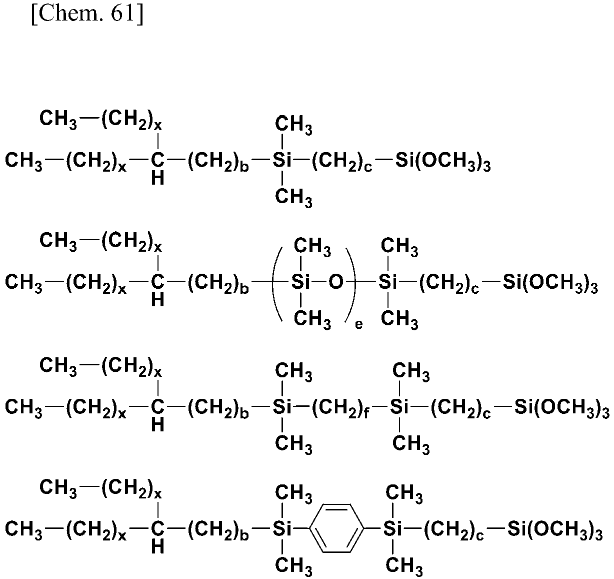

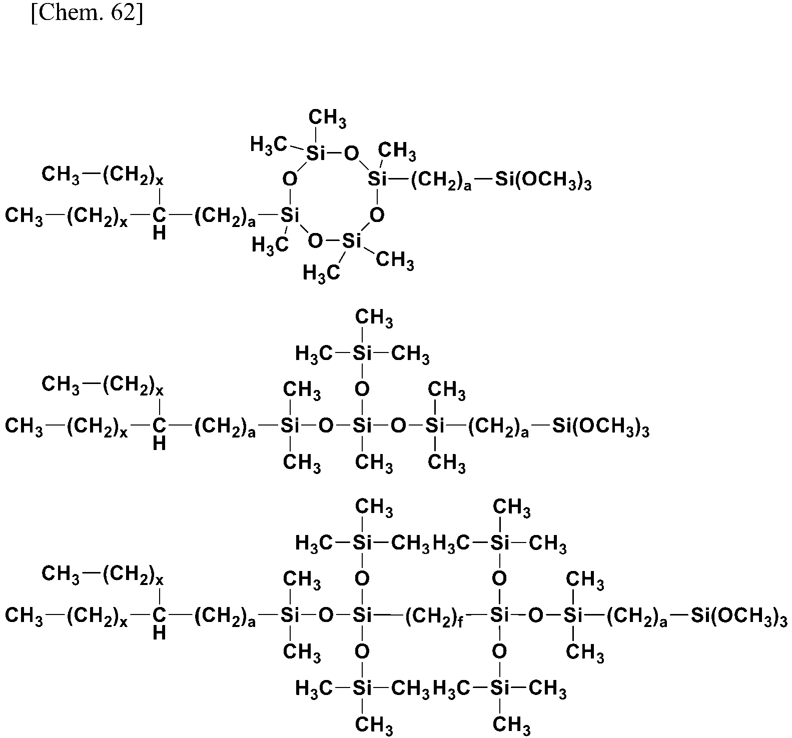

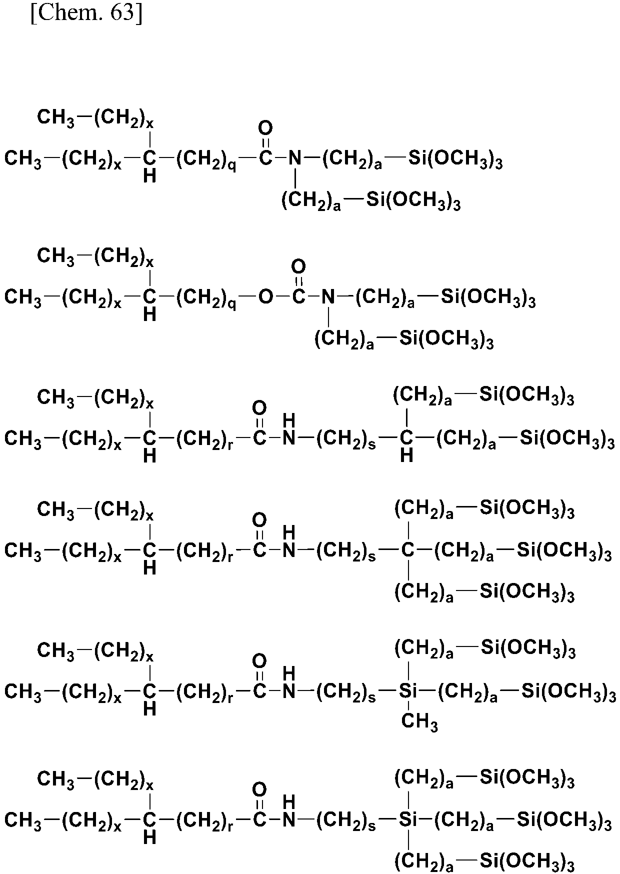

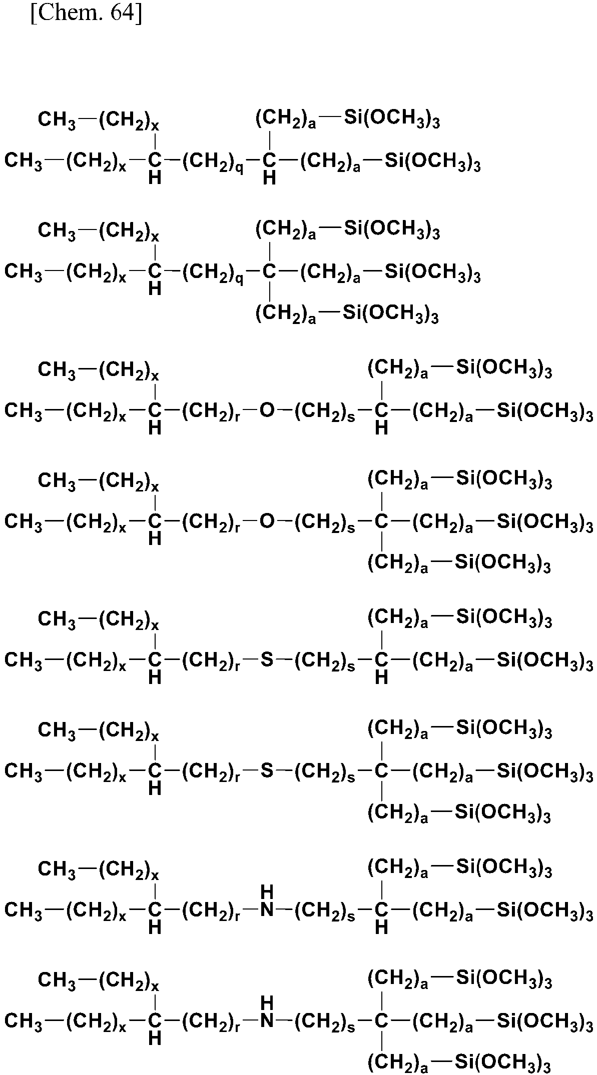

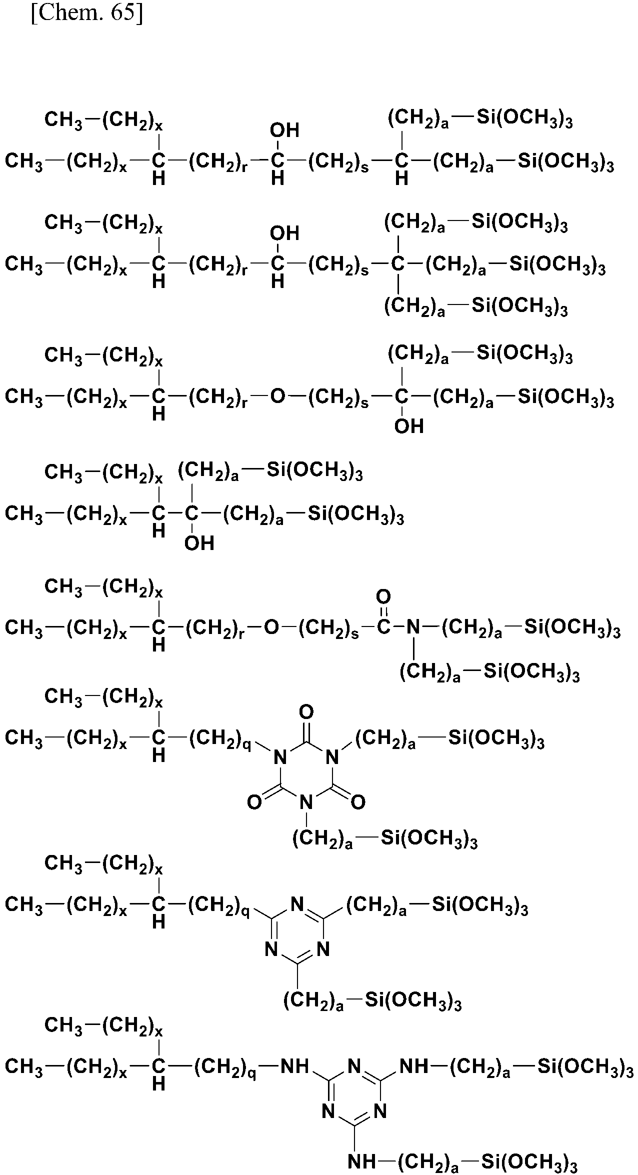

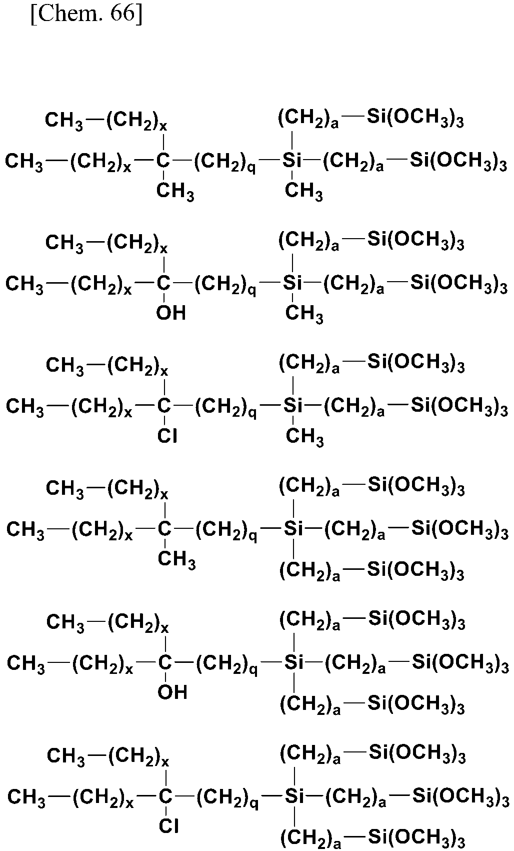

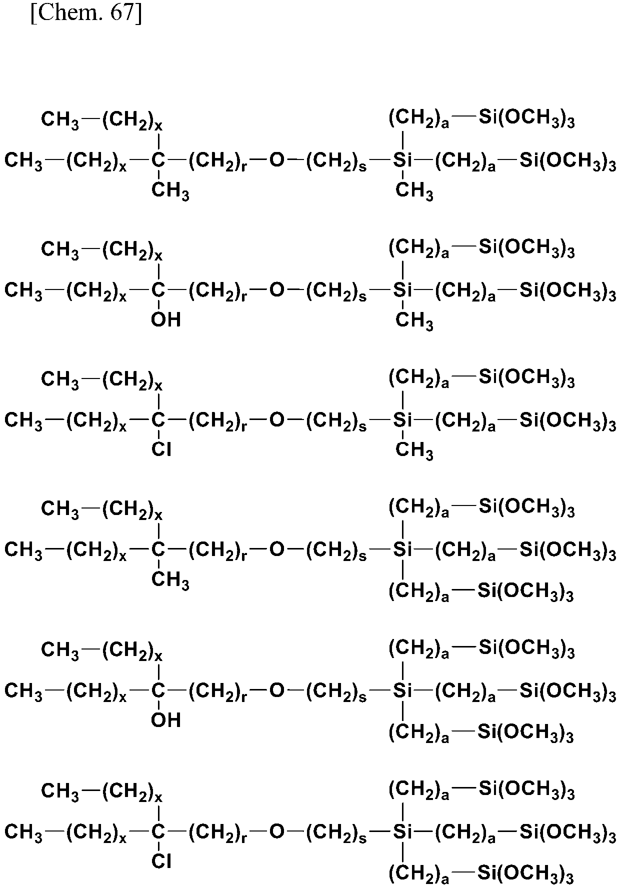

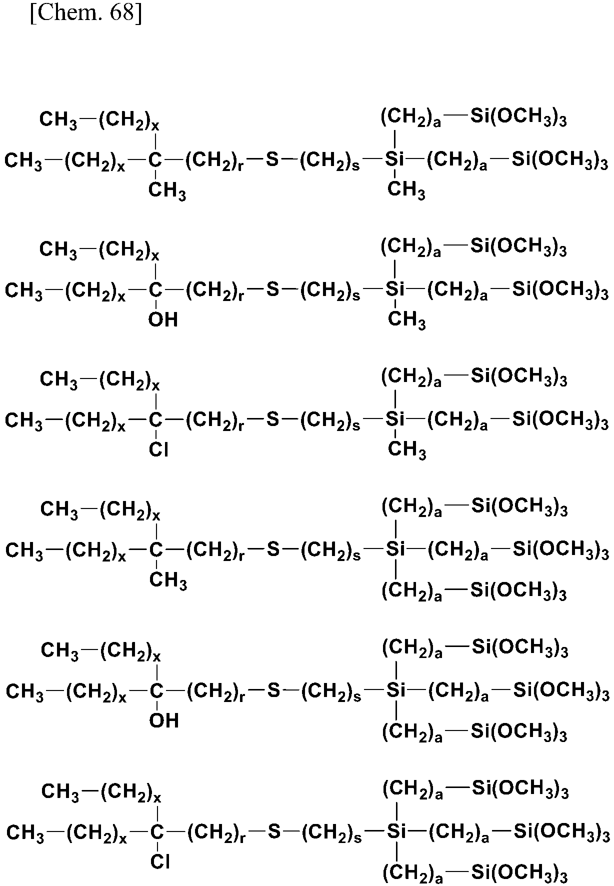

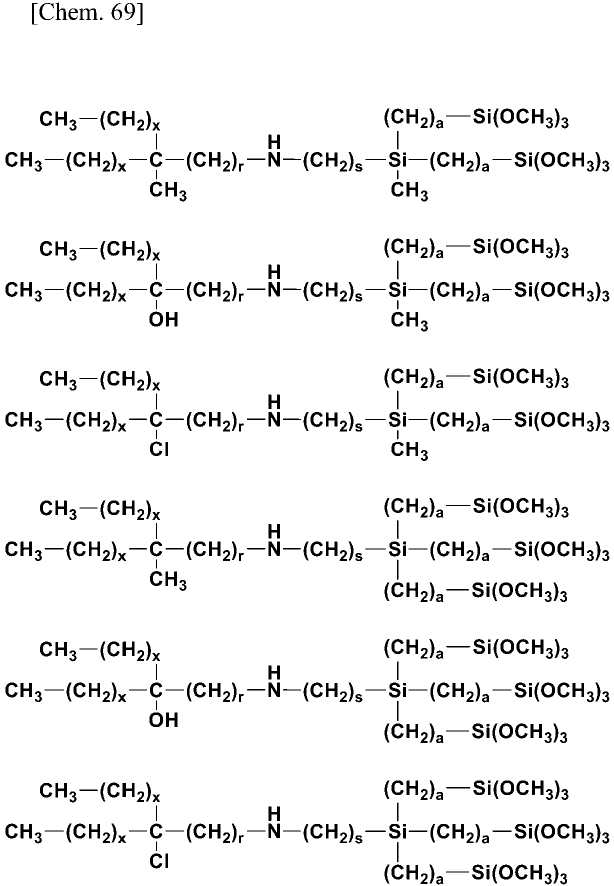

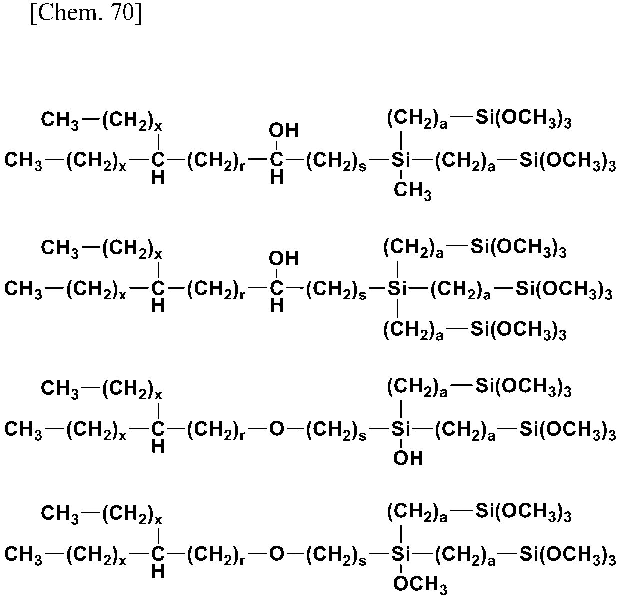

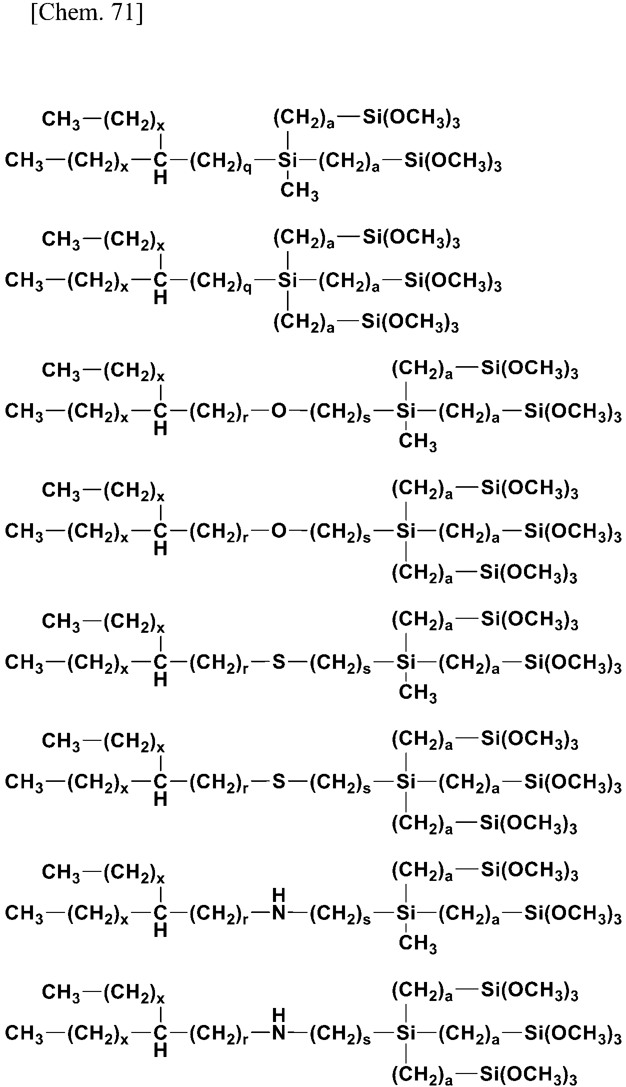

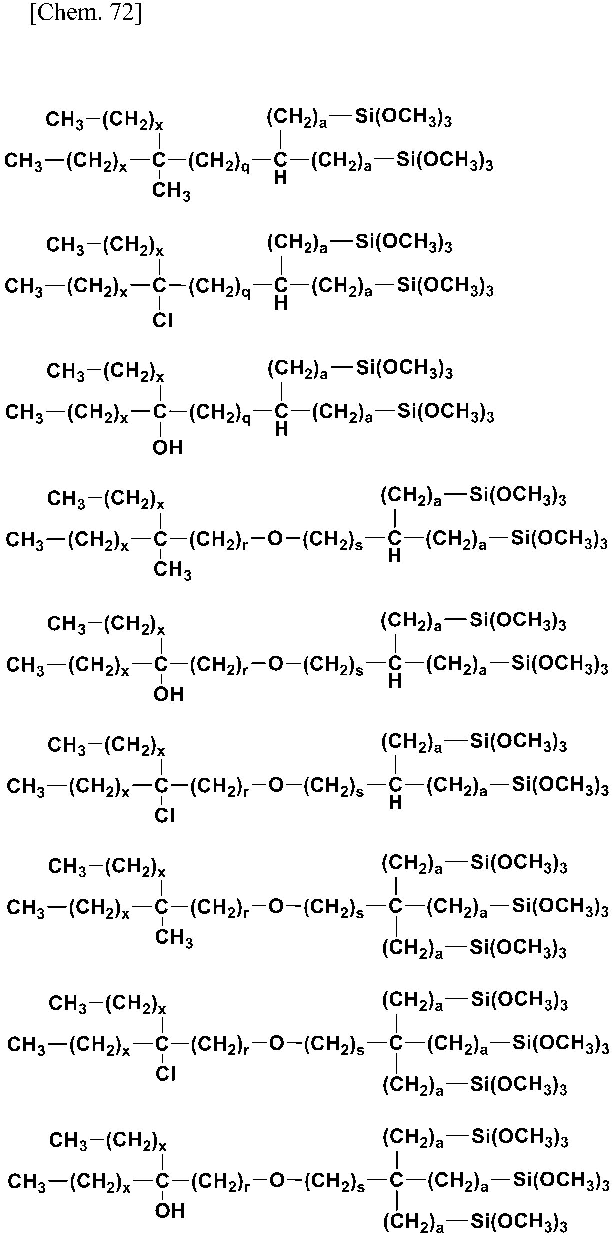

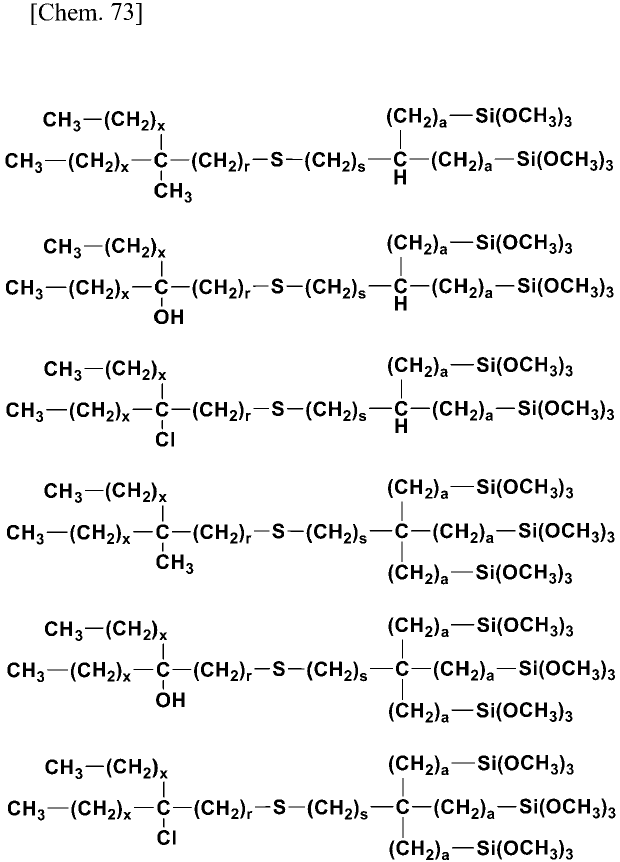

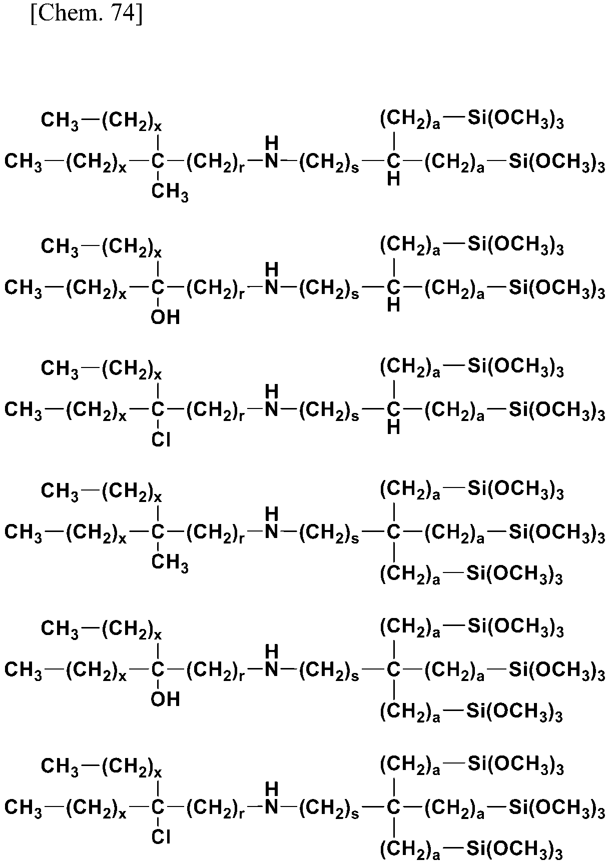

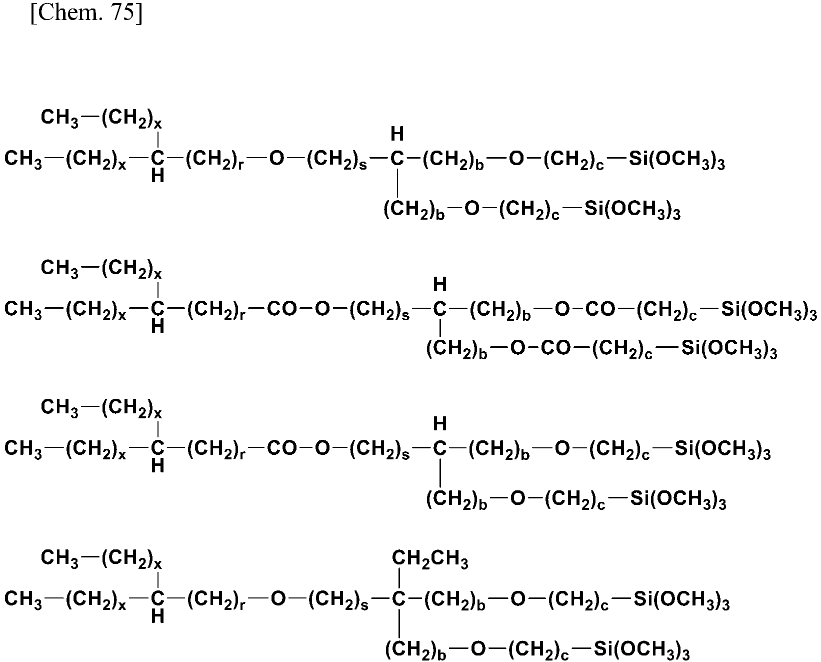

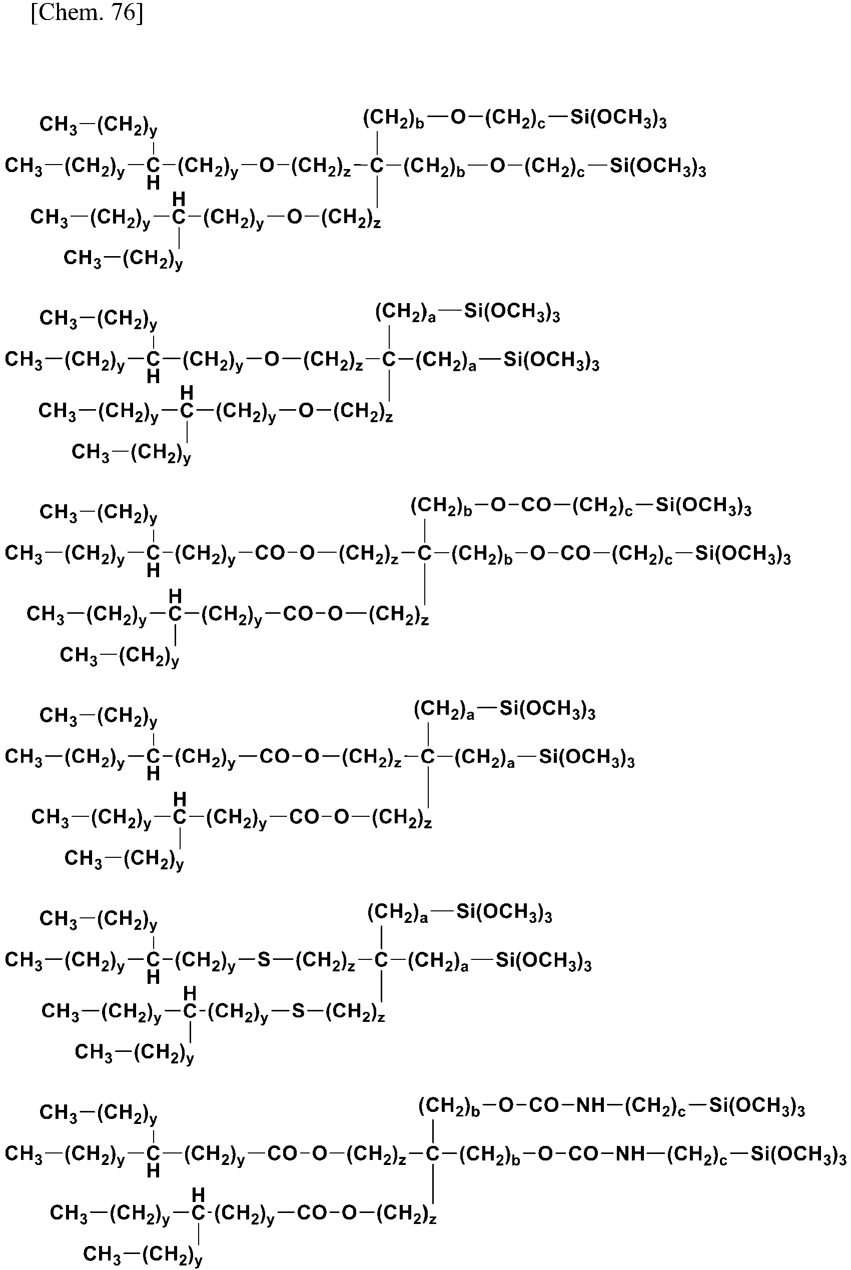

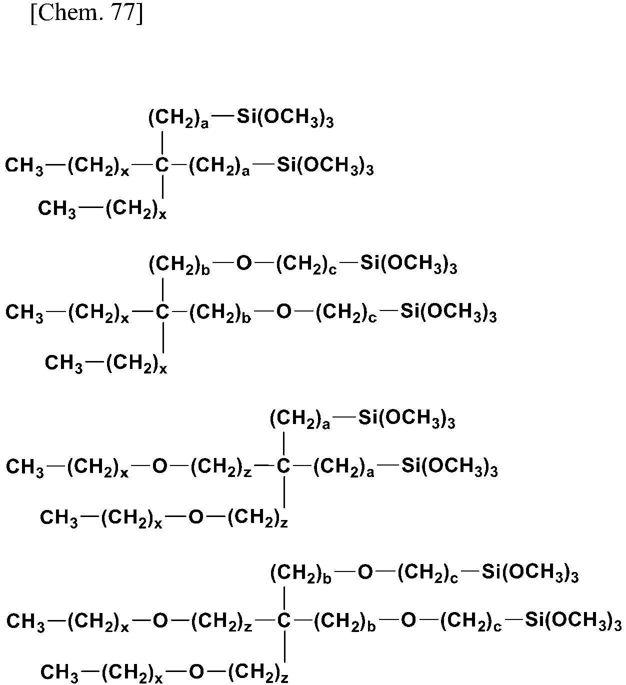

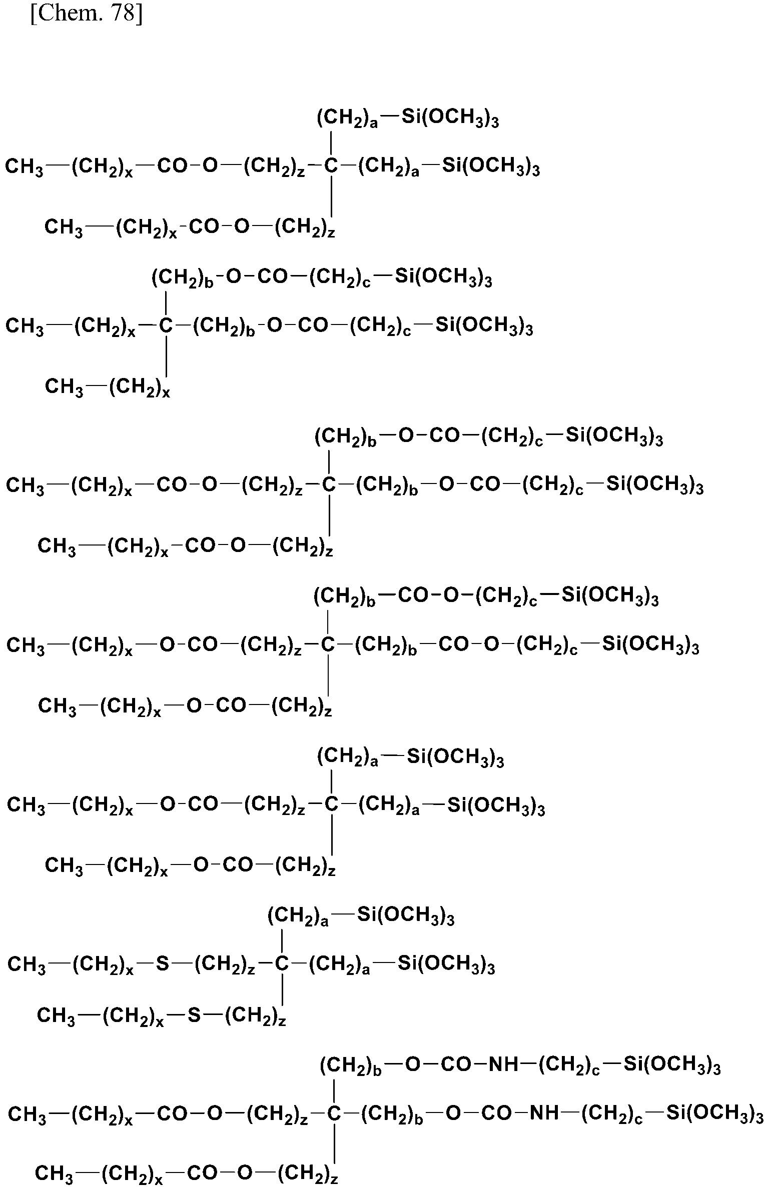

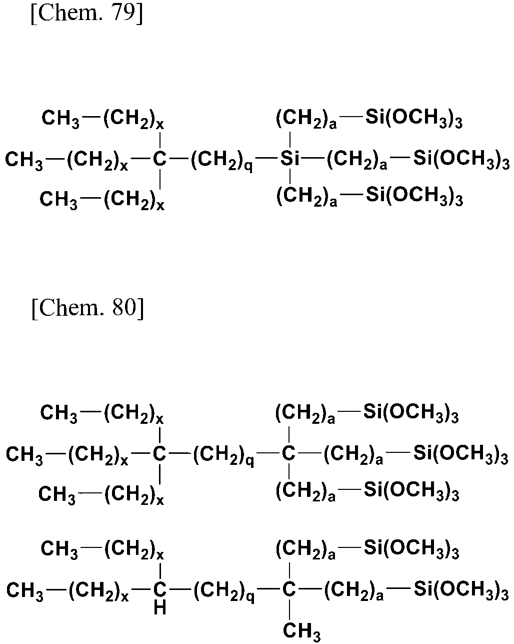

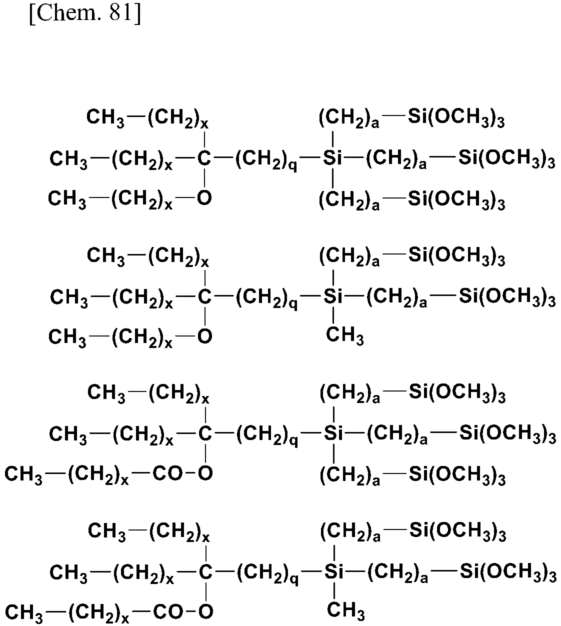

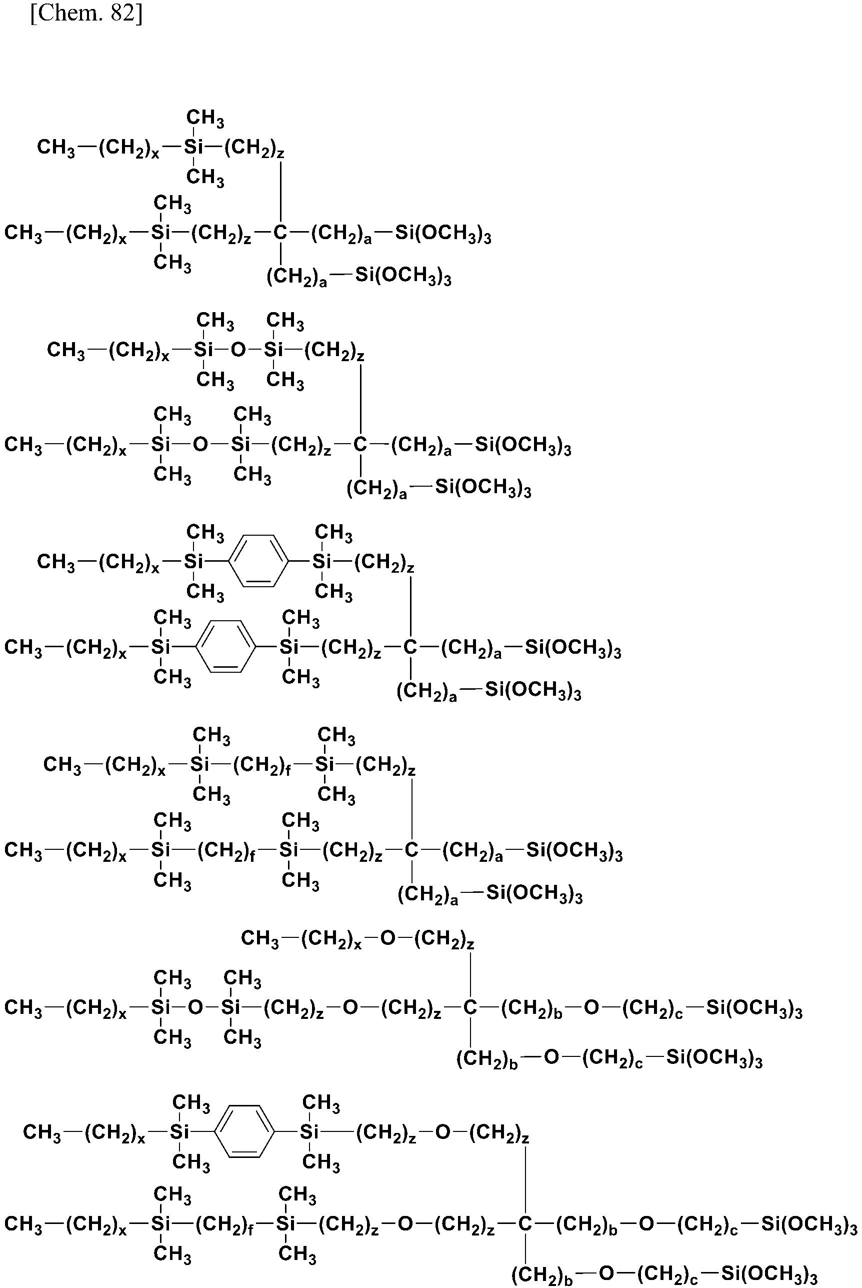

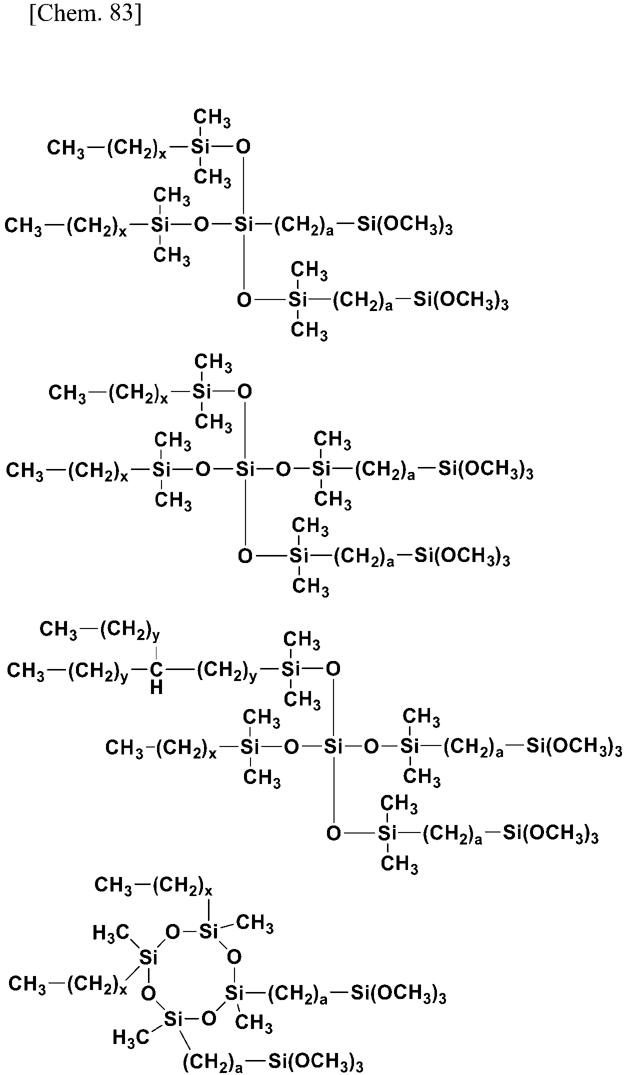

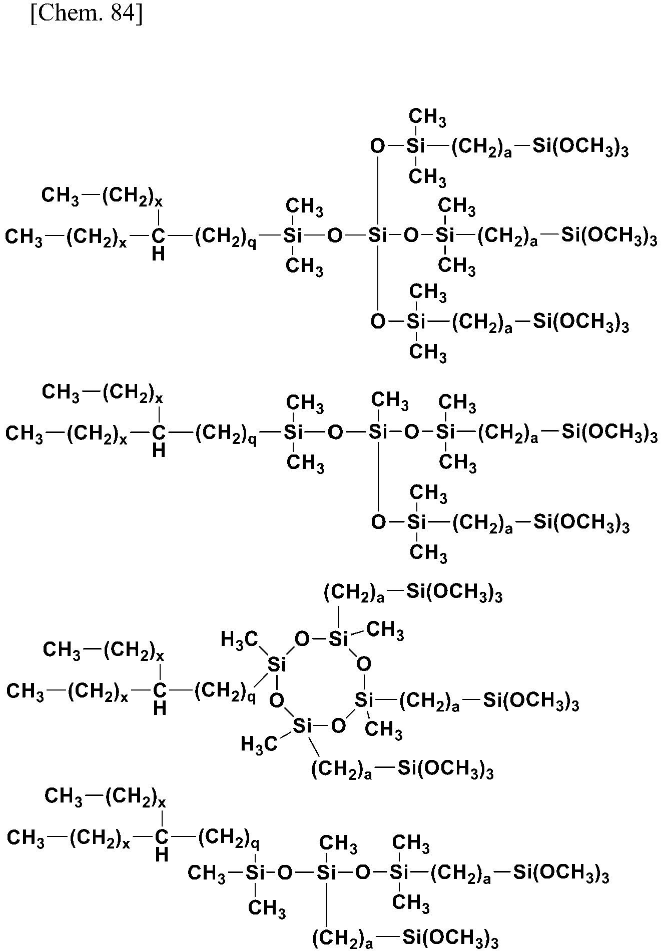

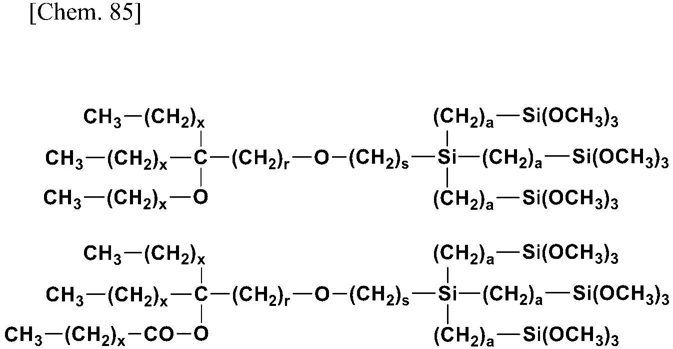

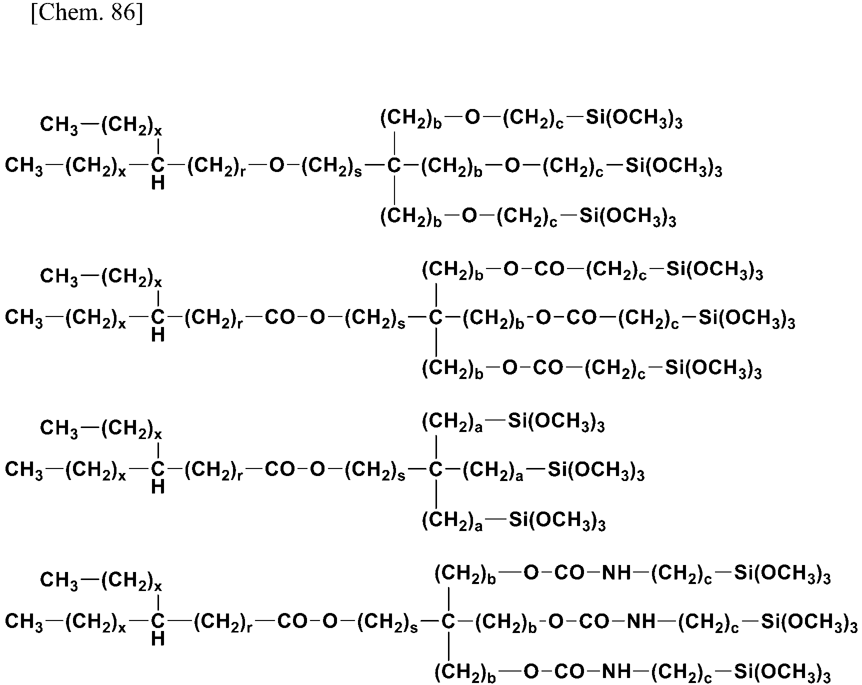

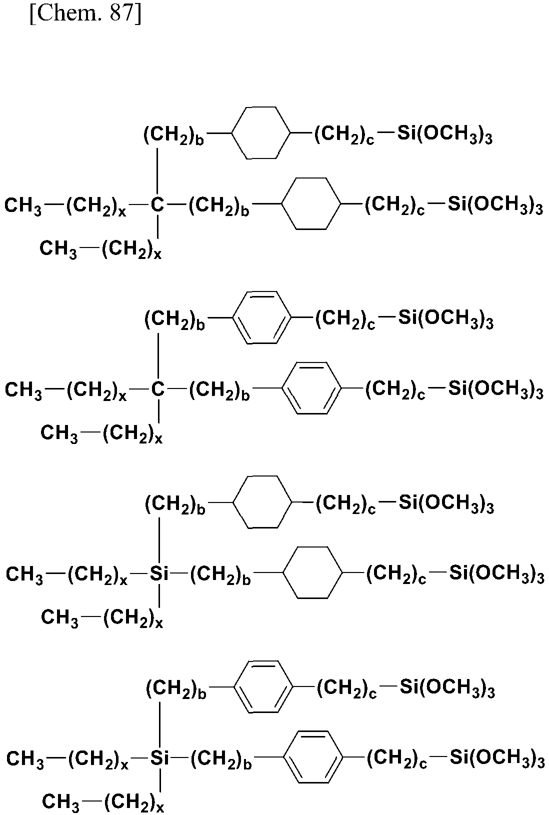

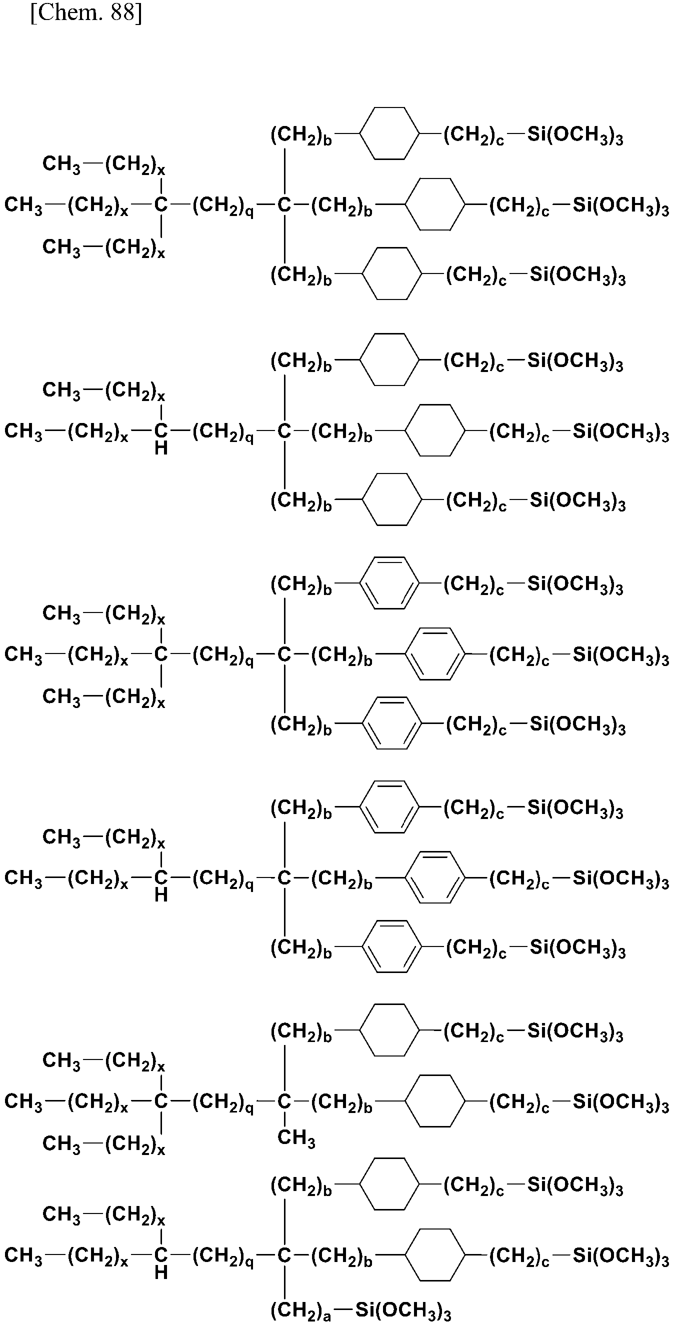

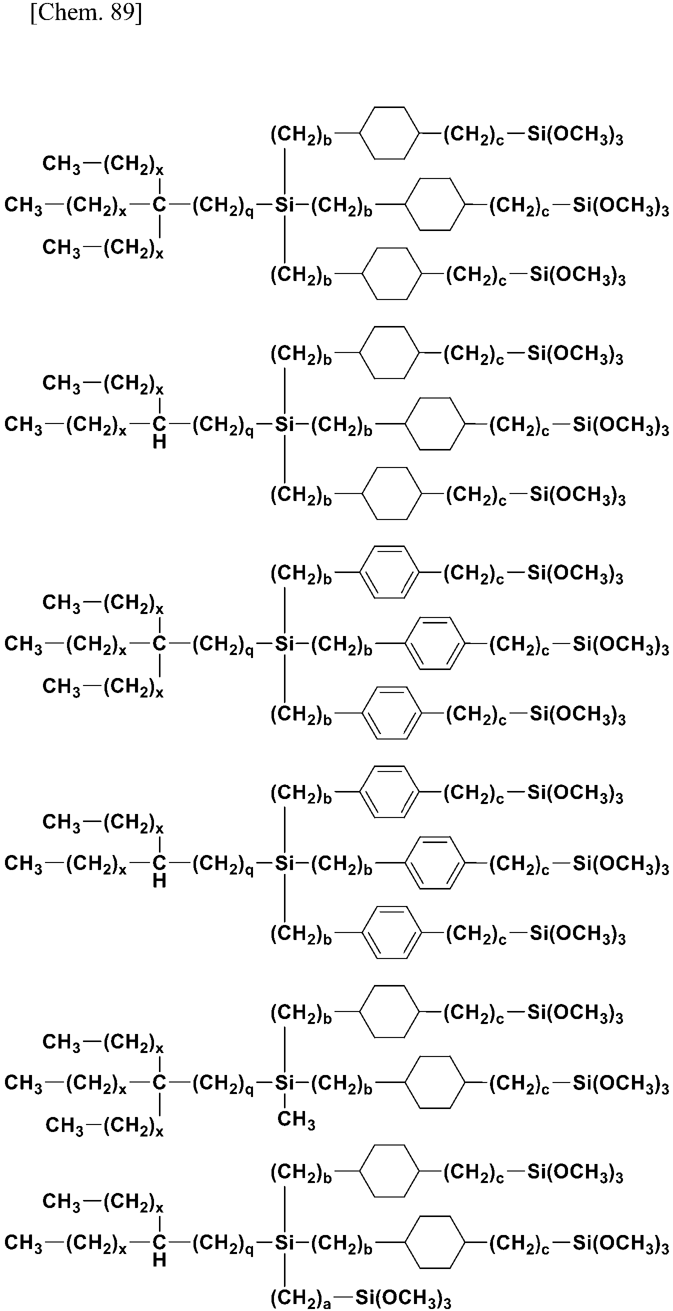

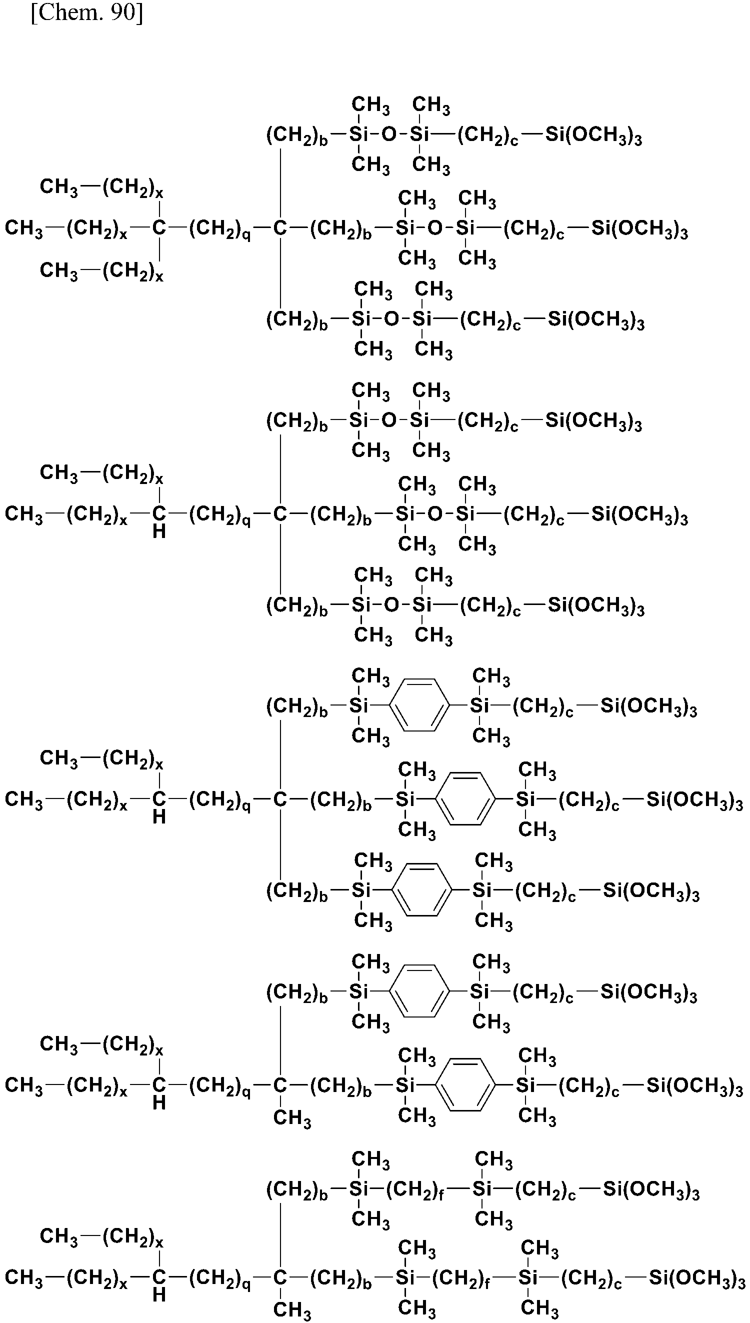

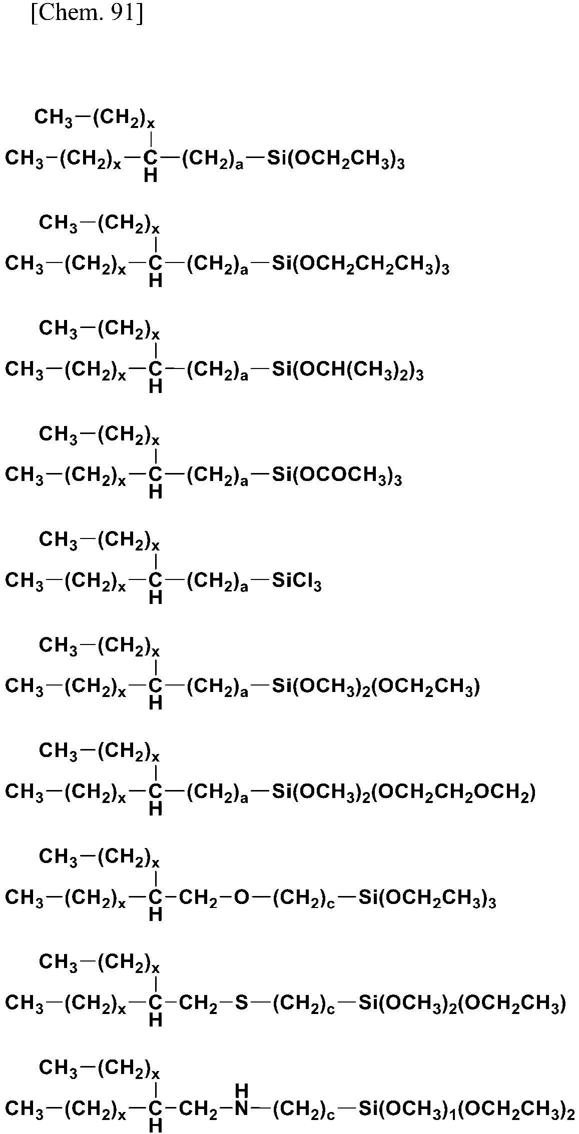

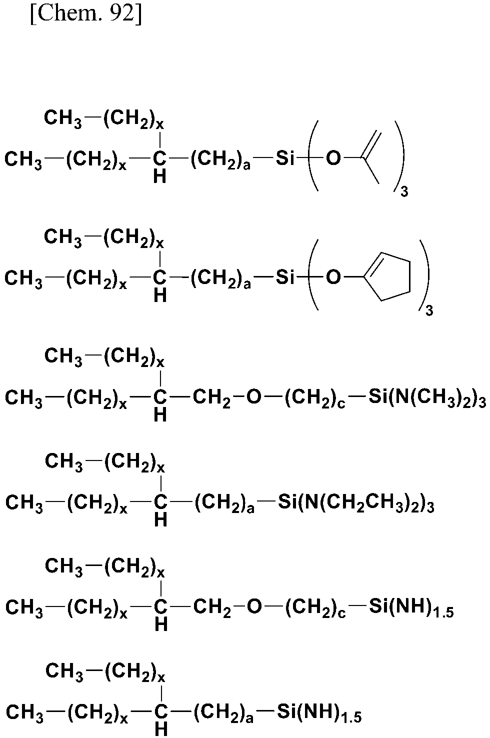

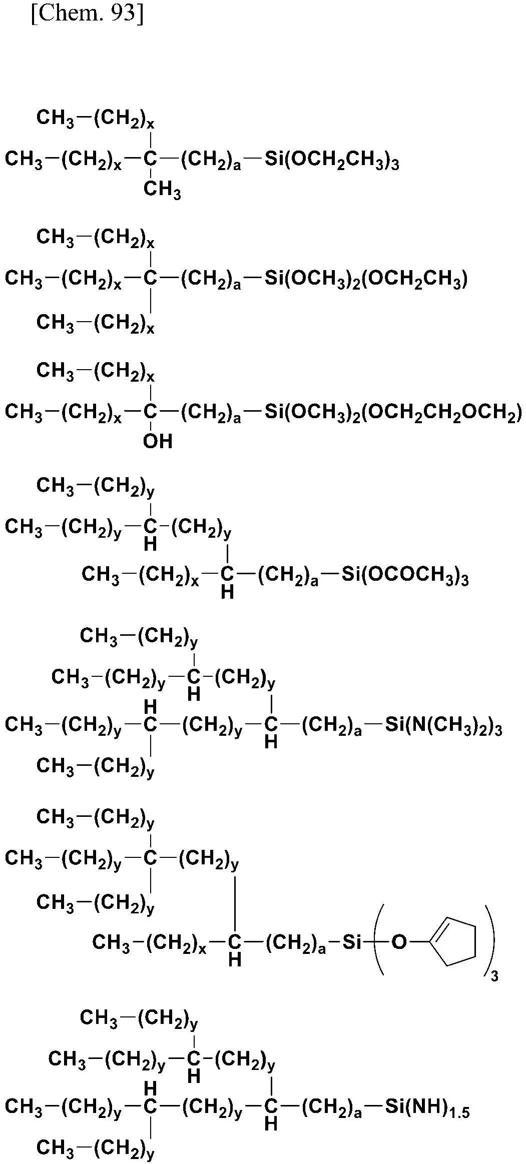

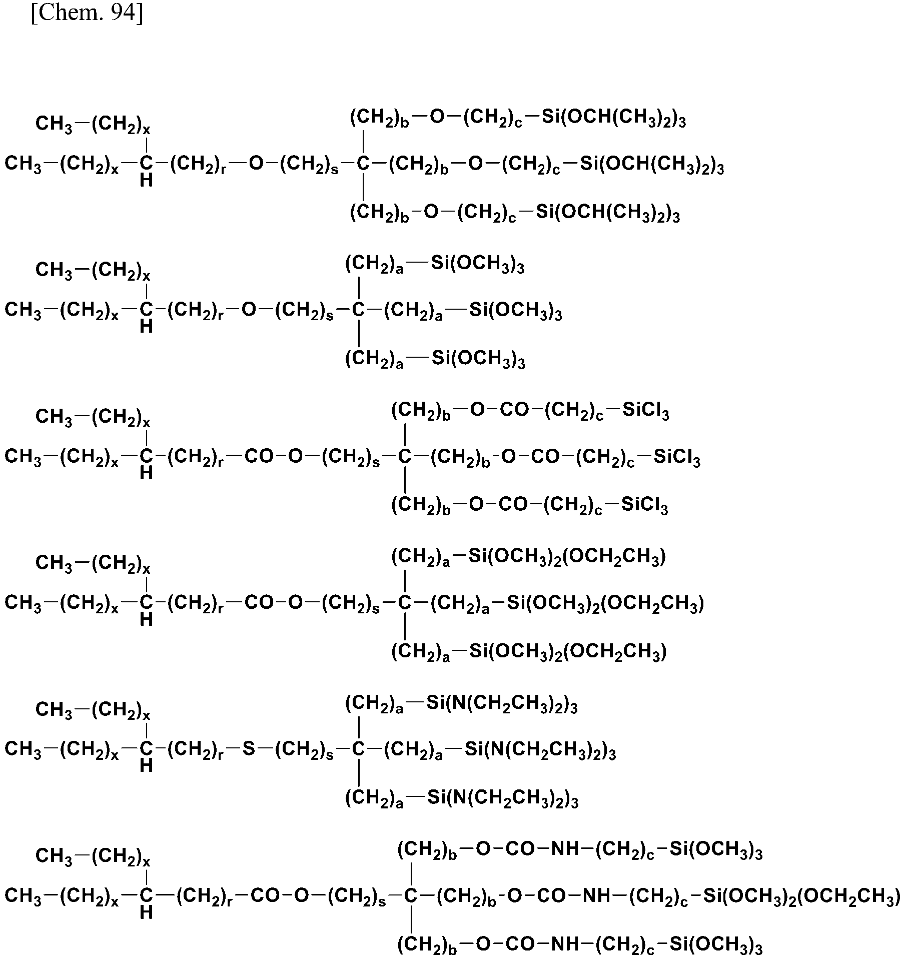

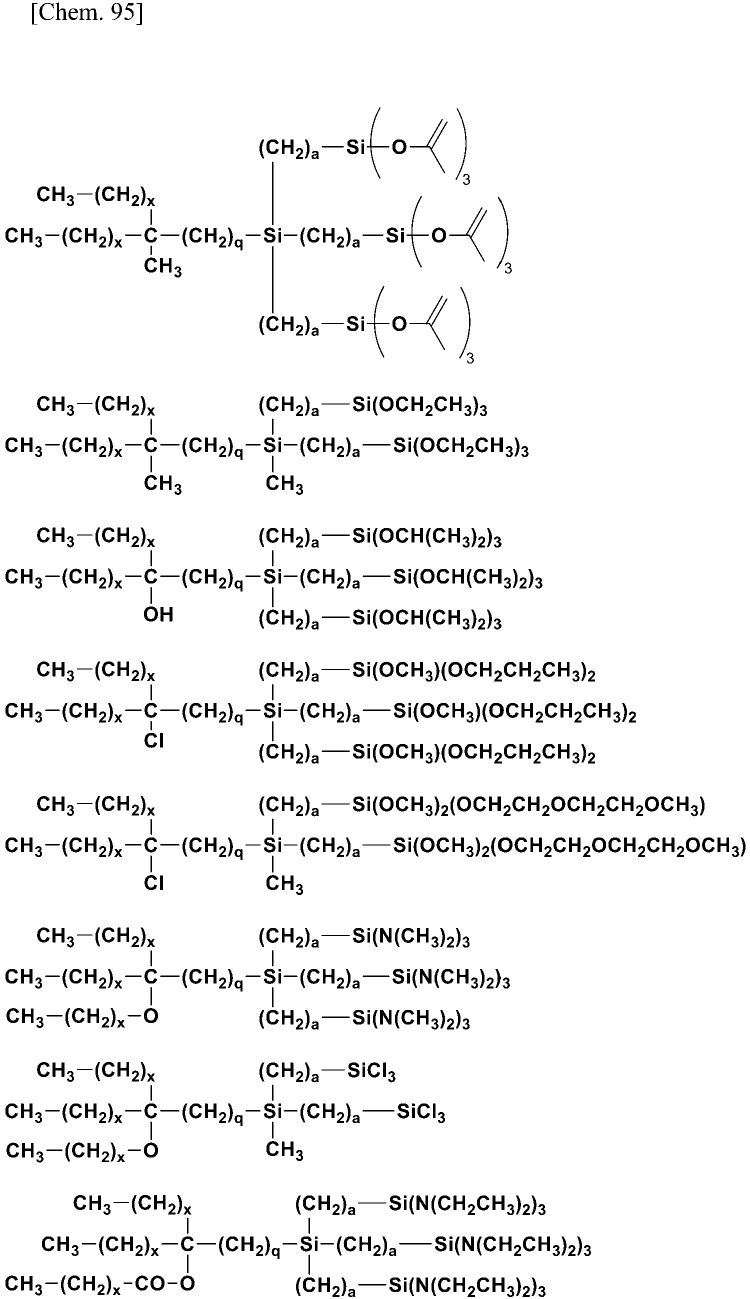

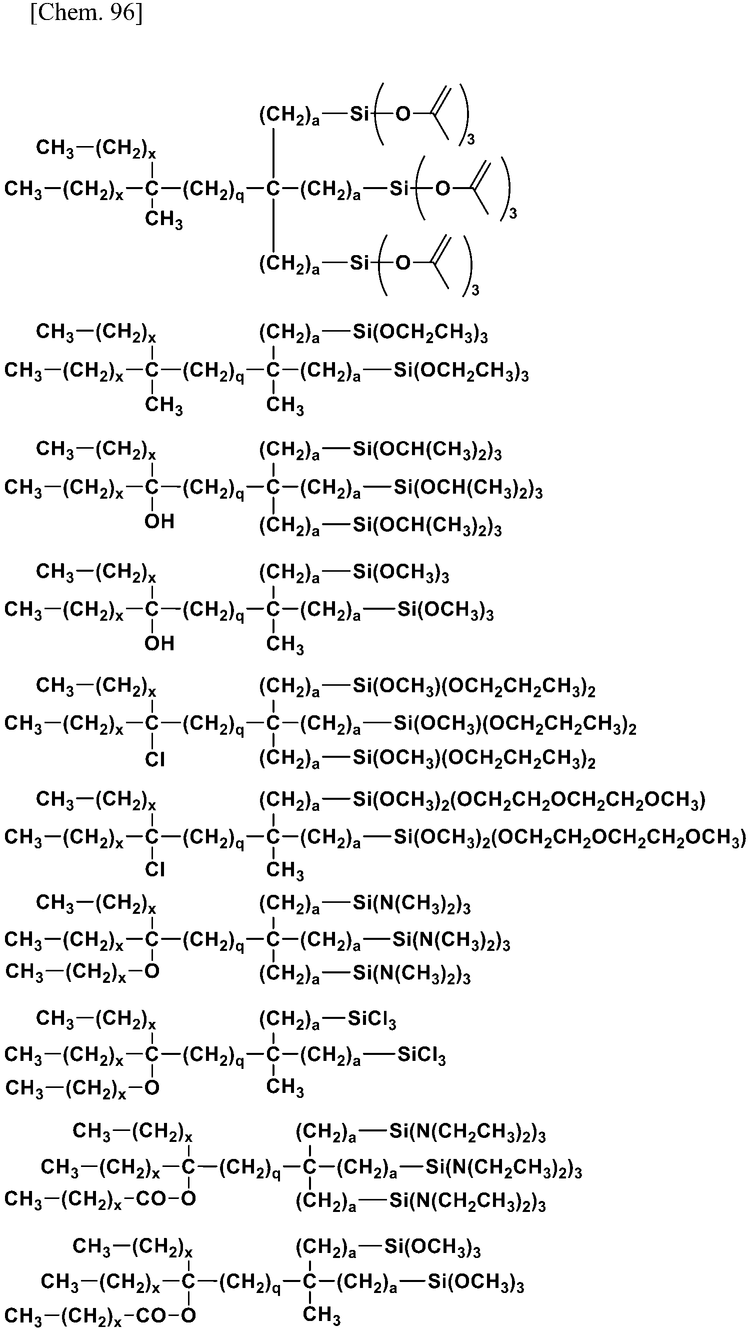

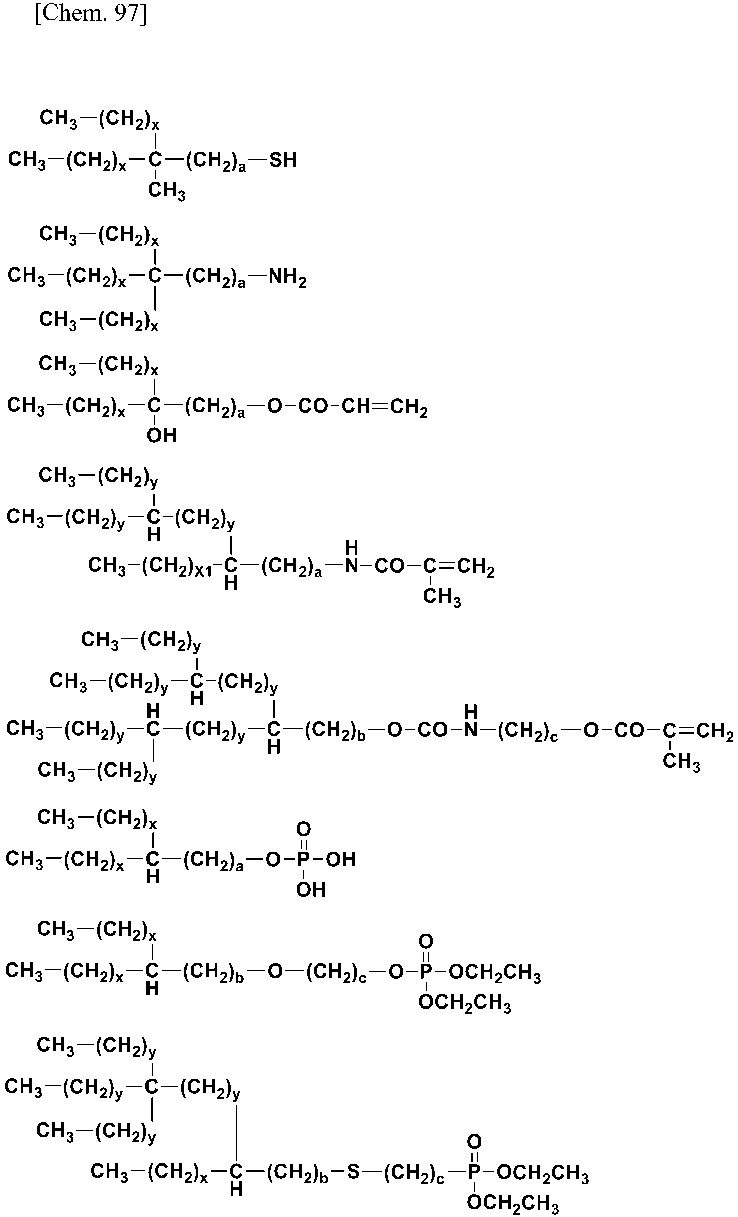

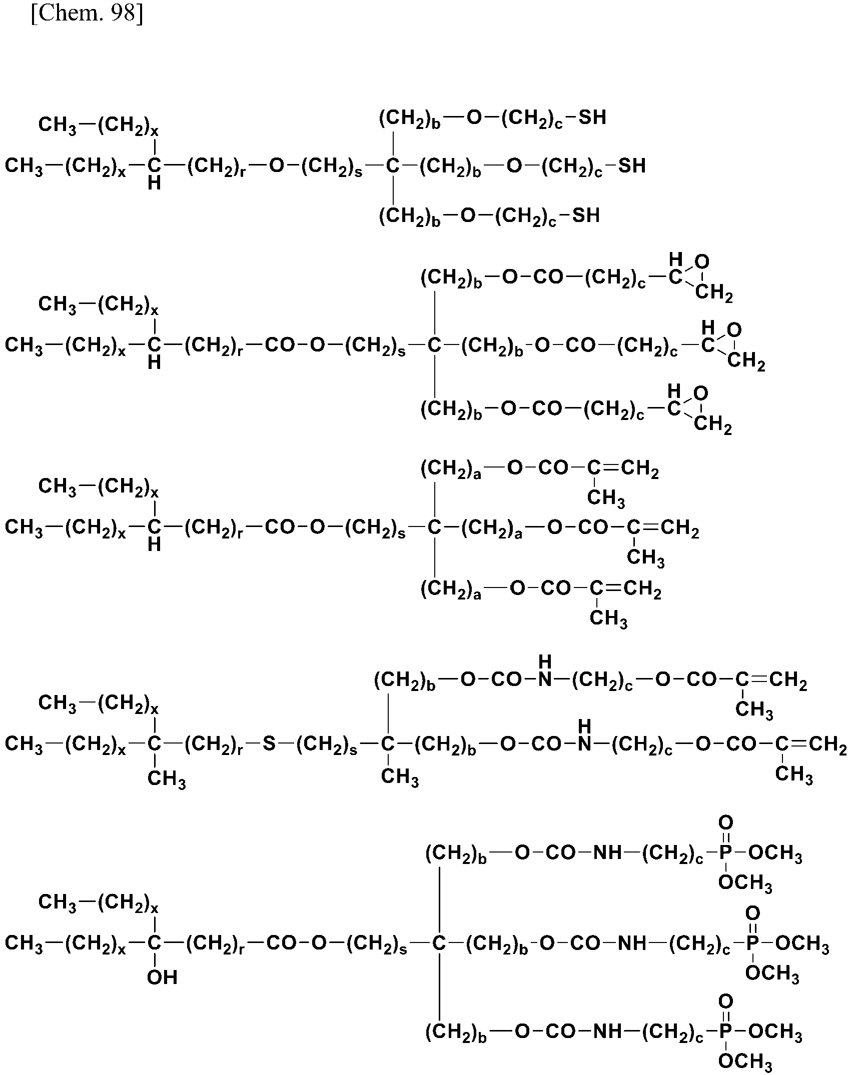

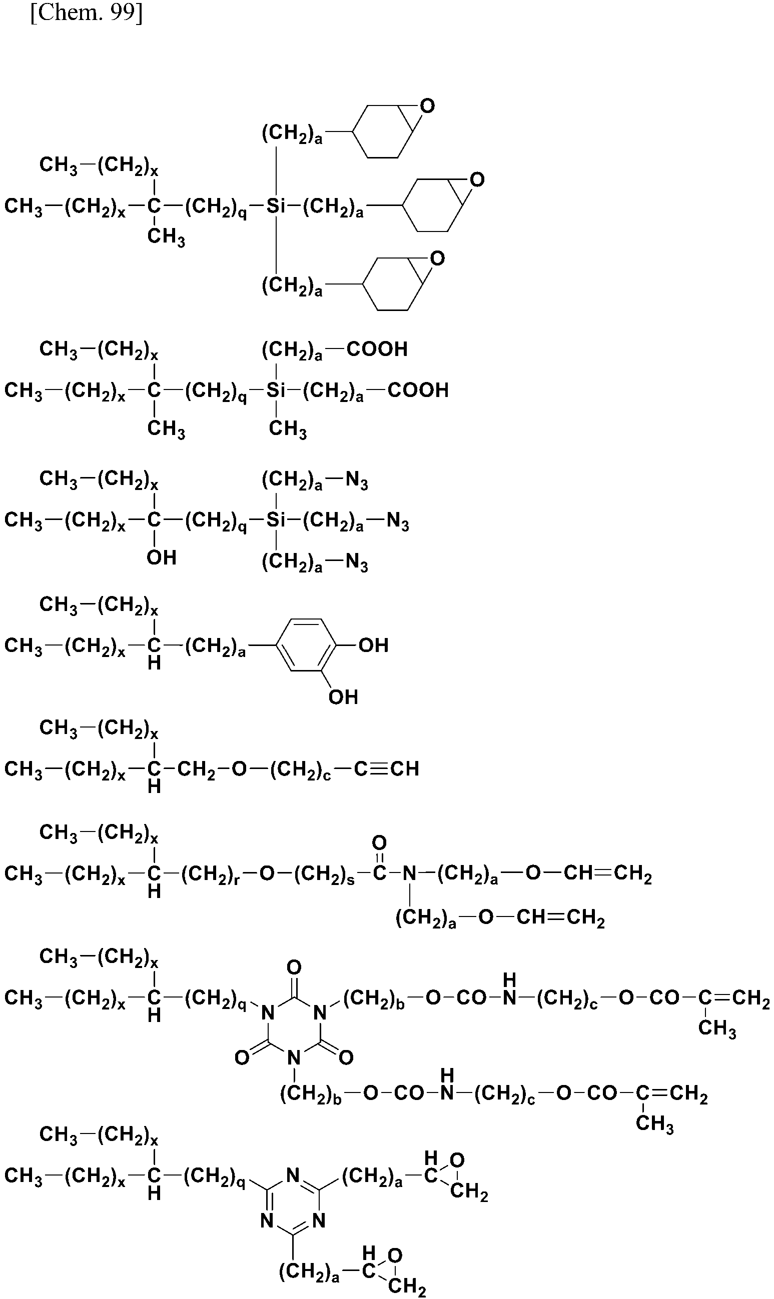

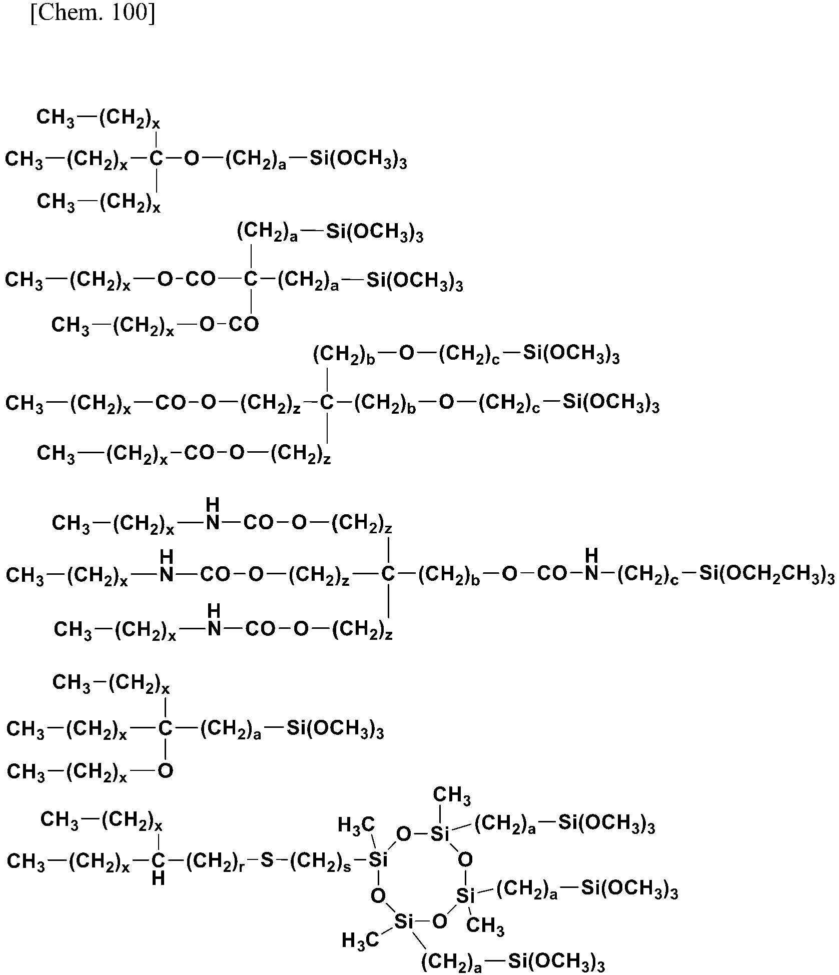

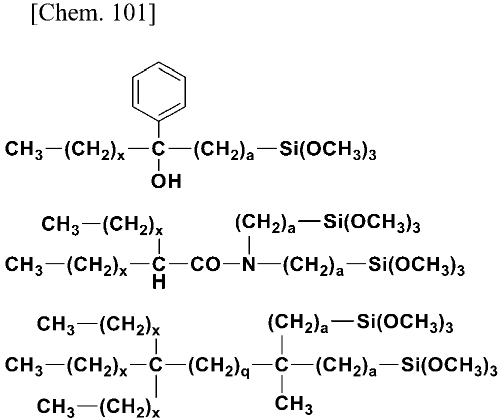

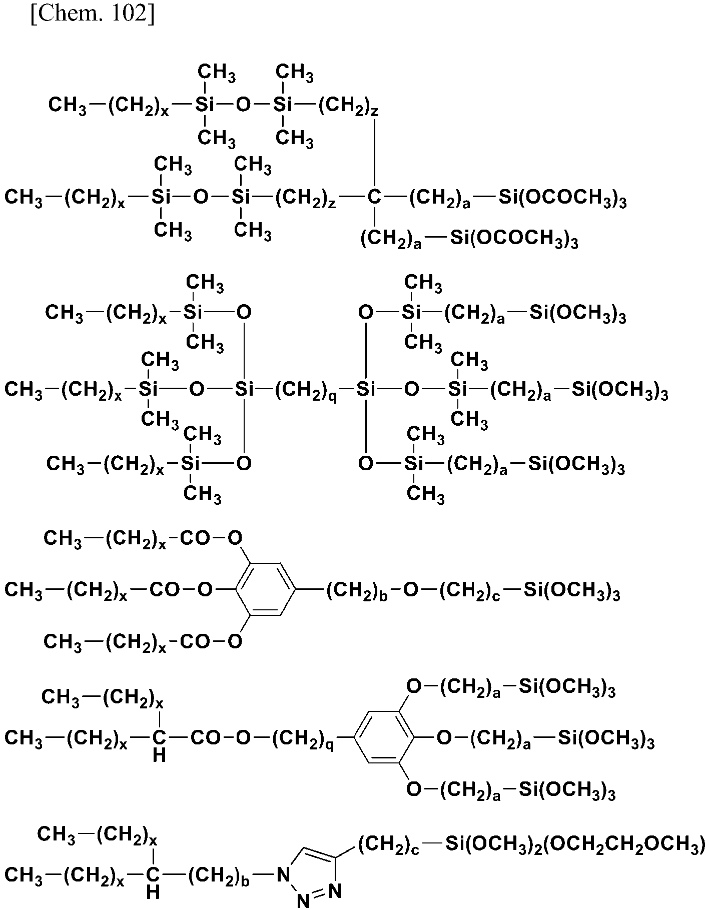

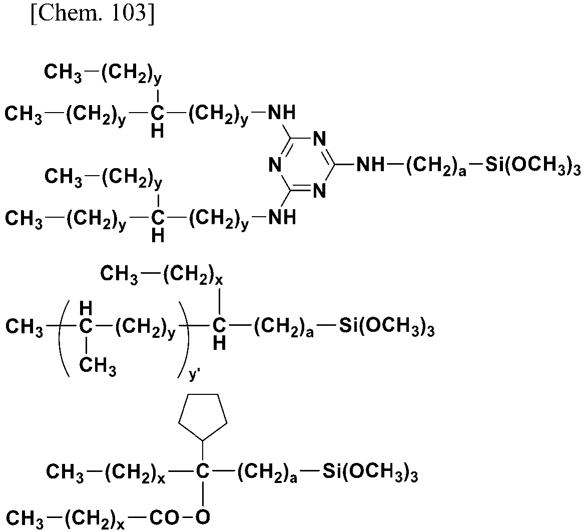

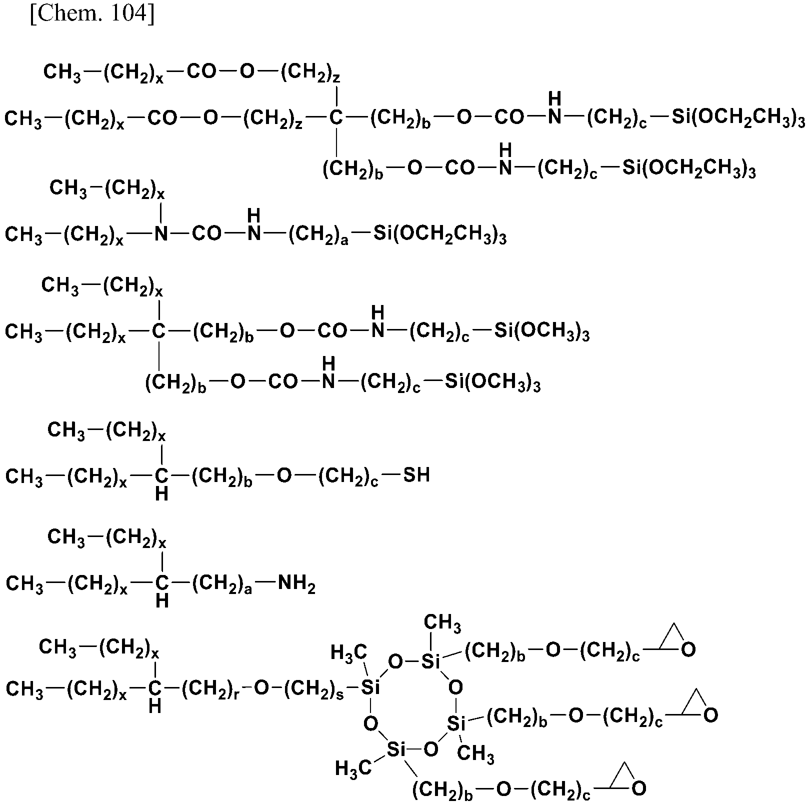

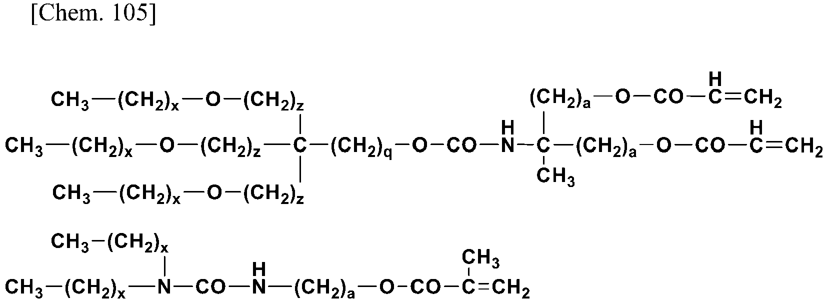

Herein x, y, z, q, r, s, a, b, c, d, e and f are independently as defined above.

Examples of the method for preparing the hydrocarbon end group-containing compound having formula (1) are described below.

### [Preparation method 1]

The hydrocarbon end group-containing compound having formula (1), specifically a compound terminated with a hydrolyzable silyl group can be prepared by mixing a hydrocarbon end group-containing compound having an alkenyl group at one end with a compound having a SiH group and a hydrolyzable silyl group and effecting hydrosilation addition reaction in the presence of a hydrosilation reaction catalyst. Notably, when the compound having a SiH group and a hydrolyzable silyl group is a compound wherein the hydrolyzable group is halogen, the desired compound can be prepared by cnverting the substituent group (halogen) on the silyl group to another hydrolyzable group.

Exemplary of the hydrocarbon end group-containing compound having an alkenyl group at one end is a compound having the formula (1A).

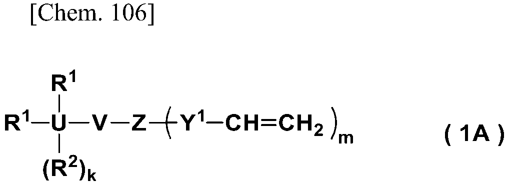

Herein R¹, R², U, V, Z, k and m are as defined above. Y¹ is independently a divalent hydrocarbon group of preferably 1 to 18 carbon atoms, which may contain at least one element selected from oxygen, nitrogen, sulfur and silicon.

In formula (1A), Y¹ is independently a divalent hydrocarbon group of preferably 1 to 18 carbon atoms, which may contain at least one element selected from oxygen, nitrogen, sulfur and silicon, examples of which are shown below. In the structure shown below, the valence bond on the left is attached to Z and the valence bond on the right is attached to the carbon atom.

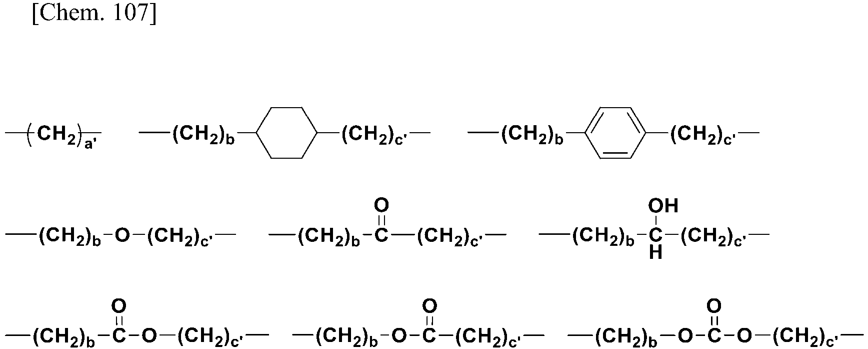

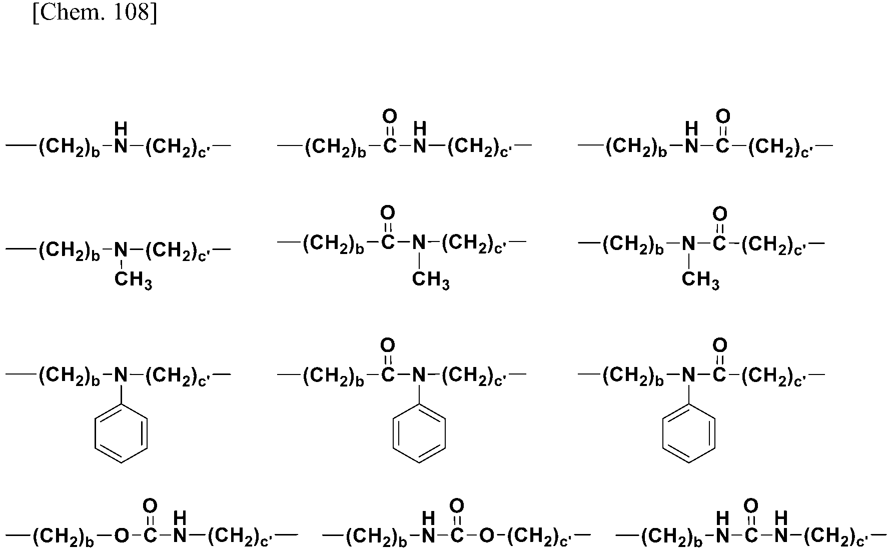

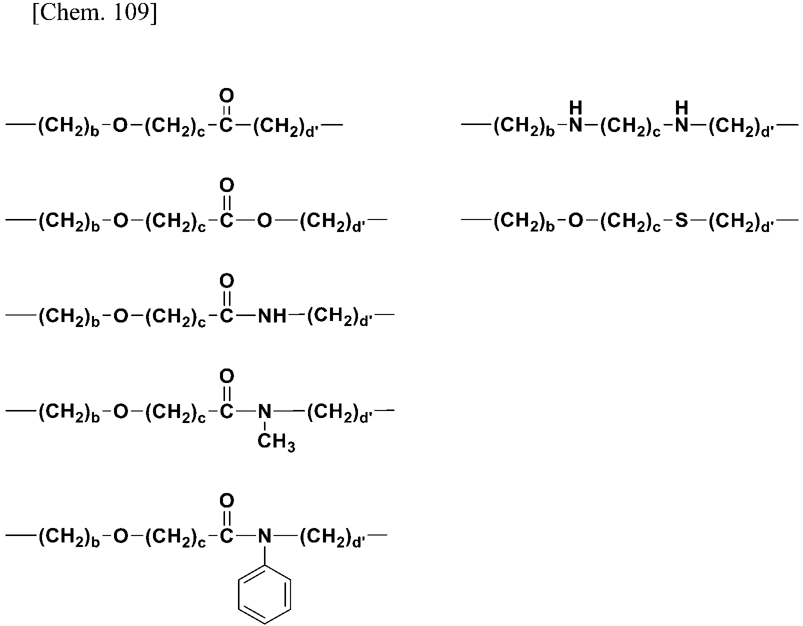

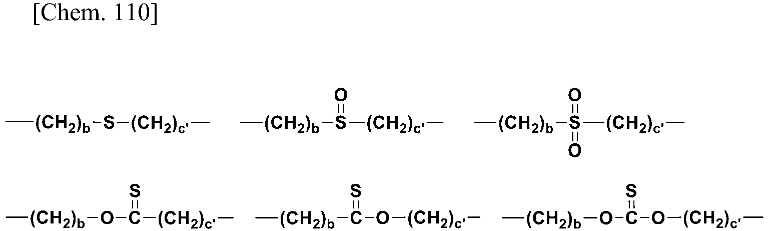

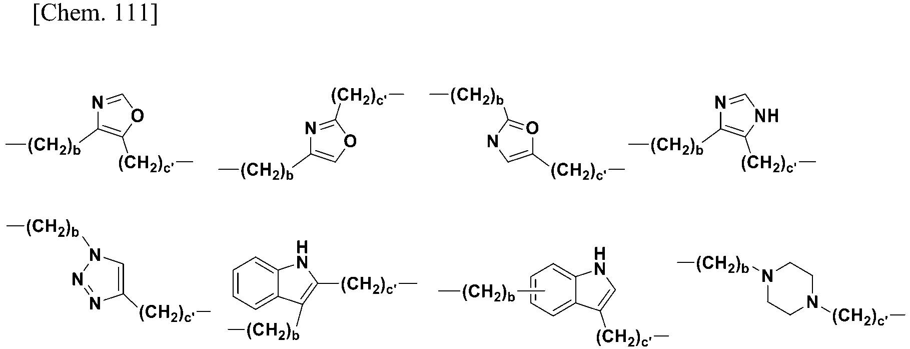

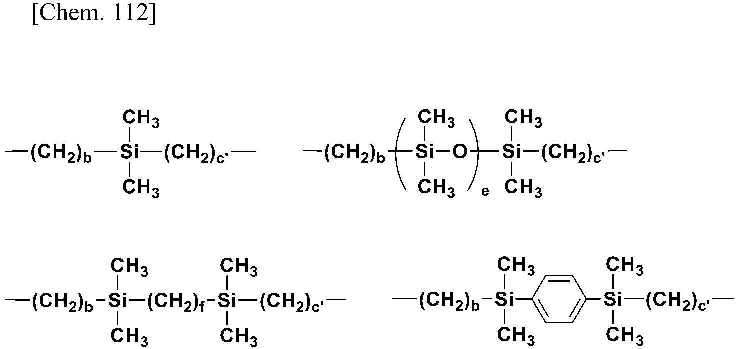

Herein a' is independently an integer of 0 to 8, b and c each are an integer of 1 to 8, c' and d' each are an integer of 0 to 6, the sum of b and c' is an integer of 2 to 8, the sum of b, c and d' is an integer of 3 to 8, e is an integer of 1 to 9, and f is an integer of 2 to 4.

Examples of the compound having formula (1A) are shown below.

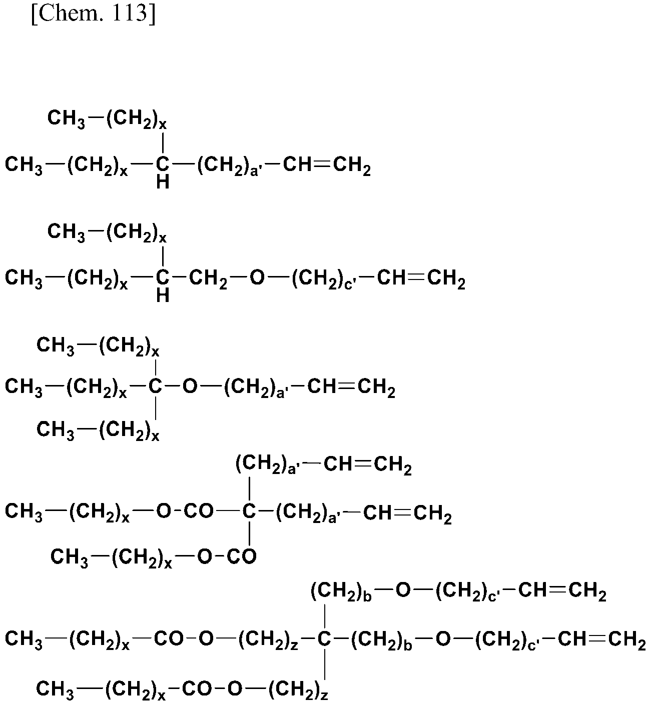

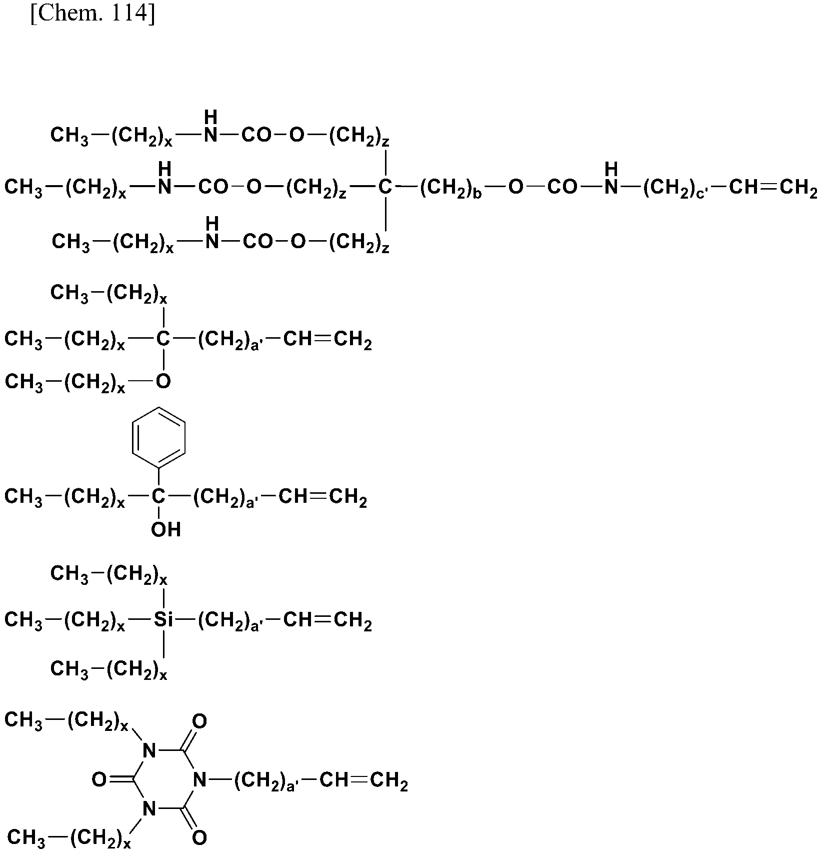

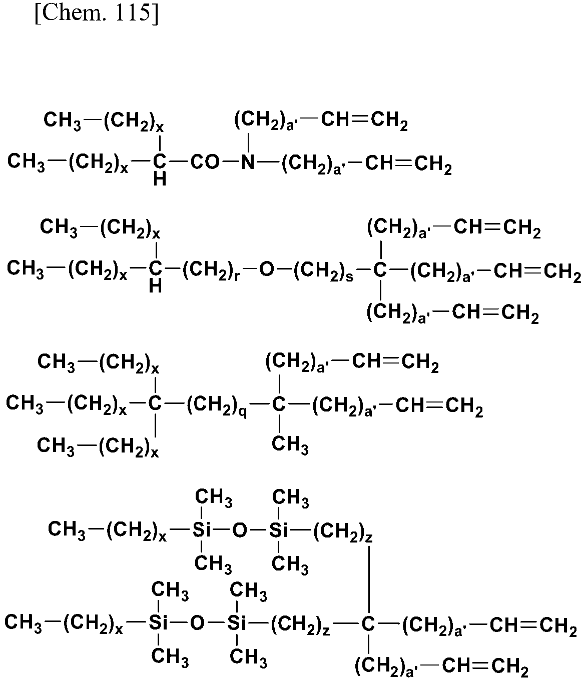

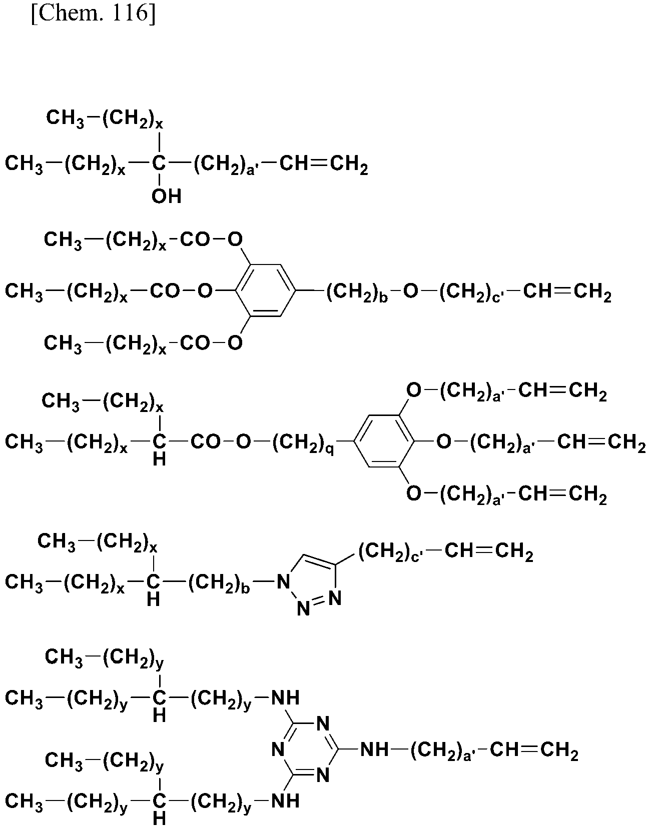

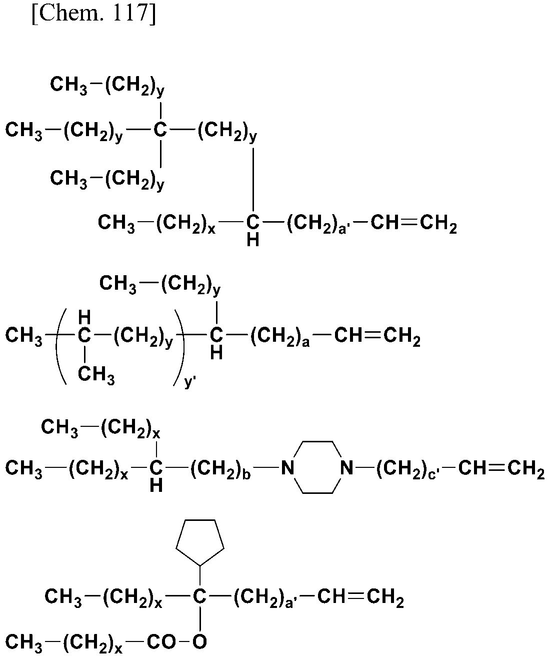

Herein x, y, z, q, a, a', b, c, and c' are independently as defined above.

Exemplary of the compound having a SiH group and a hydrolyzable silyl group are trimethoxysilane, triethoxysilane, triacetoxysilane, and trichlorosilane.

In Preparation Method 1, the amount of the compound having a SiH group and a hydrolyzable silyl group is preferably 1 to 6 mol, more preferably 1.5 to 4 mol per mol of alkenyl group in the hydrocarbon end group-containing compound having an alkenyl group at one end.

In Preparation Method 1, exemplary of the hydrosilation reaction catalyst are platinum group metal based catalysts including platinum black, chloroplatinic acid, alcohol-modified products of chloroplatinic acid, complexes of chloroplatinic acid with olefins, aldehydes, vinylsiloxanes, and acetylene alcohols, tetrakis(triphenylphosphine)palladium, and chlorotris(triphenylphosphine)rhodium. Inter alia, platinum based compounds such as vinylsiloxane coordination compounds are preferred. Notably, the platinum based compound is preferably used as a solution in a solvent such as toluene, lower alcohols, higher alcohols or silicones.

The amount of the hydrosilation reaction catalyst used is preferably to provide 0.001 to 1,000 ppm, more preferably 0.01 to 100 ppm by weight of transition metal based on the weight of the hydrocarbon end group-containing compound having an alkenyl group at one end.

In Preparation Method 1, a solvent may be used in carrying out the reaction. Examples of the solvent include aromatic hydrocarbons such as toluene and xylene, aliphatic or alicyclic hydrocarbons such as n-pentane, n-hexane and cyclohexane, cyclic ethers such as tetrahydrofuran and dioxane, and ketones such as acetone and methyl ethyl ketone.

The amount of the solvent used is preferably 0 to 1,000 parts by weight, more preferably 50 to 200 parts by weight per 100 parts by weight of the hydrocarbon end group-containing compound having an alkenyl group at one end.

In Preparation Method 1, the preferred conditions for the reaction of the hydrocarbon end group-containing compound having an alkenyl group at one end with the compound having a SiH group and a hydrolyzable silyl group include a temperature of 20 to 120°C, especially 60 to 100°C, and a time of 0.5 to 72 hours, especially 1 to 36 hours.

In one example of Preparation Method 1 wherein the compound having a SiH group and a hydrolyzable silyl group is a compound whose hydrolyzable group is halogen (i.e., compound having a SiH group and a halogenated silyl group), typically trichlorosilane, after the reaction, the substituent group (i.e., halogen) on the silyl group is converted to another hydrolyzable group, for example, alkoxy group, specifically methoxy. Examples of the compound which can be used in converting the substituent group (i.e., halogen) on the silyl group to another hydrolyzable group include methanol, ethanol, isopropanol, ethylene glycol, monomethyl ether and trimethyl orthoformate.

The amount of the compound used is preferably 3 to 9 mol, more preferably 3 to 5 mol per mol of halogen in the reaction product of the hydrocarbon end group-containing compound having an alkenyl group at one end with the compound having a SiH group and a halogenated silyl group

The preferred conditions for the reaction of converting the substituent group (i.e., halogen) on the silyl group to another hydrolyzable group include a temperature of 0 to 80°C, especially 20 to 60°C, and a time of 0.5 to 72 hours, especially 1 to 36 hours.

Another example of the method for preparing the hydrocarbon end group-containing compound having formula (1) is described below.

### [Preparation method 2]

The hydrocarbon end group-containing compound having formula (1), specifically a compound terminated with a hydrolyzable silyl group can be prepared by mixing a hydrocarbon end group-containing compound having a SiH group at one end with a compound having an alkenyl group and a hydrolyzable silyl group and effecting hydrosilation addition reaction in the presence of a hydrosilation reaction catalyst.

Exemplary of the hydrocarbon end group-containing compound having a SiH group at one end are compounds having the formulae (1B) and (1C): 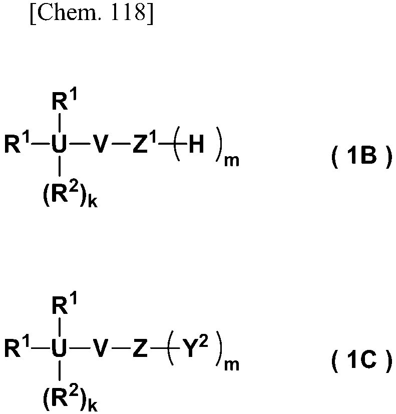 wherein R¹, R², U, V, Z, k and m are as defined above, Z¹ is a straight, trivalent to octavalent organopolysiloxane residue of 2 to 10 silicon atoms, or branched or cyclic, trivalent to octavalent organopolysiloxane residue of 3 to 10 silicon atoms, and Y² is independently a monovalent hydrocarbon group having silicon or a siloxane bond and a SiH group at one end.

In formula (1B), Z¹ is a tri- to octavalent straight organopolysiloxane residue of 2 to 10, especially 2 to 8 silicon atoms, or tri- to octavalent branched or cyclic organopolysiloxane residue of 3 to 10, especially 3 to 8 silicon atoms, examples of which are shown below. In the structure shown below, the valence bond on the left is attached to V and the valence bond on the right is attached to H.

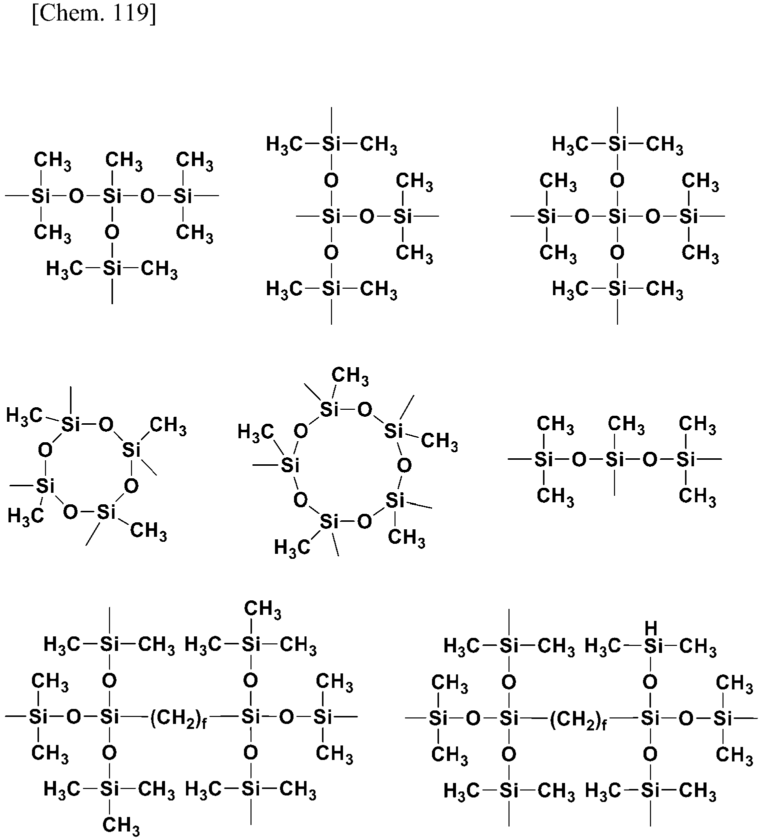

Herein f is an integer of 2 to 4.

Examples of the compound having formula (1B) are shown below.

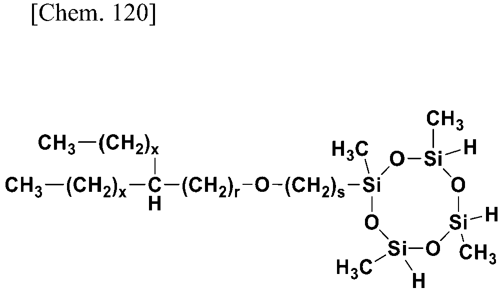

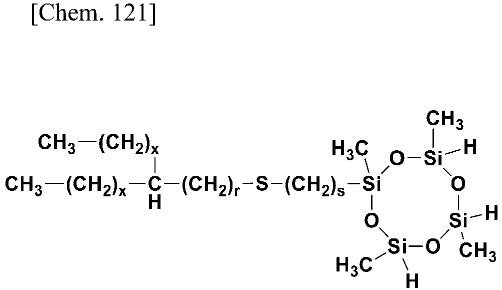

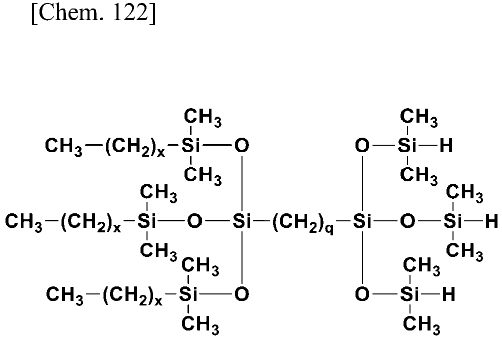

Herein x, q, r and s are each independently as defined above.

In formula (1C), Y² is independently a monovalent hydrocarbon group having silicon or a siloxane bond and a SiH group at one end, examples of which are shown below.

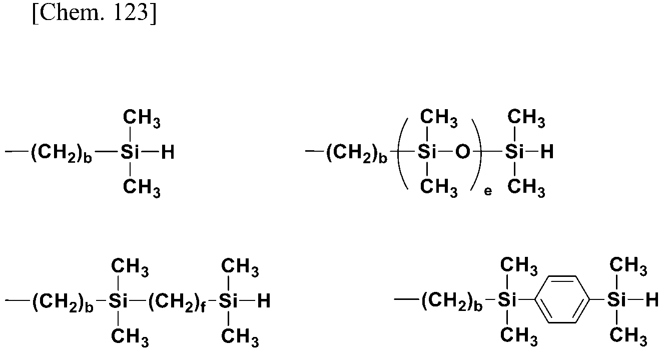

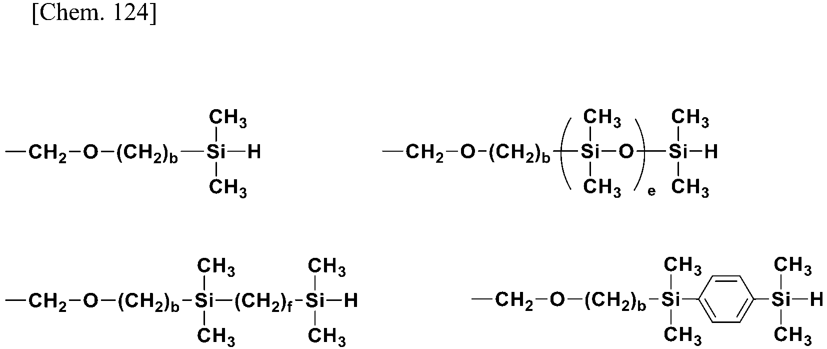

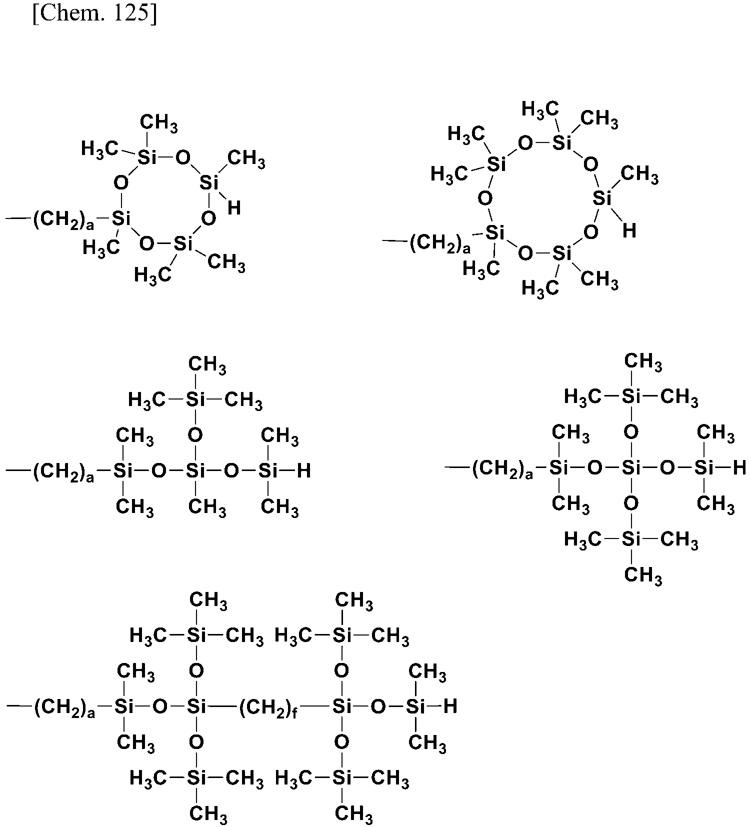

Herein a is an integer of 1 to 10, b is an integer of 1 to 8, e is an integer of 1 to 9, and f is an integer of 2 to 4.

Examples of the compound having formula (1C) are shown below.

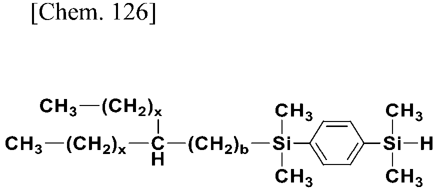

Herein x and b are each independently as defined above.

Of the compounds having an alkenyl group and a reactive group, typically a hydrolyzable silyl group, examples of the compound having an alkenyl group and a hydrolyzable silyl group include vinyltrimethoxysilane, allyltrimethoxysilane, and octenyltrimethoxysilane.

Exemplary of the compound having an alkenyl group and a reactive group other than a hydrolyzable silyl group is allyl glycidyl ether.

In Preparation Method 2, the amount of the compound having an alkenyl group and a reactive group, typically a hydrolyzable silyl group is preferably 1 to 5 mol, more preferably 1 to 3 mol per mol of SiH group in the hydrocarbon end group-containing compound having a SiH group at one end.

In Preparation Method 2, the hydrosilation reaction catalyst is as exemplified above for Preparation Method 1. Platinum based compounds such as vinylsiloxane coordination compounds are preferred. Notably, the platinum based compound is preferably used as a solution in a solvent such as toluene, lower alcohols, higher alcohols or silicones.

The amount of the hydrosilation reaction catalyst used is preferably to provide 0.001 to 1,000 ppm, more preferably 0.01 to 100 ppm by weight of transition metal based on the weight of the hydrocarbon end group-containing compound having a SiH group at one end.

In Preparation Method 2, a solvent may be used in carrying out the reaction. The solvent is as exemplified above for Preparation Method 1.

The amount of the solvent used is preferably 0 to 1,000 parts by weight, more preferably 50 to 200 parts by weight per 100 parts by weight of the hydrocarbon end group-containing compound having a SiH group at one end.

In Preparation Method 2, the preferred reaction conditions include a temperature of 20 to 120°C, especially 60 to 100°C, and a time of 0.5 to 72 hours, especially 1 to 36 hours.

A further example of the method for preparing the hydrocarbon end group-containing compound having formula (1) is described below.

### [Preparation Method 3]

The hydrocarbon end group-containing compound having formula (1), specifically a polysilazane compound terminated with an amino-containing silyl group and/or a polymer thereof can be prepared by mixing a hydrocarbon end group-containing compound having an alkenyl group at one end with trichlorosilane, effecting reaction in the presence of a hydrosilation reaction catalyst, and reacting the reaction product with ammonia gas.

The reaction product of the hydrocarbon end group-containing compound having an alkenyl group at one end with trichlorosilane can be prepared as in Preparation Method 1.

In Preparation Method 3, the amount of ammonia gas used is preferably 1 to 300 cc/min, more preferably 30 to 200 cc/min.

In Preparation Method 3, the preferred conditions for the reaction of the reaction product of the hydrocarbon end group-containing compound having an alkenyl group at one end and trichlorosilane with ammonia gas include room temperature (23±15°C, the same holds true, hereinafter), especially 20 to 30°C, and a time of 2 to 36 hours, especially 4 to 12 hours.

A still further example of the method for preparing the hydrocarbon end group-containing compound having formula (1) is described below.

### [Preparation Method 4]

The hydrocarbon end group-containing compound having formula (1), specifically a compound having a reactive group (e.g., hydrolyzable silyl group or (meth)acryloyloxy group) bonded at one end via a urethane bond can be prepared by mixing a hydrocarbon end group-containing compound having a hydroxy group at one end with a compound having an isocyanate group and a reactive group (e.g., hydrolyzable silyl group or (meth)acryloyloxy group), and effecting reaction in the presence of a catalyst. As used herein, the (meth)acryloyloxy group designates acryloyloxy or methacryloyloxy group.

Exemplary of the hydrocarbon end group-containing compound having a hydroxy group at one end are compounds having the formulae (1D) and (1E): 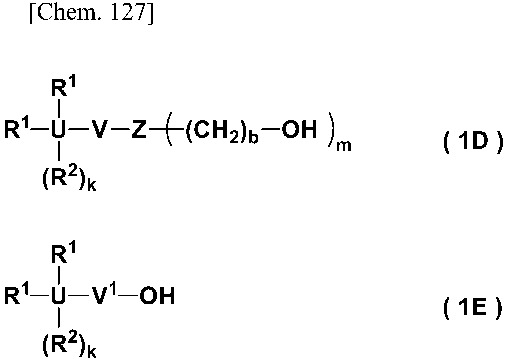 wherein R¹, R², U, V, Z, k, m and b are as defined above, and V¹ is a C₁-C₁₀ divalent hydrocarbon group.

Examples of the compound having formula (1D) are shown below.

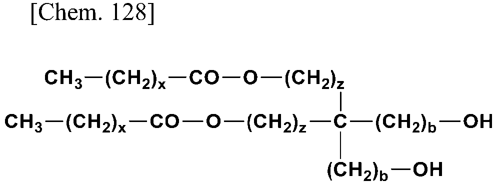

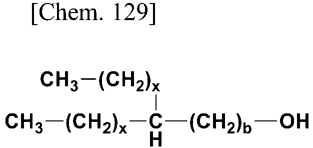

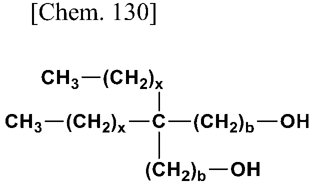

Herein x, z and b are each independently as defined above.

In formula (1E), V¹ is a C₁-C₁₀ divalent hydrocarbon group, preferably alkylene group, examples of which are shown below.

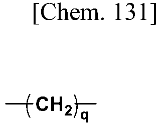

Herein q is as defined above.

Examples of the compound having formula (1E) are shown below.

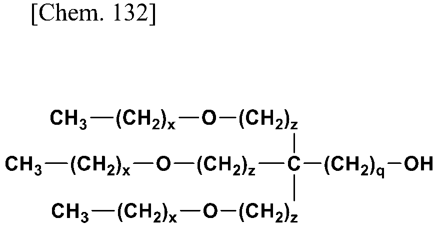

Herein x, z and q are each independently as defined above.

Examples of the compound having an isocyanate group and a reactive group include (3-isocyanatopropyl)trimethoxysilane, (3-isocyanatopropyl)triethoxysilane, and 1,1-(bisacryloyloxymethyl)ethyl isocyanate.

In Preparation Method 4, the amount of the compound having an isocyanate group and a reactive group used is preferably 1 to 3 mol, more preferably 1 to 1.5 mol per mol of hydroxy group in the hydrocarbon end group-containing compound having a hydroxy group at one end.

In Preparation Method 4, examples of the catalyst include titanium compounds such as titanium tetra-2-ethylhexoxide, tetra-n-butyl titanate, and tetra-n-propyl titanate, zirconium compounds such as tetra-n-butyl zirconate and tetra-n-propyl zirconate, tin compounds such as dibutyltin dimethoxide and dibutyltin dilaurate, bismuth compounds such as bismuth tris(2-ethylhexanoate), and amine catalysts such as diazabicycloundecene.

The amount of the catalyst used is preferably 0.01 to 100 parts by weight, more preferably 0.1 to 20 parts by weight per 100 parts by weight of the hydrocarbon end group-containing compound having a hydroxy group at one end.

In Preparation Method 4, a solvent may be used in carrying out the reaction. The solvent is as exemplified above for Preparation Method 1.

The amount of the solvent used is preferably 0 to 1,000 parts by weight, more preferably 50 to 200 parts by weight per 100 parts by weight of the hydrocarbon end group-containing compound having a hydroxy group at one end.

In Preparation Method 4, the preferred reaction conditions include a temperature of 20 to 100°C, especially 30 to 60°C, and a time of 0.5 to 72 hours, especially 1 to 36 hours.

A yet further example of the method for preparing the hydrocarbon end group-containing compound having formula (1) is described below.

### [Preparation Method 5]

The hydrocarbon end group-containing compound having formula (1), specifically a compound having a reactive group (e.g., phosphate group) at one end can be prepared by mixing a hydrocarbon end group-containing compound having a hydroxy group at one end with phosphorus oxychloride, reacting them, then adding water, and effecting reaction.

Examples of the hydrocarbon end group-containing compound having a hydroxy group at one end are as exemplified above for the compound having formula (1D) in Preparation Method 4.

In Preparation Method 5, the amount of phosphorus oxychloride used is preferably 1 to 4 mol, more preferably 1 to 2 mol per mol of hydroxy group in the hydrocarbon end group-containing compound having a hydroxy group at one end.

In Preparation Method 5, a solvent may be used in carrying out the reaction of the hydrocarbon end group-containing compound having a hydroxy group at one end with phosphorus oxychloride. The solvent is as exemplified above for Preparation Method 1.

The amount of the solvent used is preferably 0 to 1,000 parts by weight, more preferably 50 to 200 parts by weight per 100 parts by weight of the hydrocarbon end group-containing compound having a hydroxy group at one end.

In Preparation Method 5, the preferred conditions for the reaction of the hydrocarbon end group-containing compound having a hydroxy group at one end with phosphorus oxychloride include a temperature of 0 to 80°C, especially 15 to 50°C, and a time of 0.5 to 72 hours, especially 1 to 36 hours.

In Preparation Method 5, the amount of water used is preferably 50 to 1,000 parts by weight, more preferably 100 to 500 parts by weight per 100 parts by weight of the hydrocarbon end group-containing compound having a hydroxy group at one end.

In Preparation Method 5, the preferred conditions for the reaction of the reaction product of the hydrocarbon end group-containing compound having a hydroxy group at one end and phosphorus oxychloride with water include a temperature of 0 to 80°C, especially 15 to 50°C, and a time of 0.5 to 72 hours, especially 1 to 36 hours.

In an alternative method [Preparation Method 5'], the hydrocarbon end group-containing compound having formula (1), specifically a compound having a reactive group (e.g., phosphate ester group) at one end can be prepared by mixing a hydrocarbon end group-containing compound having a leaving group at one end with a trialkyl phosphite, and reacting them.

Examples of the hydrocarbon end group-containing compound having a leaving group at one end are as will be exemplified later for the compounds having formulae (1G) and (1H) in Preparation Method 8.

In [Preparation Method 5'], examples of the trialkyl phosphite include trimethyl phosphite and triethyl phosphite. The amount of the trialkyl phosphite used is preferably 1 to 8 mol, more preferably 1 to 4 mol per mol of leaving group in the hydrocarbon end group-containing compound having a leaving group at one end.

In [Preparation Method 5'], a solvent may be used in carrying out the reaction of the hydrocarbon end group-containing compound having a leaving group at one end with a trialkyl phosphite. The solvent is as exemplified above for Preparation Method 1.

The amount of the solvent used is preferably 0 to 1,000 parts by weight, more preferably 0 to 200 parts by weight per 100 parts by weight of the hydrocarbon end group-containing compound having a leaving group at one end.

In [Preparation Method 5'], the preferred conditions for the reaction of the hydrocarbon end group-containing compound having a leaving group at one end with a trialkyl phosphite include a temperature of 25 to 160°C, especially 80 to 140°C, and a time of 0.5 to 72 hours, especially 1 to 36 hours.

A yet further example of the method for preparing the hydrocarbon end group-containing compound having formula (1) is described below.

### [Preparation Method 6]

The hydrocarbon end group-containing compound having formula (1), specifically a compound having a reactive group (e.g., hydrolyzable silyl group or (meth)acryloyloxy group) bonded at one end via a urea bond can be prepared by mixing a hydrocarbon end group-containing compound having a NH group at one end with a compound having an isocyanate group and a reactive group (e.g., hydrolyzable silyl group or (meth)acryloyloxy group) and reacting them.

Exemplary of the hydrocarbon end group-containing compound having a NH group at one end are compounds having the formula (1F): 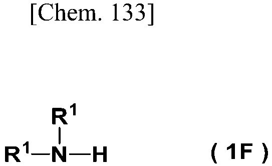 wherein R¹ is as defined above.

Examples of the compound having formula (1F) are shown below.

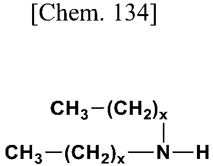

Herein x is independently as defined above.

Examples of the compound having an isocyanate group and a reactive group include (3-isocyanatopropyl)trimethoxysilane, (3-isocyanatopropyl)triethoxysilane, and 2-isocyanatoethyl methacrylate.

In Preparation Method 6, the amount of the compound having an isocyanate group and a reactive group used is preferably 1 to 3 mol, more preferably 1 to 1.5 mol per mol of the hydrocarbon end group-containing compound having a NH group at one end.

In Preparation Method 6, a solvent may be used in carrying out the reaction. The solvent is as exemplified above for Preparation Method 1.

The amount of the solvent used is preferably 0 to 1,000 parts by weight, more preferably 50 to 200 parts by weight per 100 parts by weight of the hydrocarbon end group-containing compound having a NH group at one end.

In Preparation Method 6, the preferred reaction conditions include a temperature of 0 to 100°C, especially 20 to 60°C, and a time of 0.5 to 72 hours, especially 1 to 36 hours.

A yet further example of the method for preparing the hydrocarbon end group-containing compound having formula (1) is described below.

### [Preparation Method 7]

The hydrocarbon end group-containing compound having formula (1), specifically a compound having a thiol group (e.g., mercapto group) at one end can be prepared by mixing a hydrocarbon end group-containing compound having an alkenyl group at one end with thioacetic acid, reacting them in the presence of a polymerization initiator, mixing the compound having a thioester group with a hydrosilane compound and reacting them in the presence of a Pd/C catalyst.

Examples of the hydrocarbon end group-containing compound having an alkenyl group at one end are as exemplified above for the compound having formula (1A) in Preparation Method 1.

In Preparation Method 7, the amount of thioacetic acid used is preferably 1 to 5 mol, more preferably 1 to 3 mol per mol of alkenyl group in the hydrocarbon end group-containing compound having an alkenyl group at one end.

In Preparation Method 7, examples of the polymerization initiator include azo compounds such as 2,2'-azobisisobutyronitrile, diacyl peroxides such as benzoyl peroxide and lauroyl peroxide, dialkyl peroxides such as dicumyl peroxide and di-tert-butyl peroxide, peroxycarbonates such as diisopropyl peroxydicarbonate and bis(4-tert-butylcyclohexyl) peroxydicarbonate, and alkyl peresters such as tert-butyl peroxyoctoate and tert-butyl peroxybenzoate.

The amount of the polymerization initiator used is preferably 0.01 to 3 mol, more preferably 0.1 to 1.5 mol per mol of alkenyl group in the hydrocarbon end group-containing compound having an alkenyl group at one end.

In Preparation Method 7, the preferred conditions for the reaction of the hydrocarbon end group-containing compound having an alkenyl group at one end with thioacetic acid include a temperature of 20 to 100°C, especially 40 to 80°C, and a time of 0.5 to 72 hours, especially 1 to 36 hours.

In Preparation Method 7, examples of the hydrosilane compound include trialkoxysilanes such as triethylsilane.

The amount of the hydrosilane compound used is preferably 1 to 6 mol, more preferably 1.5 to 4 mol per mol of thioester group in the reaction product of a hydrocarbon end group-containing compound having an alkenyl group at one end with thioacetic acid (i.e., compound having a thioester group).

In Preparation Method 7, the amount of the Pd/C catalyst used is preferably to provide 0.001 to 1 mol, more preferably 0.01 to 0.5 mol of Pd per mol of thioester group in the reaction product of a hydrocarbon end group-containing compound having an alkenyl group at one end with thioacetic acid (i.e., compound having a thioester group).

In Preparation Method 7, the preferred conditions for the reaction of the reaction product of a hydrocarbon end group-containing compound having an alkenyl group at one end and thioacetic acid (i.e., compound having a thioester group) with a hydrosilane compound include a temperature of 20 to 100°C, especially 40 to 80°C, and a time of 0.5 to 72 hours, especially 1 to 36 hours.

In Preparation Method 7, a solvent may be used in carrying out the reaction. The solvent is as exemplified above for Preparation Method 1.

The amount of the solvent used is preferably 0 to 1,000 parts by weight, more preferably 50 to 200 parts by weight per 100 parts by weight of the hydrocarbon end group-containing compound having an alkenyl group at one end, or the reaction product of a hydrocarbon end group-containing compound having an alkenyl group at one end with thioacetic acid (i.e., compound having a thioester group).

A yet further example of the method for preparing the hydrocarbon end group-containing compound having formula (1) is described below.

### [Preparation Method 8]

The hydrocarbon end group-containing compound having formula (1), specifically a compound having an amino group at one end can be prepared by reacting a hydrocarbon end group-containing compound having a leaving group (e.g., halogen or sulfonyl ester group) at one end with an amidating reagent.

Exemplary of the hydrocarbon end group-containing compound having a leaving group are compounds having the formulae (1G) and (1H): 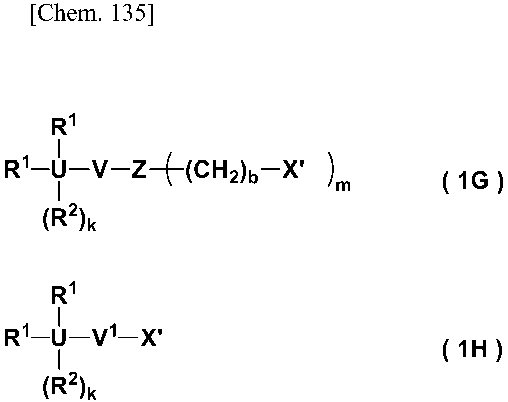 wherein R¹, R², U, V, V¹, Z, k, m, and b are as defined above, and X' is halogen or sulfonyl ester group.

Examples of the compound having formula (1G) are shown below.

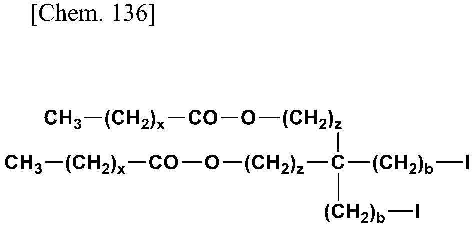

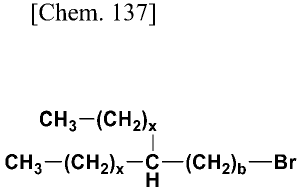

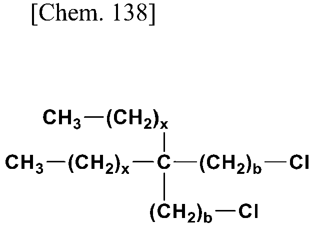

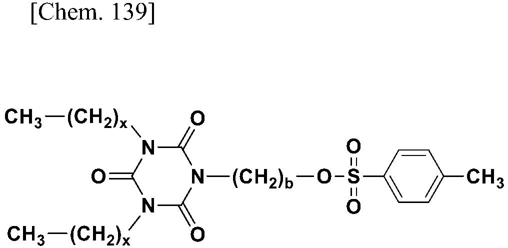

Herein x, z, and b are each independently as defined above.

Examples of the compound having formula (1H) are shown below.

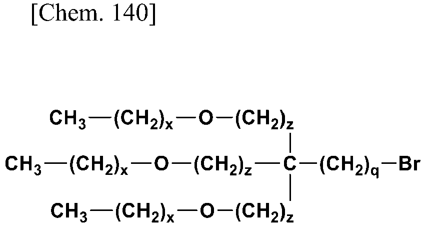

Herein x, z, and q are each independently as defined above.

Examples of the amidating reagent include phthalimide, potassium phthalimide and sodium phthalimide.

In Preparation Method 8, the amount of the amidating reagent used is preferably 1 to 6 mol, more preferably 1.5 to 3 mol per mol of leaving group in the hydrocarbon end group-containing compound having a leaving group at one end.

In Preparation Method 8, a solvent may be used in carrying out the reaction. Examples of the solvent include aromatic hydrocarbons such as toluene and xylene, aliphatic or alicyclic hydrocarbons such as n-pentane, n-hexane and cyclohexane, cyclic ethers such as tetrahydrofuran and dioxane, ketones such as acetone and methyl ethyl ketone, amides such as dimethylformamide and dimethylacetamide, and alcohols such as methanol, ethanol, and isopropanol.

The amount of the solvent used is preferably 0 to 1,000 parts by weight, more preferably 50 to 300 parts by weight per 100 parts by weight of the hydrocarbon end group-containing compound having a leaving group at one end.

In Preparation Method 8, the preferred conditions for the reaction of the hydrocarbon end group-containing compound having a leaving group at one end with the amidating reagent include a temperature of 40 to 150°C, especially 60 to 120°C, and a time of 0.5 to 72 hours, especially 1 to 36 hours.

In Preparation Method 8, when the amidating reagent is protected, deprotection is carried out. Typical of the compound which can be used for deprotection is hydrazine monohydrate.

The amount of the deprotecting compound is preferably 1 to 5 mol, more preferably 1 to 3 mol per mol of protected amino group in the reaction product of the hydrocarbon end group-containing compound having a leaving group at one end with the amidating reagent.

In Preparation Method 8, the preferred conditions for deprotection reaction include a temperature of 0 to 90°C, especially 20 to 70°C and a time of 0.5 to 72 hours, especially 1 to 36 hours.

Another embodiment of the invention is a substantially fluorine-free surface treating agent comprising the non-fluorinated (i.e., not containing fluorine in the molecule) hydrocarbon end group-containing compound having formula (1) as a main component. As long as surface treating agent contains the hydrocarbon end group-containing compound having formula (1) as a main component, it may contain an unreacted reactant prior to the introduction of a reactive group in the hydrocarbon end group-containing compound having formula (1) or a reaction intermediate. The surface treating agent preferably uses the hydrocarbon end group-containing compound wherein the reactive group is a hydrolyzable silyl group. In this case, a partial (hydrolytic) condensate obtained from partial hydrolysis of the hydrolyzable silyl group by a well-known technique may also be contained.

If necessary, a hydrolytic condensation catalyst may be added to the surface treating agent, which is selected from, for example, organotin compounds such as dibutyltin dimethoxide and dibutyltin dilaurate, organotitanium compounds such as tetra-n-butyl titanate and tetra-n-propyl titanate, organozirconium compounds such as tetra-n-butyl zirconate and tetra-n-propyl zirconate, organic acids such as acetic acid, methanesulfonic acid and carboxylic acid, mineral acids such as hydrochloric acid and sulfuric acid, and organic bases such as amine, trialkyl amines, and nitrogen-containing cyclic compounds. Of these, acetic acid, tetra-n-butyl titanate and dibutyltin dilaurate are desirable.

The amount of the hydrolytic condensation catalyst added is a catalytic amount, typically 0.001 to 5 parts by weight, especially 0.1 to 1 part by weight per 100 parts by weight of the hydrocarbon end group-containing compound (and/or a partial hydrolytic condensate thereof).

The surface treating agent may contain a suitable solvent. The solvent is desirably a non-fluorinated solvent. Preferred examples include hydrocarbon solvents such as petroleum benzine, toluene, xylene, hexane, cyclohexane, methylcyclohexane, ethylcyclohexane, heptane, octane (n-octane or isooctane), and nonane (n-nonane or isononane), ketone solvents such as acetone, methyl ethyl ketone, methyl isobutyl ketone, cyclopentanone, and cyclohexanone, ether solvents such as tetrahydrofuran (THF), dipropyl ether, dibutyl ether, methyl cyclopentyl ether, methyl tert-butyl ether, ethylene glycol dimethyl ether, diethylene glycol dimethyl ether, triethylene glycol dimethyl ether, and propylene glycol dimethyl ether, alcohol solvents such as propylene glycol monomethyl ether, butanol and isopropanol, and ester solvents such as ethyl acetate, propyl acetate, butyl acetate, pentyl acetate, and propylene glycol monomethyl ether acetate. Of these, preference is given from the aspects of solubility and wettability to toluene, hexane, heptane, isooctane, isononane, cyclopentanone, dipropyl ether, dibutyl ether, methyl cyclopentyl ether, methyl tert-butyl ether, ethylene glycol dimethyl ether, propyl acetate, butyl acetate, and propylene glycol monomethyl ether acetate.

The solvents may be used in admixture of two or more. Preferably, the hydrocarbon end group-containing compound (and partial hydrolytic condensate thereof) is uniformly dissolved in the solvent. When the hydrocarbon end group-containing compound (and partial hydrolytic condensate thereof) is dissolved in the solvent, the optimum concentration thereof varies with a particular treating method and is preferably an amount which can be readily metered. In the case of direct coating, the concentration is preferably 0.01 to 100 parts by weight, more preferably 0.05 to 30 parts by weight per 100 parts by weight of the total of the solvent and the hydrocarbon end group-containing compound (and partial hydrolytic condensate thereof). In the case of evaporation treatment, the concentration is preferably 1 to 100 parts by weight, more preferably 3 to 30 parts by weight per 100 parts by weight of the total of the solvent and the hydrocarbon end group-containing compound (and partial hydrolytic condensate thereof). In either coating, the concentration of 100 parts by weight means direct coating without the aid of the solvent.

The surface treating agent may be applied to a substrate by any of well-known methods such as brush coating, dipping, spraying and evaporation. In the case of evaporation treatment, the heating means may be either resistance heating or electron beam heating and is not critical. The curing temperature, which varies with the curing mode, is preferably 25 to 200°C, especially 25 to 150°C for 30 minutes to 36 hours, especially 1 to 24 hours in the case of direct coating (e.g., brush coating, dipping or spraying), and 20 to 200°C for 1 to 24 hours in the case of evaporation treatment. Also, the surface treating agent may be cured in moist atmosphere.

In an example wherein the hydrocarbon end group-containing compound having a hydrolyzable silyl group is used and spray coated, it is recommended that the hydrocarbon end group-containing compound is diluted with an organic solvent having water added previously, hydrolysis is carried out to form Si-OH, and thereafter spray coating is carried out. This is because the cure after coating is quick.

Although the thickness of a cured film is suitably chosen depending on the type of substrate, it is typically 0.1 to 100 nm, especially 1 to 20 nm. The film thickness is measured by any suitable means, for example, spectral reflectance measurement, X-ray reflectance measurement, spectral ellipsometry, and X-ray fluorescence measurement.

The substrate which is treated with the surface treating agent is not particularly limited and may be selected from a variety of materials including paper, textile, metals and metal oxides, glass, plastics, ceramics and quartz. The surface treating agent is effective for imparting water/oil repellency to the substrate. In particular, it is advantageously used for the surface treatment of SiO₂-coated glass and films.

The surface treating agent can form a cured film meeting a high level of water repellency, slipperiness, stain wipe-off, wear resistance and chemical resistance.

Examples of the article which is treated with the surface treating agent include optical articles and electronic parts, specifically car navigation systems, mobile phones, smartphones, digital cameras, digital video cameras, PDAs, portable audio players, car audio systems, game consoles, eyeglass lenses, camera lenses, lens filters, sunglass, medical instruments such as gastroscopes, copiers, personal computers, liquid crystal displays, organic EL displays, plasma displays, touch panel displays, protective films, and anti-reflective films. Since the surface treating agent can impart flaw resistance to the aforementioned articles, it is useful as a water repellent layer on touch panel displays, antireflective films, and eyeglass lenses.

The surface treating agent is also useful as antifouling coatings on sanitary wares such as bathtubs and washbowls, antifouling coatings on windowpanes or strengthened glass and headlamp covers of automobiles, trains and aircraft, water-repellent coatings on building exterior members, antifouling coatings on kitchen building members, antifouling and sticker/graffiti-proof coatings for telephone boxes, antifouling coatings on art works, antifouling coatings on compact disks and DVDs, parting agents to molds, paint additives, resin modifiers, flow improvers or dispersion modifiers for inorganic fillers, and lubricity improvers for tape and film.

### EXAMPLES

Synthesis Examples, Examples and Comparative Examples are given below for further illustrating the invention although the invention is not limited thereto. In Examples, the "mol" amount of a compound is the measured weight of the compound divided by the molecular weight of a polymer specified by ¹H-NMR analysis. The thickness of a film is measured by spectroscopic ellipsometry using a spectroscopic ellipsometer. Room temperature is 23°C.

### [Synthesis Example 1]

In a reactor, 1.00 g (2.96×10⁻³ mol) of a compound having the formula (A): 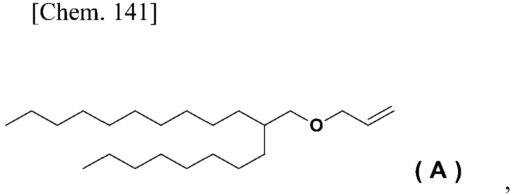 1.00 g of toluene, 1.08 g (8.87×10⁻³ mol) of trimethoxysilane, and 1.13×10⁻² g (containing 3.49×10⁻⁸ mol of Pt element) of a toluene solution of chloroplatinic acid/vinylsiloxane complex were mixed and aged at 80°C for 24 hours. Thereafter, the solvent and unreacted reactants were distilled off under reduced pressure, obtaining 1.30 g of a product.

Analysis by ¹H-NMR proved that the product had the structure represented by the following formula (B).

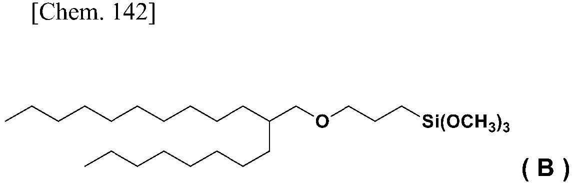

### [Synthesis Example 2]

In a reactor, 1.00 g (3.10×10⁻³ mol) of a compound having the formula (C): 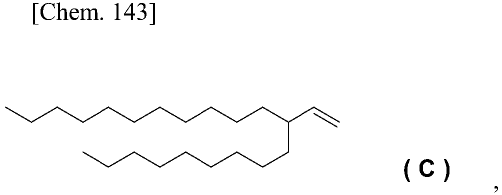 1.00 g of toluene, 1.14 g (9.31×10⁻³ mol) of trimethoxysilane, and 1.18×10⁻² g (containing 3.66×10⁻⁸ mol of Pt element) of a toluene solution of chloroplatinic acid/vinylsiloxane complex were mixed and aged at 80°C for 24 hours. Thereafter, the solvent and unreacted reactants were distilled off under reduced pressure, obtaining 1.25 g of a product.

Analysis by ¹H-NMR proved that the product had the structure represented by the following formula (D).

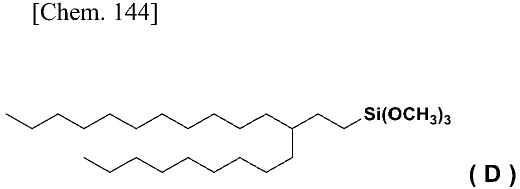

### [Synthesis Example 3]

In a reactor, 1.00 g (1.73×10⁻³ mol) of a compound having the formula (E): 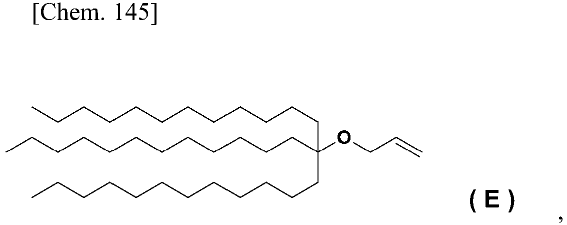 1.00 g of toluene, 0.636 g (5.20×10⁻³ mol) of trimethoxysilane, and 6.62×10⁻³ g (containing 2.05×10⁻⁸ mol of Pt element) of a toluene solution of chloroplatinic acid/vinylsiloxane complex were mixed and aged at 80°C for 24 hours. Thereafter, the solvent and unreacted reactants were distilled off under reduced pressure, obtaining 1.13 g of a product.

Analysis by ¹H-NMR proved that the product had the structure represented by the following formula (F).

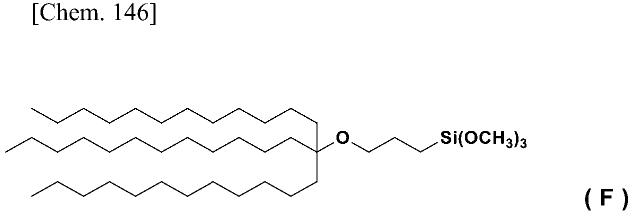

### [Synthesis Example 4]

In a reactor, 1.00 g (1.25×10⁻³ mol) of a compound having the formula (G): 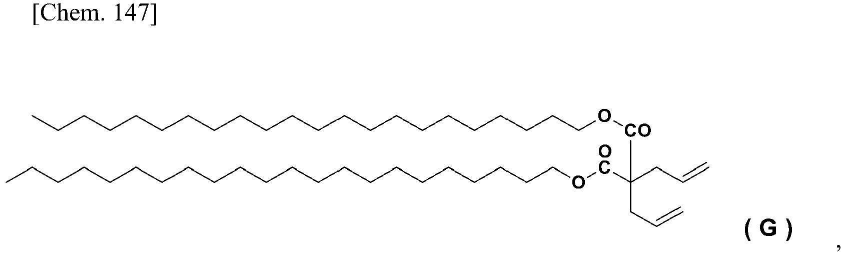 1.00 g of toluene, 0.458 g (3.75×10⁻³ mol) of trimethoxysilane, and 4.77×10⁻² g (containing 1.47×10⁻⁸ mol of Pt element) of a toluene solution of chloroplatinic acid/vinylsiloxane complex were mixed and aged at 80°C for 24 hours. Thereafter, the solvent and unreacted reactants were distilled off under reduced pressure, obtaining 1.10 g of a product.

Analysis by ¹H-NMR proved that the product had the structure represented by the following formula (H).

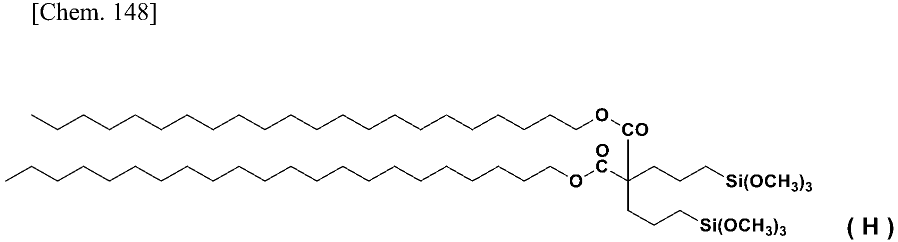

### [Synthesis Example 5]

In a reactor, 1.00 g (1.34×10⁻³ mol) of a compound having the formula (I): 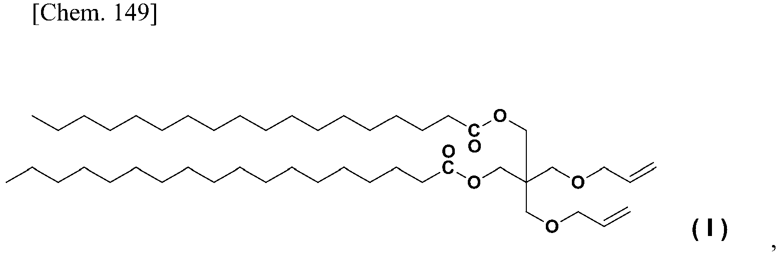 1.00 g of toluene, 0.490 g (4.01×10⁻³ mol) of trimethoxysilane, and 5.10×10⁻³ g (containing 1.58×10⁻⁸ mol of Pt element) of a toluene solution of chloroplatinic acid/vinylsiloxane complex were mixed and aged at 80°C for 24 hours. Thereafter, the solvent and unreacted reactants were distilled off under reduced pressure, obtaining 1.11 g of a product.

Analysis by ¹H-NMR proved that the product had the structure represented by the following formula (J).

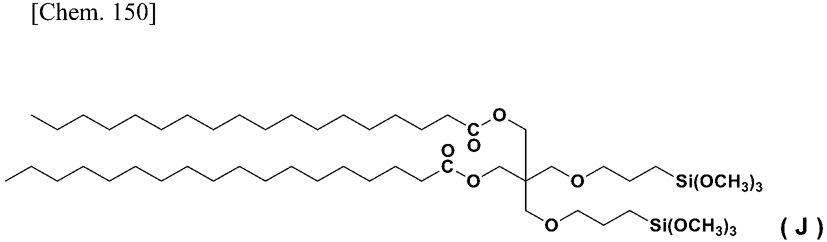

### [Synthesis Example 6]

In a reactor, 2.00 g (1.81×10⁻³ mol) of a compound having the formula (K): 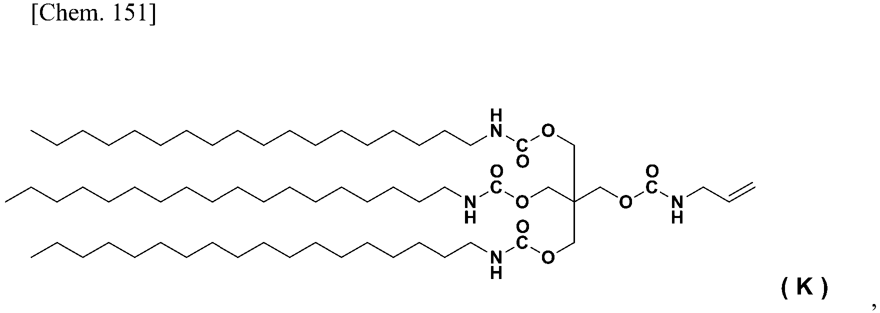 2.00 g of toluene, 0.891 g (5.43×10⁻³ mol) of triethoxysilane, and 6.91×10⁻³ g (containing 2.14×10⁻⁸ mol of Pt element) of a toluene solution of chloroplatinic acid/vinylsiloxane complex were mixed and aged at 80°C for 24 hours. Thereafter, the solvent and unreacted reactants were distilled off under reduced pressure, obtaining 2.15 g of a product.

Analysis by ¹H-NMR proved that the product had the structure represented by the following formula (L).

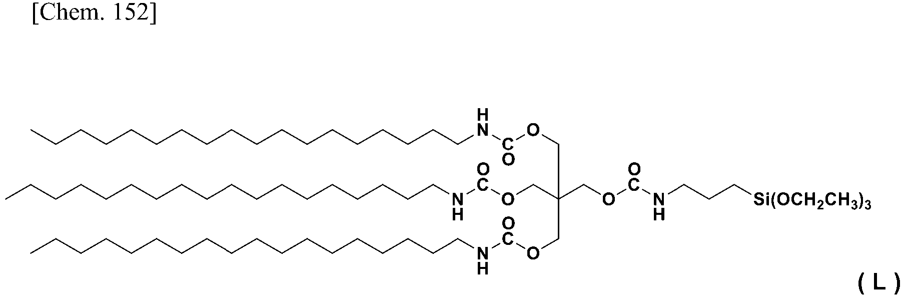

### [Synthesis Example 7]

In a reactor, 1.00 g (1.37×10⁻³ mol) of a compound having the formula (M): 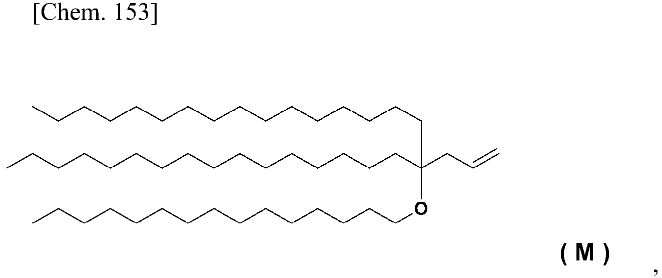 1.00 g of toluene, 0.502 g (4.11×10⁻³ mol) of trimethoxysilane, and 5.23×10⁻³ g (containing 1.62×10⁻⁸ mol of Pt element) of a toluene solution of chloroplatinic acid/vinylsiloxane complex were mixed and aged at 80°C for 24 hours. Thereafter, the solvent and unreacted reactants were distilled off under reduced pressure, obtaining 1.09 g of a product.

Analysis by ¹H-NMR proved that the product had the structure represented by the following formula (N).

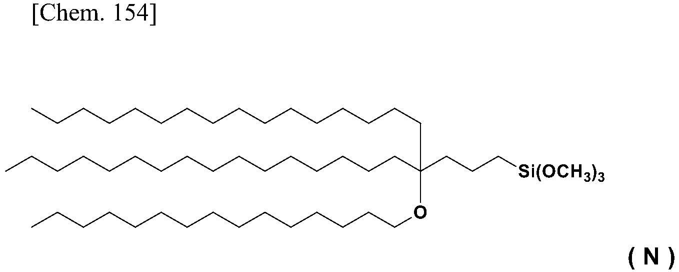

### [Synthesis Example 8]

In a reactor, 1.00 g (1.29×10⁻³ mol) of a compound having the formula (O): 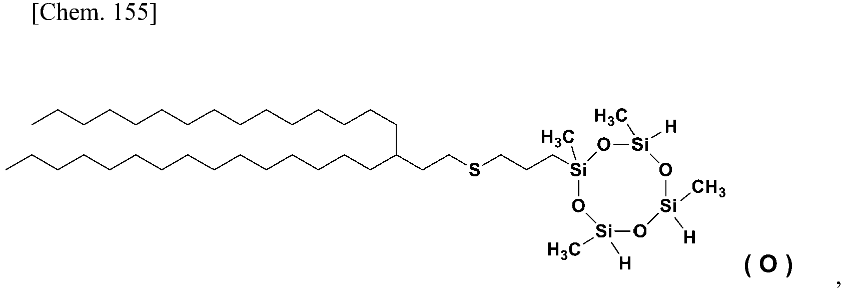 2.00 g of toluene, 1.25 g (7.73×10⁻³ mol) of allyltrimethoxysilane, and 4.92×10⁻³ g (containing 1.52×10⁻⁸ mol of Pt element) of a toluene solution of chloroplatinic acid/vinylsiloxane complex were mixed and aged at 80°C for 24 hours. Thereafter, the solvent and unreacted reactants were distilled off under reduced pressure, obtaining 1.58 g of a product.

Analysis by ¹H-NMR proved that the product had the structure represented by the following formula (P).

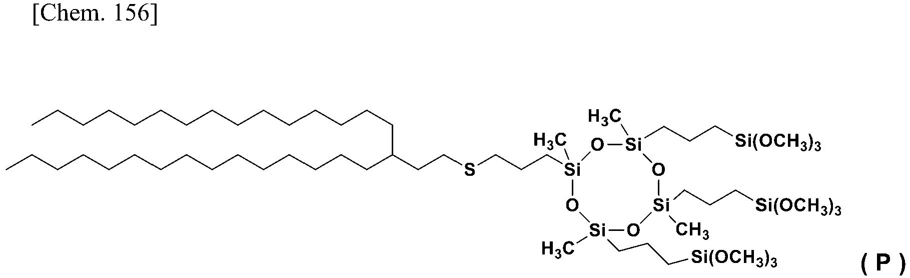

### [Synthesis Example 9]

In a reactor, 1.00 g (2.59×10⁻³ mol) of a compound having the formula (Q): 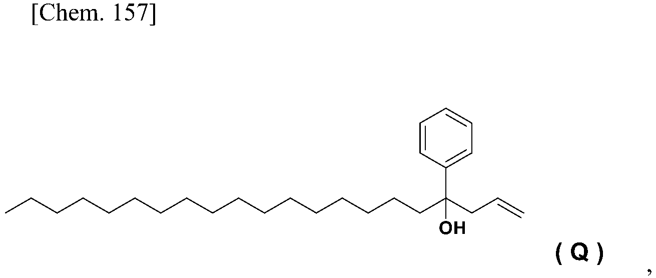 1.00 g of toluene, 0.949 g (7.77×10⁻³ mol) of trimethoxysilane, and 9.89×10⁻³ g (containing 3.06×10⁻⁸ mol of Pt element) of a toluene solution of chloroplatinic acid/vinylsiloxane complex were mixed and aged at 80°C for 24 hours. Thereafter, the solvent and unreacted reactants were distilled off under reduced pressure, obtaining 1.30 g of a product.

Analysis by ¹H-NMR proved that the product had the structure represented by the following formula (R).

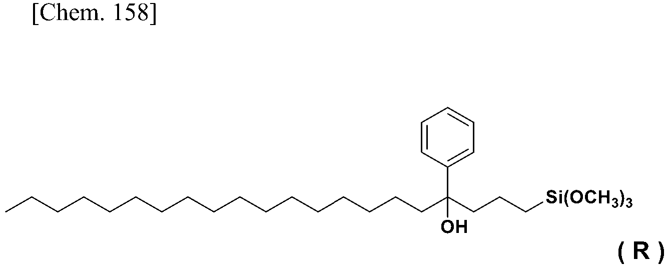

### [Synthesis Example 10]

In a reactor, 1.00 g (1.29×10⁻³ mol) of a compound having the formula (S): 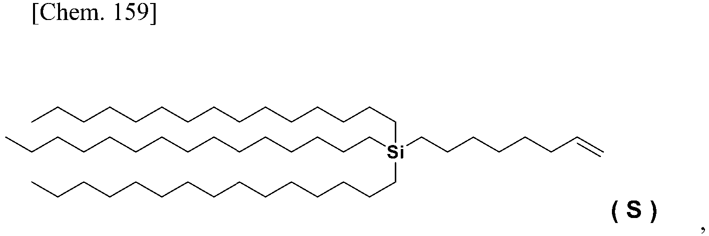 1.00 g of toluene, 0.474 g (3.88×10⁻³ mol) of trimethoxysilane, and 4.94×10⁻³ g (containing 1.53×10⁻⁸ mol of Pt element) of a toluene solution of chloroplatinic acid/vinylsiloxane complex were mixed and aged at 80°C for 24 hours. Thereafter, the solvent and unreacted reactants were distilled off under reduced pressure, obtaining 1.14 g of a product.

Analysis by ¹H-NMR proved that the product had the structure represented by the following formula (T).

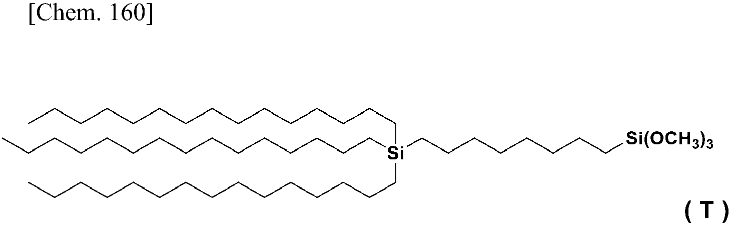

### [Synthesis Example 11]

In a reactor, 1.00 g (1.48×10⁻³ mol) of a compound having the formula (U): 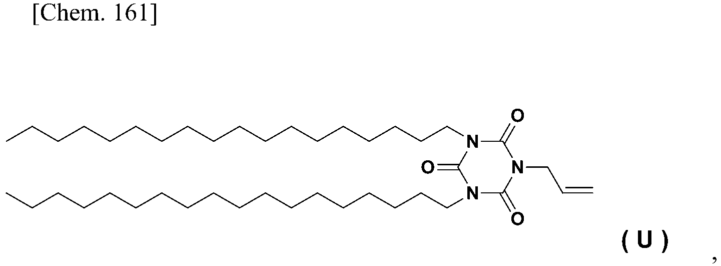 1.00 g of toluene, 0.544 g (4.45 ×10⁻³ mol) of trimethoxysilane, and 5.67×10⁻³ g (containing 1.75×10⁻⁸ mol of Pt element) of a toluene solution of chloroplatinic acid/vinylsiloxane complex were mixed and aged at 80°C for 24 hours. Thereafter, the solvent and unreacted reactants were distilled off under reduced pressure, obtaining 1.12 g of a product.

Analysis by ¹H-NMR proved that the product had the structure represented by the following formula (V).

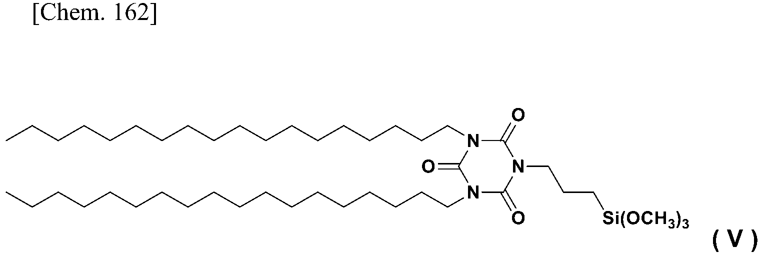

### [Synthesis Example 12]

In a reactor, 1.00 g (1.79×10⁻³ mol) of a compound having the formula (W): 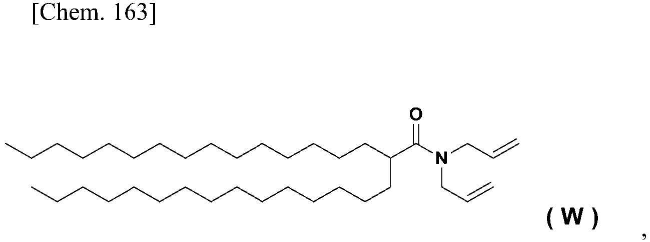 1.00 g of toluene, 1.31 g (1.07×10⁻² mol) of trimethoxysilane, and 6.83×10⁻³ g (containing 2.11×10⁻⁸ mol of Pt element) of a toluene solution of chloroplatinic acid/vinylsiloxane complex were mixed and aged at 80°C for 24 hours. Thereafter, the solvent and unreacted reactants were distilled off under reduced pressure, obtaining 1.36 g of a product.

Analysis by ¹H-NMR proved that the product had the structure represented by the following formula (X).

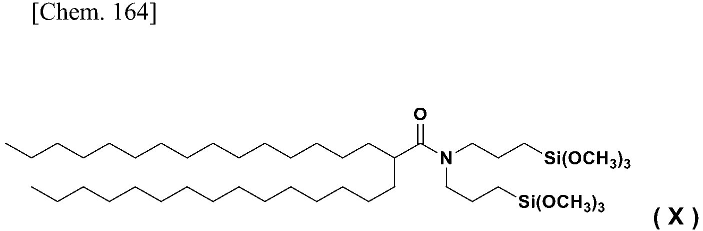

### [Synthesis Example 13]

In a reactor, 1.00 g (1.99×10⁻³ mol) of a compound having the formula (Y): 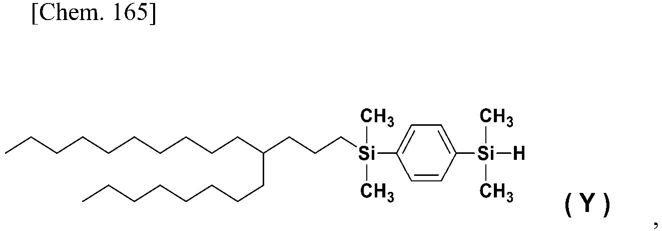 1.00 g of toluene, 0.884 g (5.97×10⁻³ mol) of vinyltrimethoxysilane, and 7.60×10⁻³ g (containing 2.35×10⁻⁸ mol of Pt element) of a toluene solution of chloroplatinic acid/vinylsiloxane complex were mixed and aged at 80°C for 24 hours. Thereafter, the solvent and unreacted reactants were distilled off under reduced pressure, obtaining 1.26 g of a product.

Analysis by ¹H-NMR proved that the product had the structure represented by the following formula (Z).

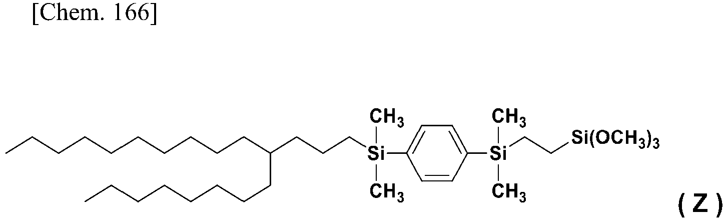

### [Synthesis Example 14]

In a reactor, 1.00 g (2.46×10⁻³ mol) of a compound having the formula (AA): 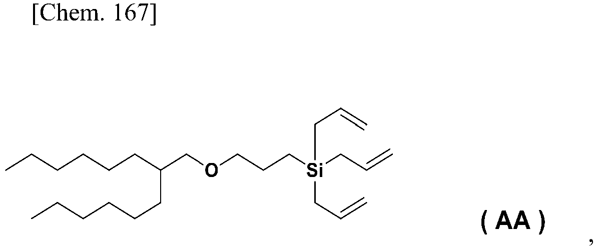 1.00 g of toluene, 2.71 g (2.21×10⁻² mol) of trimethoxysilane, and 9.40×10⁻³ g (containing 2.91×10⁻⁸ mol of Pt element) of a toluene solution of chloroplatinic acid/vinylsiloxane complex were mixed and aged at 80°C for 24 hours. Thereafter, the solvent and unreacted reactants were distilled off under reduced pressure, obtaining 1.75 g of a product.

Analysis by ¹H-NMR proved that the product had the structure represented by the following formula (AB).

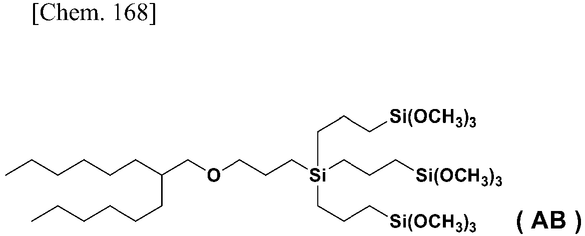

### [Synthesis Example 15]

In a reactor, 1.00 g (1.75×10⁻³ mol) of a compound having the formula (AC): 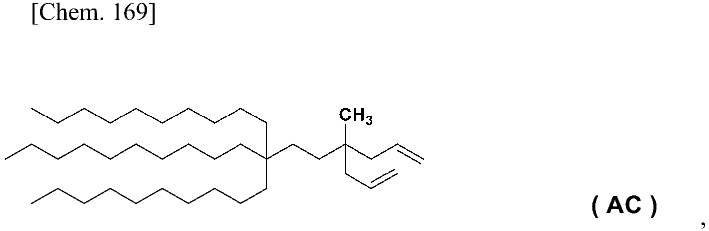 1.00 g of toluene, 1.28 g (1.05×10⁻² mol) of trimethoxysilane, and 6.67×10⁻³ g (containing 2.06×10⁻⁸ mol of Pt element) of a toluene solution of chloroplatinic acid/vinylsiloxane complex were mixed and aged at 80°C for 24 hours. Thereafter, the solvent and unreacted reactants were distilled off under reduced pressure, obtaining 1.40 g of a product.

Analysis by ¹H-NMR proved that the product had the structure represented by the following formula (AD).

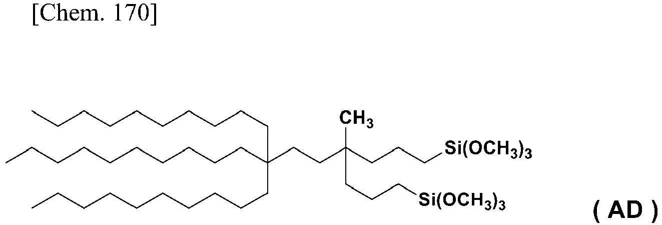

### [Synthesis Example 16]

In a reactor, 1.00 g (1.20×10⁻³ mol) of a compound having the formula (AE): 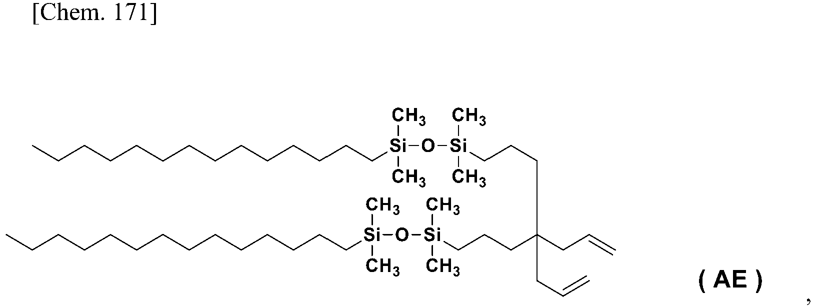 1.00 g of toluene, 1.48 g (7.17×10⁻³ mol) of triacetoxysilane, and 4.56×10⁻³ g (containing 1.41×10⁻⁸ mol of Pt element) of a toluene solution of chloroplatinic acid/vinylsiloxane complex were mixed and aged at 80°C for 24 hours. Thereafter, the solvent and unreacted reactants were distilled off under reduced pressure, obtaining 1.43 g of a product.

Analysis by ¹H-NMR proved that the product had the structure represented by the following formula (AF).

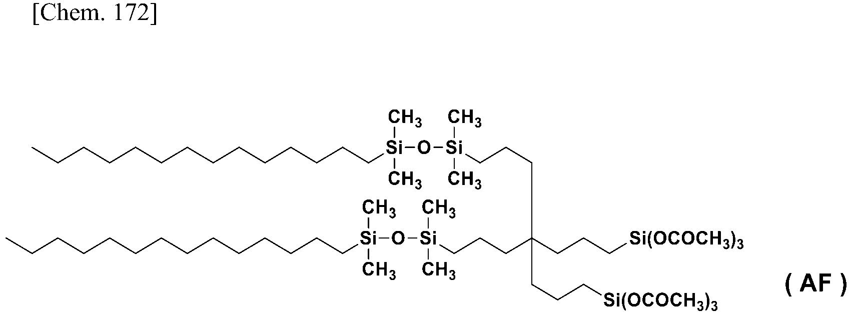

### [Synthesis Example 17]

In a reactor, 1.00 g (1.69×10⁻³ mol) of a compound having the formula (AG): 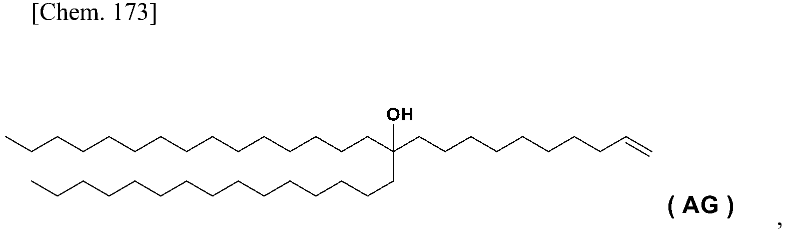 1.00 g of toluene, 0.621 g (5.08×10⁻³ mol) of trimethoxysilane, and 6.47×10⁻³ g (containing 2.00×10⁻⁸ mol of Pt element) of a toluene solution of chloroplatinic acid/vinylsiloxane complex were mixed and aged at 80°C for 24 hours. Thereafter, the solvent and unreacted reactants were distilled off under reduced pressure, obtaining 1.18 g of a product.

Analysis by ¹H-NMR proved that the product had the structure represented by the following formula (AH).

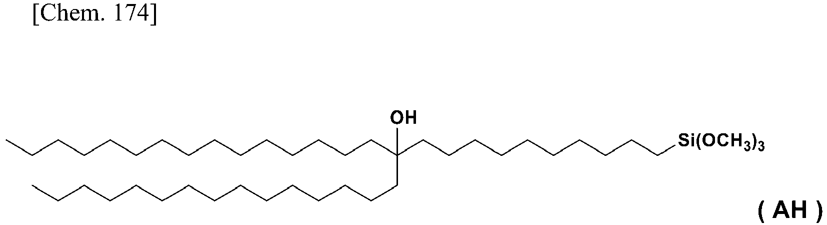

### [Synthesis Example 18]

In a reactor, 1.00 g (1.15×10⁻³ mol) of a compound having the formula (AI): 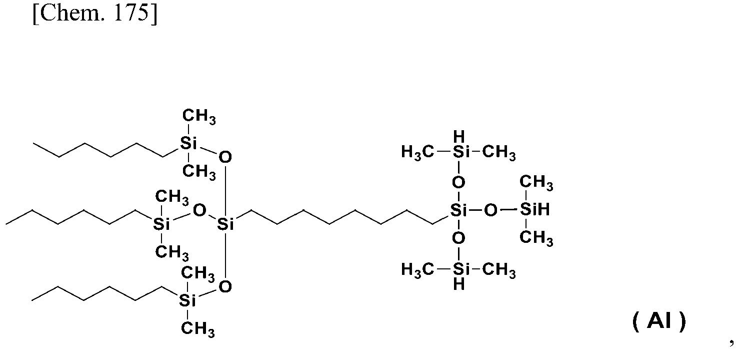 2.00 g of toluene, 2.40 g (1.03×10⁻² mol) of octenyltrimethoxysilane, and 4.39×10⁻³ g (containing 1.36×10⁻⁸ mol of Pt element) of a toluene solution of chloroplatinic acid/vinylsiloxane complex were mixed and aged at 80°C for 24 hours. Thereafter, the solvent and unreacted reactants were distilled off under reduced pressure, obtaining 1.18 g of a product.

Analysis by ¹H-NMR proved that the product had the structure represented by the following formula (AJ).

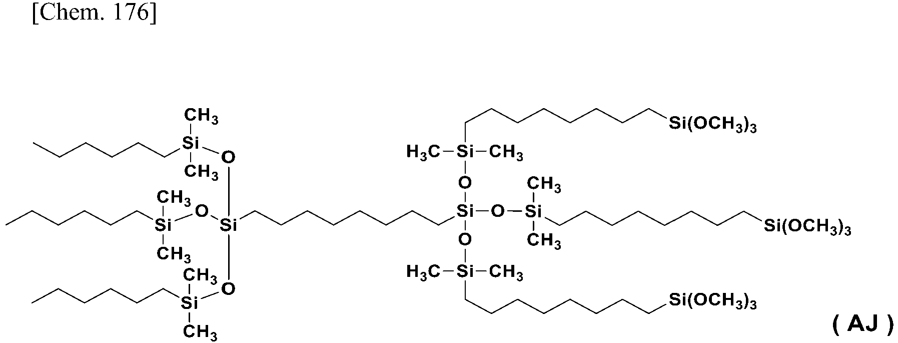

### [Synthesis Example 19]

In a reactor, 1.00 g (1.10×10⁻³ mol) of a compound having the formula (AK): 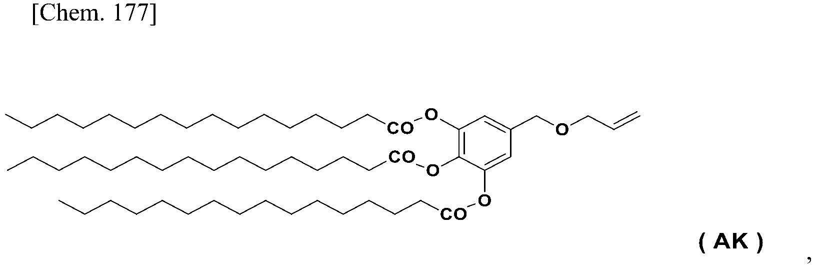 1.00 g of toluene, 0.403 g (3.29×10⁻³ mol) of trimethoxysilane, and 4.19×10⁻³ g (containing 1.30×10⁻⁸ mol of Pt element) of a toluene solution of chloroplatinic acid/vinylsiloxane complex were mixed and aged at 80°C for 24 hours. Thereafter, the solvent and unreacted reactants were distilled off under reduced pressure, obtaining 1.10 g of a product.

Analysis by ¹H-NMR proved that the product had the structure represented by the following formula (AL).

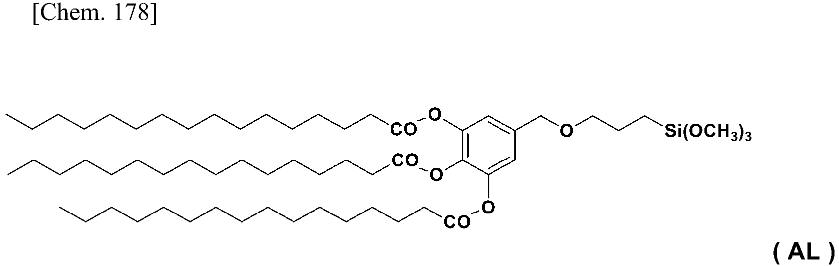

### [Synthesis Example 20]

In a reactor, 1.00 g (1.35×10⁻³ mol) of a compound having the formula (AM): 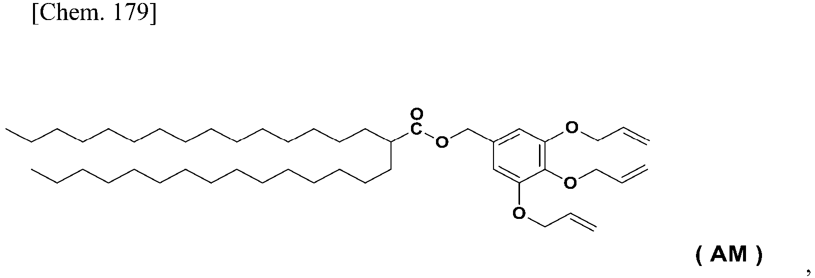 1.00 g of toluene, 1.49 g (1.22×10⁻² mol) of trimethoxysilane, and 5.17×10⁻³ g (containing 1.60×10⁻⁸ mol of Pt element) of a toluene solution of chloroplatinic acid/vinylsiloxane complex were mixed and aged at 80°C for 24 hours. Thereafter, the solvent and unreacted reactants were distilled off under reduced pressure, obtaining 1.47 g of a product.

Analysis by ¹H-NMR proved that the product had the structure represented by the following formula (AN).

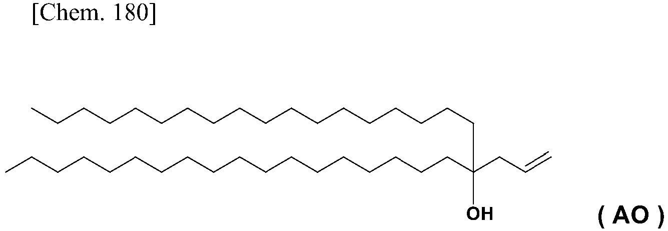

### [Synthesis Example 21]

In a reactor, 1.00 g (1.73×10⁻³ mol) of a compound having the formula (AO): 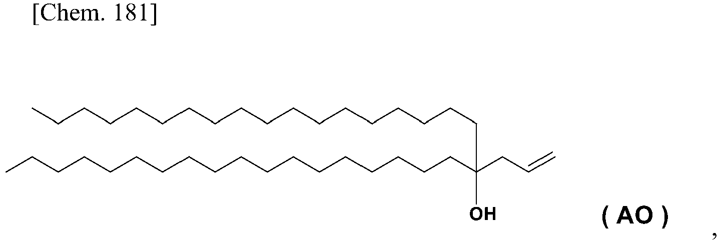 1.00 g of toluene, 0.636 g (5.20×10⁻³ mol) of trimethoxysilane, and 6.62×10⁻³ g (containing 2.05×10⁻⁸ mol of Pt element) of a toluene solution of chloroplatinic acid/vinylsiloxane complex were mixed and aged at 80°C for 24 hours. Thereafter, the solvent and unreacted reactants were distilled off under reduced pressure, obtaining 1.20 g of a product.

Analysis by ¹H-NMR proved that the product had the structure represented by the following formula (AP).

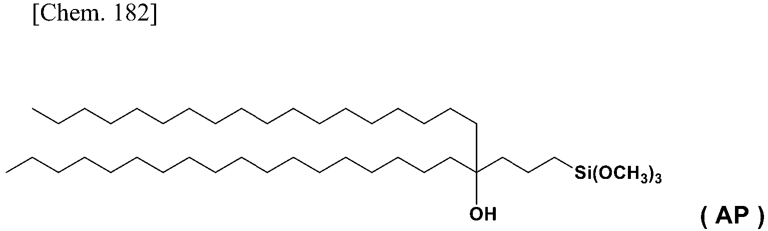

### [Synthesis Example 22]

In a reactor, 1.00 g (1.99×10⁻³ mol) of a compound having the formula (AQ): 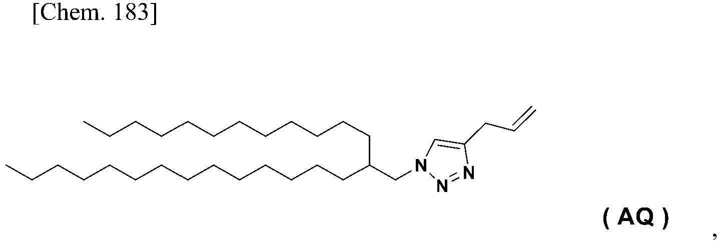 1.00 g of toluene, 0.810 g (5.98×10⁻³ mol) of trichlorosilane, and 7.62×10⁻³ g (containing 2.35×10⁻⁸ mol of Pt element) of a toluene solution of chloroplatinic acid/vinylsiloxane complex were mixed and aged at 60°C for 24 hours. Thereafter, the solvent and unreacted reactants were distilled off under reduced pressure, obtaining a product. The product was mixed with 1.00 g of toluene and 0.151 g (1.99×10⁻³ mol) of ethylene glycol monomethyl ether and aged at 50°C for 8 hours. The solution was further mixed with 1.27×10⁻² g (3.99×10⁻⁴ mol) of methanol and 0.633 g (5.97×10⁻³ mol) of trimethyl orthoformate and aged at room temperature for 24 hours. Thereafter, the solvent and unreacted reactants were distilled off under reduced pressure, obtaining 1.20 g of a product.

Analysis by ¹H-NMR proved that the product had the structure represented by the following formula (AR).

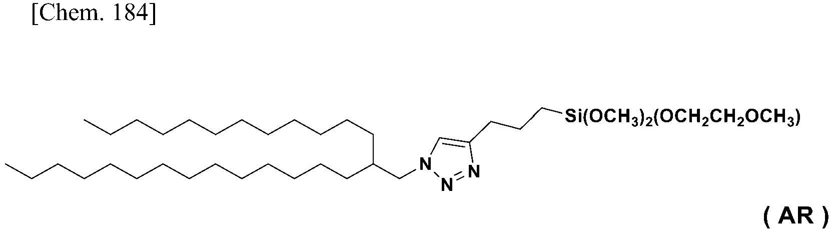

### [Synthesis Example 23]

In a reactor, 1.00 g (1.49×10⁻³ mol) of a compound having the formula (AS): 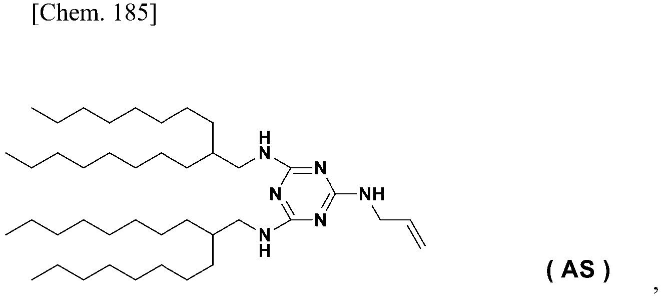 1.00 g of toluene, 0.547 g (4.47×10⁻³ mol) of trimethoxysilane, and 5.69×10⁻³ g (containing 1.76×10⁻⁸ mol of Pt element) of a toluene solution of chloroplatinic acid/vinylsiloxane complex were mixed and aged at 80°C for 24 hours. Thereafter, the solvent and unreacted reactants were distilled off under reduced pressure, obtaining 1.11 g of a product.

Analysis by ¹H-NMR proved that the product had the structure represented by the following formula (AT).

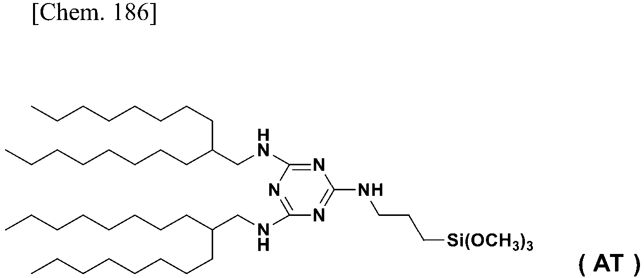

### [Synthesis Example 24]

In a reactor, 1.00 g (1.83×10⁻³ mol) of a compound having the formula (AU): 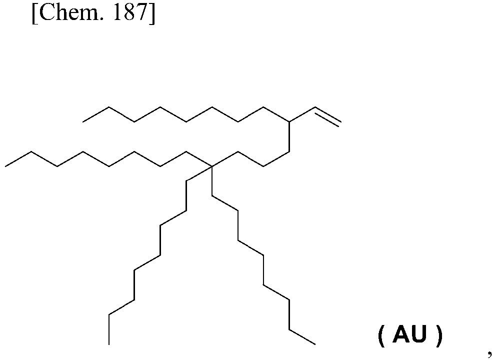 1.00 g of toluene, 0.671 g (5.49×10⁻³ mol) of trimethoxysilane, and 6.99×10⁻³ g (containing 2.16×10⁻⁸ mol of Pt element) of a toluene solution of chloroplatinic acid/vinylsiloxane complex were mixed and aged at 80°C for 24 hours. Thereafter, the solvent and unreacted reactants were distilled off under reduced pressure, obtaining 1.17 g of a product.

Analysis by ¹H-NMR proved that the product had the structure represented by the following formula (AV).

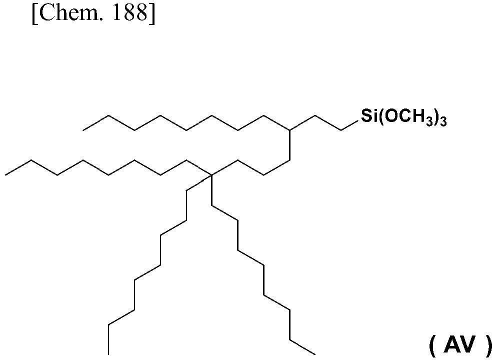

### [Synthesis Example 25]

In a reactor, 1.00 g (1.98×10⁻³ mol) of a compound having the formula (AW): 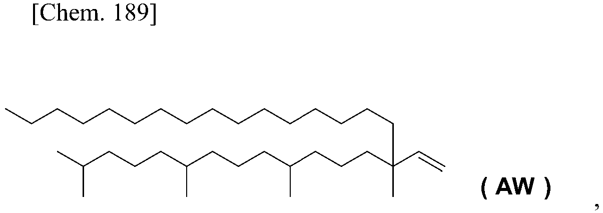 1.00 g of toluene, 0.727 g (5.95×10⁻³ mol) of trimethoxysilane, and 5.57×10⁻³ g (containing 2.34×10⁻⁸ mol of Pt element) of a toluene solution of chloroplatinic acid/vinylsiloxane complex were mixed and aged at 80°C for 24 hours. Thereafter, the solvent and unreacted reactants were distilled off under reduced pressure, obtaining 1.22 g of a product.

Analysis by ¹H-NMR proved that the product had the structure represented by the following formula (AX).

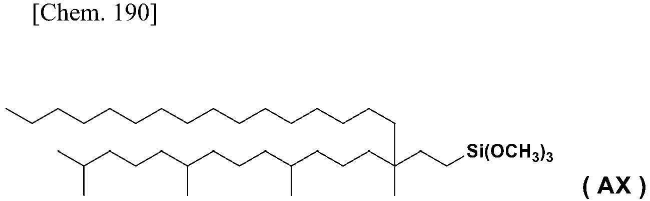

### [Synthesis Example 26]

In a reactor, 1.00 g (1.52×10⁻³ mol) of a compound having the formula (AY): 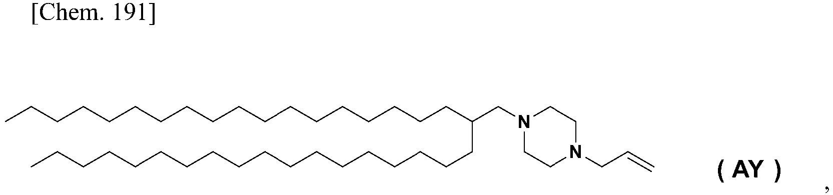 1.00 g of toluene, 0.557 g (4.55×10⁻³ mol) of trimethoxysilane, and 5.80×10⁻³ g (containing 1.79×10⁻⁸ mol of Pt element) of a toluene solution of chloroplatinic acid/vinylsiloxane complex were mixed and aged at 80°C for 24 hours. Thereafter, the solvent and unreacted reactants were distilled off under reduced pressure, obtaining 1.13 g of a product.

Analysis by ¹H-NMR proved that the product had the structure represented by the following formula (AZ).

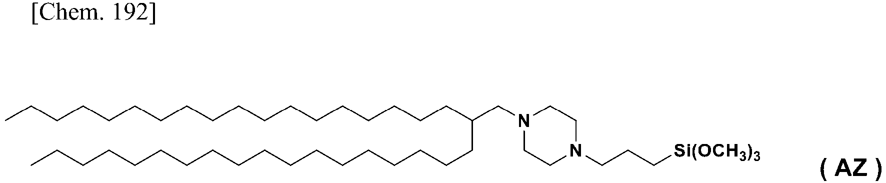

### [Synthesis Example 27]

In a reactor, 1.00 g (1.83×10⁻³ mol) of a compound having the formula (BA): 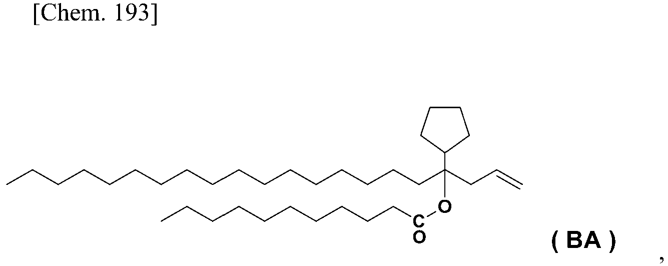 1.00 g of toluene, 0.671 g (5.49×10⁻³ mol) of trimethoxysilane, and 6.99×10⁻³ g (containing 2.16×10⁻⁸ mol of Pt element) of a toluene solution of chloroplatinic acid/vinylsiloxane complex were mixed and aged at 80°C for 24 hours. Thereafter, the solvent and unreacted reactants were distilled off under reduced pressure, obtaining 1.21 g of a product.

Analysis by ¹H-NMR proved that the product had the structure represented by the following formula (BB).

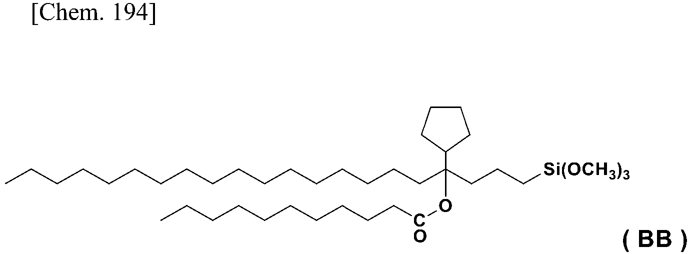

### [Synthesis Example 28]

In a reactor, 1.00 g (1.97×10⁻³ mol) of a compound having the formula (BC): 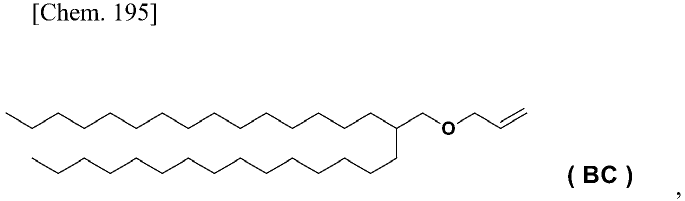 1.00 g of toluene, 0.802 g (5.92×10⁻³ mol) of trichlorosilane, and 7.54×10⁻³ g (containing 2.33×10⁻⁸ mol of Pt element) of a toluene solution of chloroplatinic acid/vinylsiloxane complex were mixed and aged at 60°C for 24 hours. Thereafter, the solvent and unreacted reactants were distilled off under reduced pressure, obtaining a product. The product was mixed with 3.00 g of toluene and aged at room temperature for 6 hours while bubbling ammonia gas at a feed rate of 40 cc/min. After the mixture was filtered, the solvent and unreacted reactants were distilled off under reduced pressure, obtaining 1.01 g of a product.

Analysis by ¹H-NMR proved that the product had the structure represented by the following formula (BD).

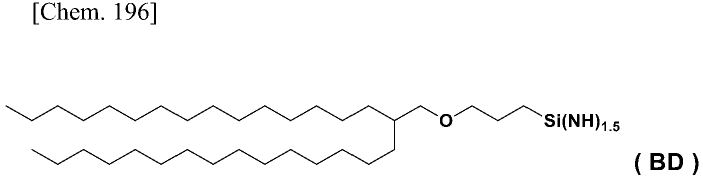

### [Synthesis Example 29]

In a reactor, 1.00 g (1.50×10⁻³ mol) of a compound having the formula (BE): 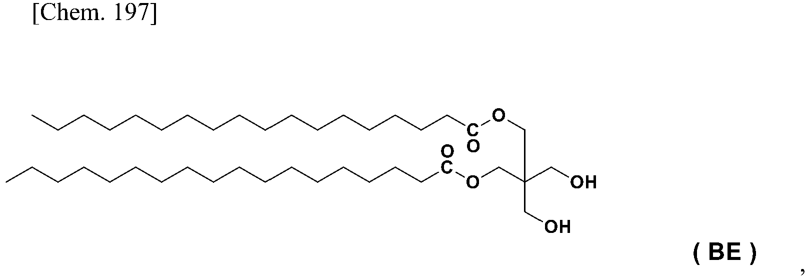 1.00 g of toluene, 0.777 g (3.14×10⁻³ mol) of (3-isocyanatopropyl)triethoxysilane, and 4.22×10⁻² g (7.48×10⁻⁵ mol) of titanium tetra-2-ethylhexoxide were mixed and aged at 50°C for 24 hours. Thereafter, the solvent and unreacted reactants were distilled off under reduced pressure, obtaining 1.70 g of a product.

Analysis by ¹H-NMR proved that the product had the structure represented by the following formula (BF).

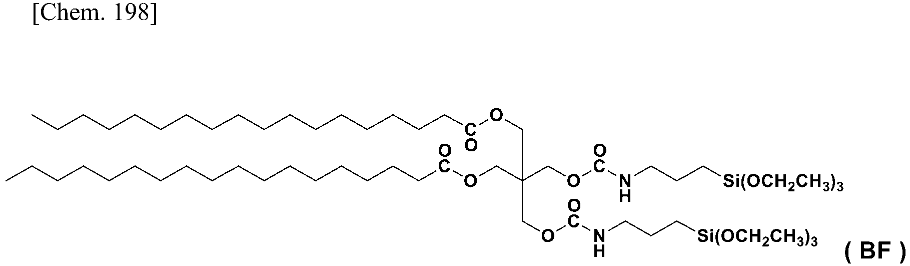

### [Synthesis Example 30]

In a reactor, 1.00 g (2.44×10⁻³ mol) of a compound having the formula (BG): 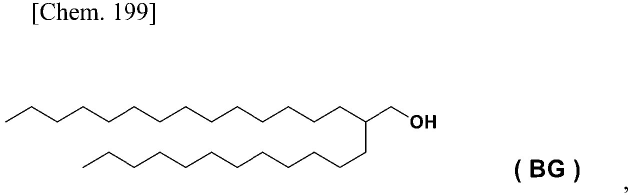 1.00 g of toluene, 0.550 g (2.68×10⁻³ mol) of (3-isocyanatopropyl)trimethoxysilane, and 6.88×10⁻² g (1.22×10⁻⁴ mol) of titanium tetra-2-ethylhexoxide were mixed and aged at 50°C for 24 hours. Thereafter, the solvent and unreacted reactants were distilled off under reduced pressure, obtaining 1.46 g of a product.

Analysis by ¹H-NMR proved that the product had the structure represented by the following formula (BH).

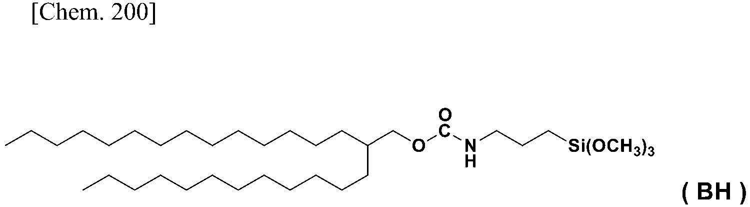

### [Synthesis Example 31]

In a reactor, 1.00 g (1.92×10⁻³ mol) of a compound having the formula (BI): 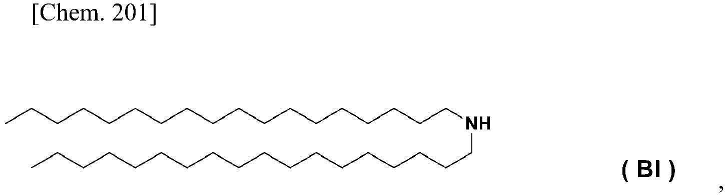 1.00 g of toluene, and 0.521 g (2.11×10⁻³ mol) of (3-isocyanatopropyl)triethoxysilane were mixed and aged at 50°C for 24 hours. Thereafter, the solvent and unreacted reactants were distilled off under reduced pressure, obtaining 1.45 g of a product.

Analysis by ¹H-NMR proved that the product had the structure represented by the following formula (BJ).

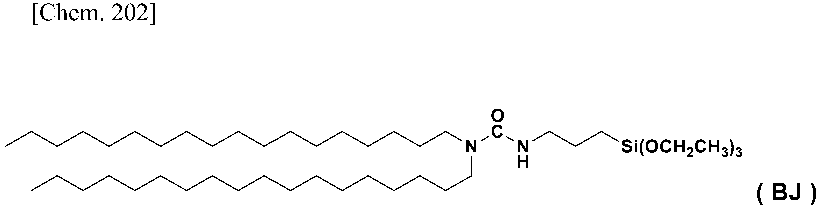

### [Synthesis Example 32]

In a reactor, 1.00 g (3.33×10⁻³ mol) of a compound having the formula (BK): 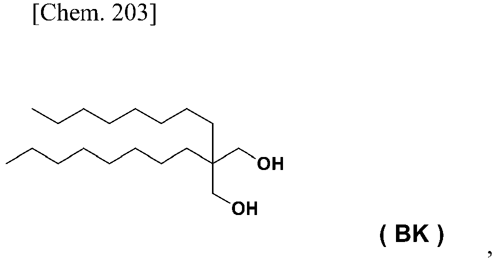 1.00 g of toluene, 1.43 g (6.99×10⁻³ mol) of (3-isocyanatopropyl)trimethoxysilane, and 9.40×10⁻² g (1.67×10⁻⁴ mol) of titanium tetra-2-ethylhexoxide were mixed and aged at 50°C for 24 hours. Thereafter, the solvent and unreacted reactants were distilled off under reduced pressure, obtaining 2.34 g of a product.

Analysis by ¹H-NMR proved that the product had the structure represented by the following formula (BL).

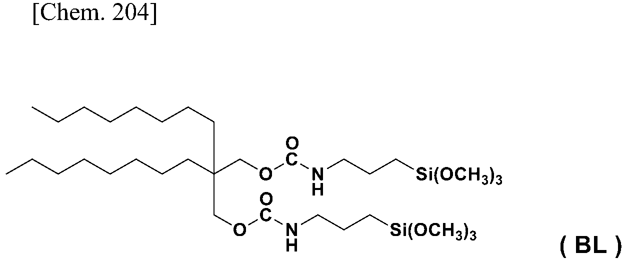

### [Synthesis Example 33]

In a reactor, 1.00 g (2.61×10⁻³ mol) of a compound having the formula (BM): 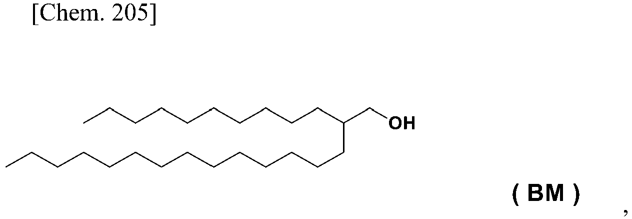 1.00 g of toluene, and 0.441 g (2.88×10⁻³ mol) of phosphorus oxychloride were mixed and aged at room temperature for 8 hours. To the solution, 4.00 g of water was added, followed by aging at room temperature for 1 hour. After the water layer was removed by separatory operation, the solvent and unreacted reactants were distilled off under reduced pressure, obtaining 1.31 g of a product.

Analysis by ¹H-NMR proved that the product had the structure represented by the following formula (BN).

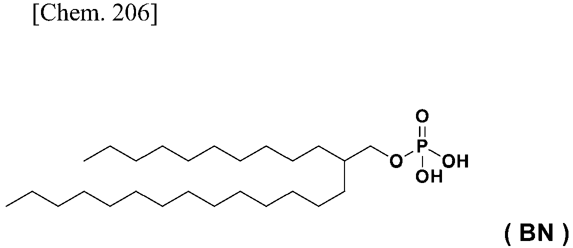

### [Synthesis Example 34]

In a reactor, 1.00 g (2.37×10⁻³ mol) of a compound having the formula (BO): 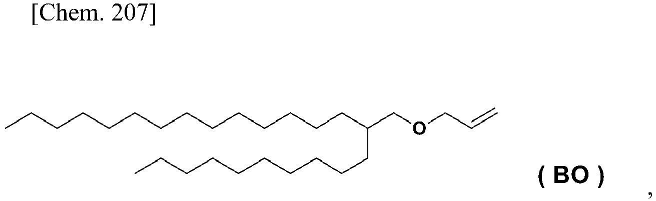 1.00 g of toluene, 0.360 g (4.73×10⁻³ mol) of thioacetic acid, and 0.389 g (2.37×10⁻⁵ mol) of AIBN (2,2'-azobisisobutyronitrile) were mixed and aged at 70°C for 24 hours. To the solution, 2.00 g of sodium hydrogencarbonate was added, followed by separatory operation. The solvent and unreacted reactants were distilled off under reduced pressure. The compound thus obtained was mixed with 1.00 g of toluene, 0.826 g (7.10×10⁻³ mol) of triethylsilane, and 1.26×10⁻² g (containing 1.18×10⁻⁴ mol of Pd element) of Pd/C and aged at 80°C for 24 hours. To the solution, 2.00 g of 2M hydrochloric acid was added, followed by aging at 50°C for 6 hours. After the water layer was removed by separatory operation, the solvent and unreacted reactants were distilled off under reduced pressure, obtaining 0.976 g of a product.

Analysis by ¹H-NMR proved that the product had the structure represented by the following formula (BP).

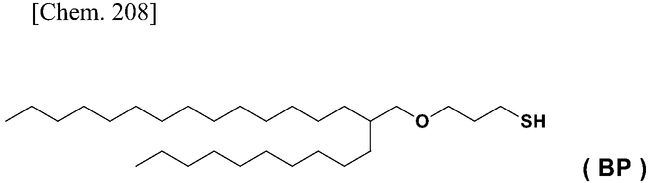

### [Synthesis Example 35]

In a reactor, 1.00 g (3.01×10⁻³ mol) of a compound having the formula (BQ): 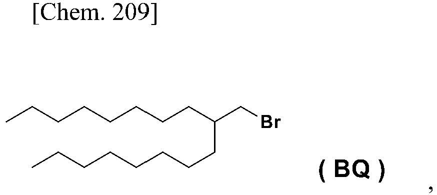 2.00 g of N,N-dimethylformamide (DMF), and 0.836 g (4.52×10⁻³ mol) of potassium phthalimide were mixed and aged at 110°C for 24 hours. To the solution, 2.00 g of water was added, followed by separatory operation. The solvent and unreacted reactants were distilled off under reduced pressure. The compound thus obtained was mixed with 2.00 g of ethanol and 0.301 g (6.02×10⁻³ mol) of hydrazine monohydrate and aged at 65°C for 24 hours. To the solution, 4.00 g of water was added, followed by aging at room temperature for 1 hour. After the water layer was removed by separatory operation, the solvent and unreacted reactants were distilled off under reduced pressure, obtaining 0.936 g of a product.

Analysis by ¹H-NMR proved that the product had the structure represented by the following formula (BR).

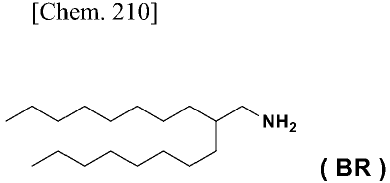

### [Synthesis Example 36]

In a reactor, 1.00 g (1.31×10⁻³ mol) of a compound having the formula (BS): 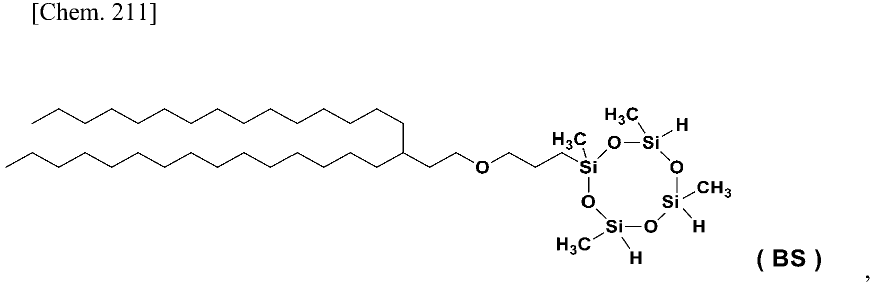 2.00 g of toluene, 0.750 g (6.57×10⁻³ mol) of allyl glycidyl ether, and 5.02×10⁻³ g (containing 1.55×10⁻⁸ mol of Pt element) of a toluene solution of chloroplatinic acid/vinylsiloxane complex were mixed and aged at 80°C for 24 hours. Thereafter, the solvent and unreacted reactants were distilled off under reduced pressure, obtaining 1.38 g of a product.

Analysis by ¹H-NMR proved that the product had the structure represented by the following formula (BT).

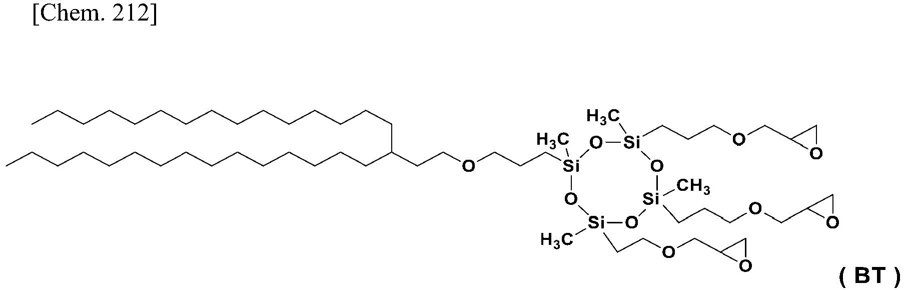

### [Synthesis Example 37]

In a reactor, 1.00 g (1.12×10⁻³ mol) of a compound having the formula (BU): 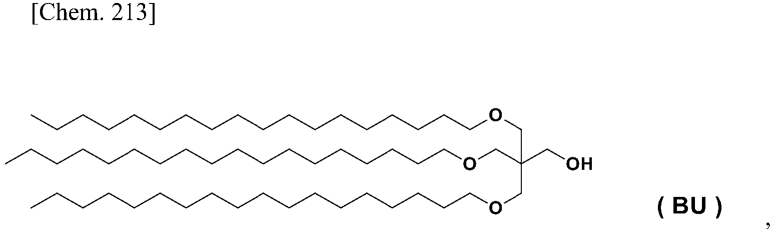 1.00 g of toluene, 0.281 g (1.18×10⁻³ mol) of Karenz BEI (1,1-(bisacryloyloxymethyl)ethyl isocyanate), and 3.16×10⁻² g (5.60×10⁻⁵ mol) of titanium tetra-2-ethylhexoxide were mixed and aged at 50°C for 24 hours. Thereafter, the solvent and unreacted reactants were distilled off under reduced pressure, obtaining 1.25 g of a product.

Analysis by ¹H-NMR proved that the product had the structure represented by the following formula (BV).

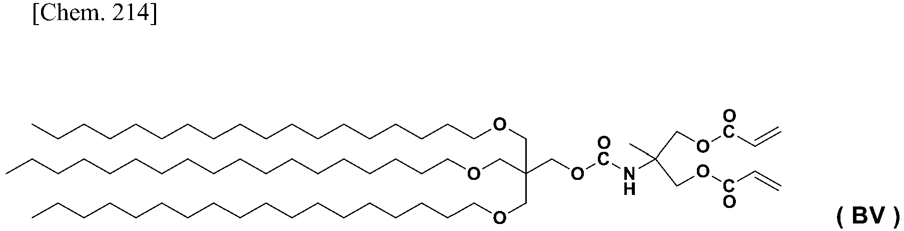

### [Synthesis Example 38]

In a reactor, 1.00 g (2.81×10⁻³ mol) of a compound having the formula (BW): 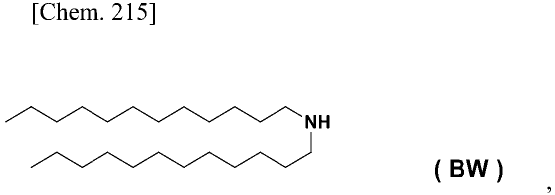 1.00 g of toluene, and 0.461 g (2.97×10⁻³ mol) of Karenz MOI (2-isocyanatoethyl methacrylate) were mixed and aged at 50°C for 24 hours. Thereafter, the solvent and unreacted reactants were distilled off under reduced pressure, obtaining 1.43 g of a product.

Analysis by ¹H-NMR proved that the product had the structure represented by the following formula (BX).

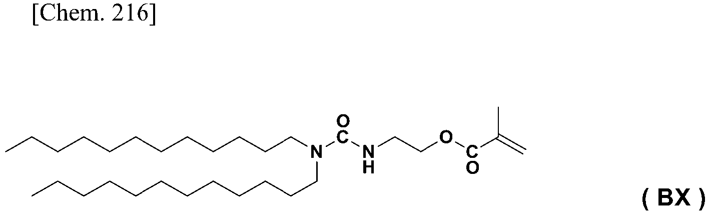

### [Synthesis Example 39]

In a reactor, 1.00 g (1.82×10⁻³ mol) of a compound having the formula (BY): 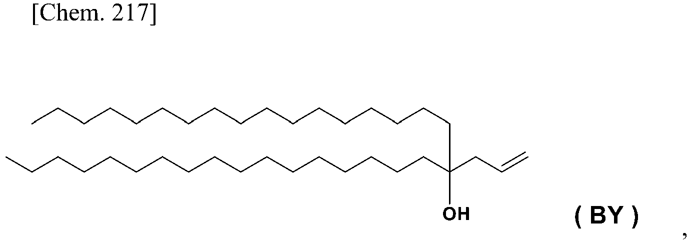 1.00 g of toluene, 0.668 g (5.47×10⁻³ mol) of trimethoxysilane, and 6.96×10⁻³ g (containing 2.15×10⁻⁸ mol of Pt element) of a toluene solution of chloroplatinic acid/vinylsiloxane complex were mixed and aged at 80°C for 24 hours. Thereafter, the solvent and unreacted reactants were distilled off under reduced pressure, obtaining 1.21 g of a product.

Analysis by ¹H-NMR proved that the product had the structure represented by the following formula (BZ).

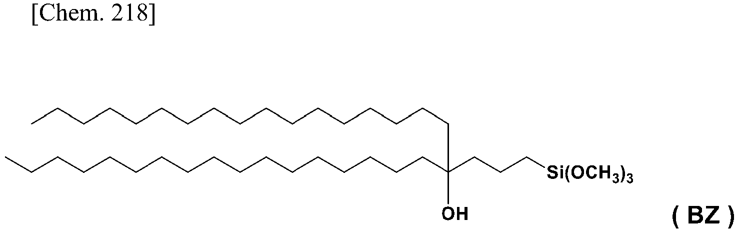

### [Synthesis Example 40]

In a reactor, 1.00 g (1.59×10⁻³ mol) of a compound having the formula (CA): 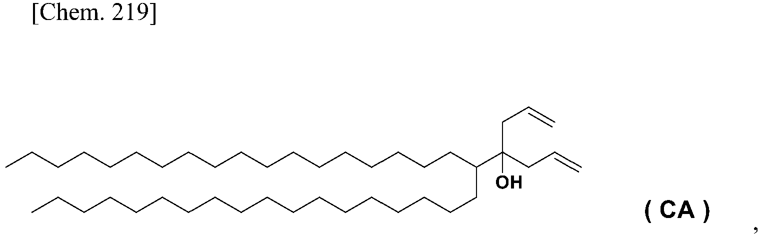 1.00 g of toluene, 1.16 g (9.51×10⁻³ mol) of trimethoxysilane, and 6.06×10⁻³ g (containing 1.87×10⁻⁸ mol of Pt element) of a toluene solution of chloroplatinic acid/vinylsiloxane complex were mixed and aged at 80°C for 24 hours. Thereafter, the solvent and unreacted reactants were distilled off under reduced pressure, obtaining 1.35 g of a product.

Analysis by ¹H-NMR proved that the product had the structure represented by the following formula (CB).

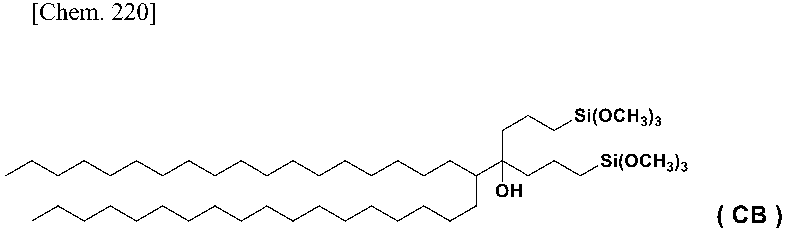

### [Synthesis Example 41]

In a reactor, 1.00 g (1.51×10⁻³ mol) of a compound having the formula (CC): 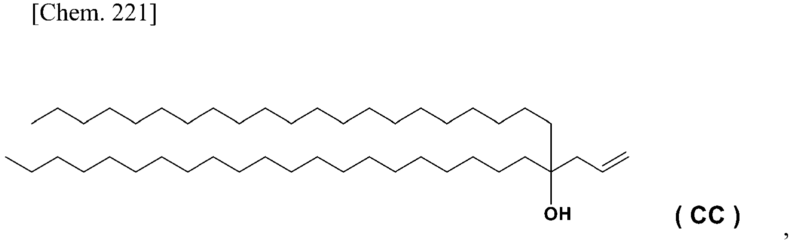 1.00 g of toluene, 0.555 g (4.54×10⁻³ mol) of trimethoxysilane, and 5.78×10⁻³ g (containing 1.79×10⁻⁸ mol of Pt element) of a toluene solution of chloroplatinic acid/vinylsiloxane complex were mixed and aged at 80°C for 24 hours. Thereafter, the solvent and unreacted reactants were distilled off under reduced pressure, obtaining 1.13 g of a product.

Analysis by ¹H-NMR proved that the product had the structure represented by the following formula (CD).

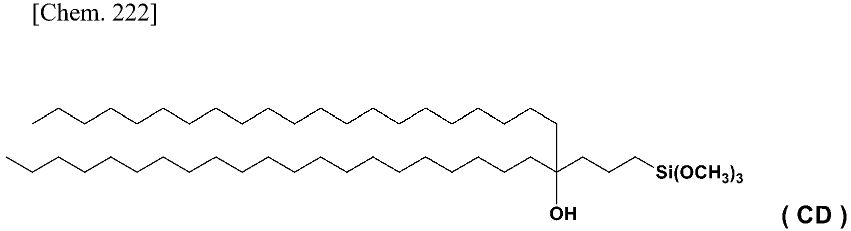

### [Synthesis Example 42]

In a reactor, 1.00 g (2.06×10⁻³ mol) of a compound having the formula (CE): 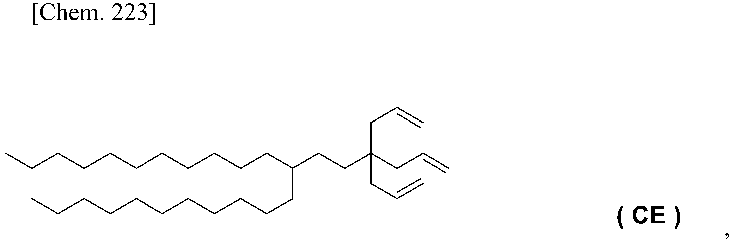 1.00 g of toluene, 2.26 g (1.84×10⁻² mol) of trimethoxysilane, and 7.85×10⁻³ g (containing 2.43×10⁻⁸ mol of Pt element) of a toluene solution of chloroplatinic acid/vinylsiloxane complex were mixed and aged at 80°C for 24 hours. Thereafter, the solvent and unreacted reactants were distilled off under reduced pressure, obtaining 1.42 g of a product.

Analysis by ¹H-NMR proved that the product had the structure represented by the following formula (CF).

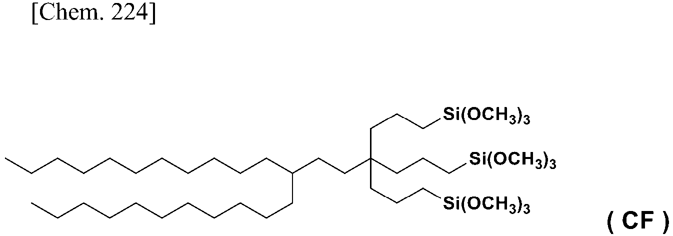

### [Synthesis Example 43]

In a reactor, 1.00 g (2.44×10⁻³ mol) of a compound having the formula (CG): 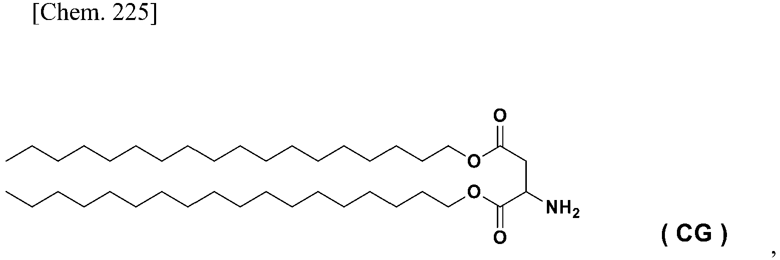 1.00 g of toluene, and 0.354 g (1.73×10⁻³ mol) of (3-isocyanatopropyl)trimethoxysilane were mixed and aged at 60°C for 24 hours. Thereafter, the solvent and unreacted reactants were distilled off under reduced pressure, obtaining 1.60 g of a product.

Analysis by ¹H-NMR proved that the product had the structure represented by the following formula (CH).

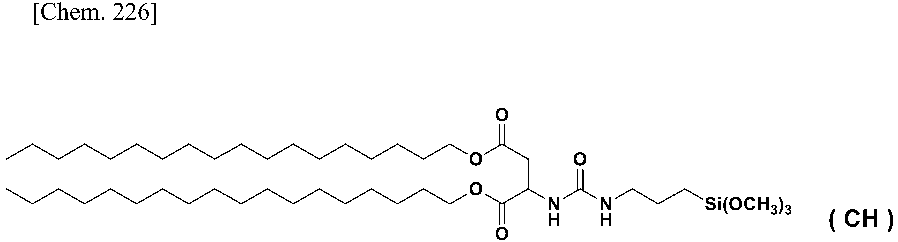

### [Example 1]

A surface treating agent was prepared by dissolving the compound obtained in Synthesis Example 1 in toluene in a concentration of 20% by weight.

### [Example 2]

A surface treating agent was prepared by dissolving the compound obtained in Synthesis Example 2 in isooctane in a concentration of 20% by weight.

### [Example 3]

A surface treating agent was prepared by dissolving the compound obtained in Synthesis Example 3 in dibutyl ether in a concentration of 20% by weight.

### [Example 4]

A surface treating agent was prepared by dissolving the compound obtained in Synthesis Example 4 in dibutyl ether in a concentration of 20% by weight.

### [Example 5]

A surface treating agent was prepared by dissolving the compound obtained in Synthesis Example 5 in toluene in a concentration of 10% by weight.

### [Example 6]

A surface treating agent was prepared by dissolving the compound obtained in Synthesis Example 7 in isononane in a concentration of 20% by weight.

### [Example 7]

A surface treating agent was prepared by dissolving the compound obtained in Synthesis Example 8 in dibutyl ether in a concentration of 5% by weight.

### [Example 8]

A surface treating agent was prepared by dissolving the compound obtained in Synthesis Example 9 in propylene glycol monomethyl ether acetate in a concentration of 30% by weight.

### [Example 9]

A surface treating agent was prepared by dissolving the compound obtained in Synthesis Example 10 in toluene in a concentration of 80% by weight.

### [Example 10]

A surface treating agent was prepared by dissolving the compound obtained in Synthesis Example 11 in toluene in a concentration of 90% by weight.

### [Example 11]

A surface treating agent was prepared by dissolving the compound obtained in Synthesis Example 12 in isooctane in a concentration of 10% by weight.

### [Example 12]

A surface treating agent was prepared by dissolving the compound obtained in Synthesis Example 15 in dibutyl ether in a concentration of 50% by weight.

### [Example 13]

A surface treating agent was prepared by dissolving the compound obtained in Synthesis Example 17 in 50/50 hexane/isooctane mixture in a concentration of 20% by weight.

### [Example 14]

A surface treating agent was prepared by dissolving the compound obtained in Synthesis Example 18 in toluene in a concentration of 20% by weight.

### [Example 15]

A surface treating agent was prepared by dissolving the compound obtained in Synthesis Example 20 in toluene in a concentration of 20% by weight.

### [Example 16]

A surface treating agent was prepared by dissolving the compound obtained in Synthesis Example 21 in dibutyl ether in a concentration of 20% by weight.

### [Example 17]

A surface treating agent was prepared by dissolving the compound obtained in Synthesis Example 24 in isooctane in a concentration of 20% by weight.

### [Example 18]

A surface treating agent was prepared by dissolving the compound obtained in Synthesis Example 26 in isooctane in a concentration of 20% by weight.

### [Example 19]

A surface treating agent was prepared by dissolving the compound obtained in Synthesis Example 28 in isooctane in a concentration of 20% by weight.

### [Example 20]

A surface treating agent was prepared by dissolving the compound obtained in Synthesis Example 31 in isooctane in a concentration of 20% by weight.

### [Example 21]

A surface treating agent was prepared by dissolving the compound obtained in Synthesis Example 32 in propylene glycol monomethyl ether acetate in a concentration of 20% by weight.

### [Example 22]

The compound obtained in Synthesis Example 14 was used as a surface treating agent without dilution.

### [Example 23]

The compound obtained in Synthesis Example 30 was used as a surface treating agent without dilution.

### [Comparative Example 1]

A surface treating agent was prepared by dissolving the compound having the formula (A'): 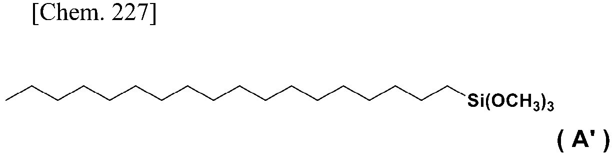 in toluene in a concentration of 20% by weight.

### [Comparative Example 2]

A surface treating agent was prepared by dissolving the compound having the formula (B'): 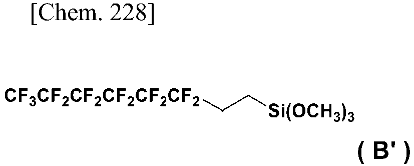 in toluene in a concentration of 20% by weight.

### [Comparative Example 3]

No surface treating agent.

### Preparation of surface treating agent and formation of cured film

Surface treating agents were prepared as in the foregoing Examples and Comparative Examples. On glass (Gorilla Glass by Corning, tradename Gorilla III, size 100 mm × 50 mm × 0.7 mm) which had been coated on its outermost surface with SiO₂ of 10 nm thick under the conditions shown below, each surface treating agent was deposited by a vacuum evaporation system (model VTR-350M by Ulvac Inc.) under conditions: pressure 2.0×10⁻² Pa and heating temperature 700°C. The coating was cured in an atmosphere at 80°C and relative humidity 80% for 1 hour and further in an atmosphere at 25°C and relative humidity 50% for 12 hours, forming a cured film having a thickness of 3 to 5 nm.

### [SiO₂ layer deposition conditions]

| | |
|---|---|
| Deposition system: | OTFC-1300 by Optorun Co., Ltd. |
| Depositing material: | SiO₂ |
| Depositing chamber pressure: | 0.015 Pa |
| Depositing rate: | 0.8 nm/s |
| Deposition thickness: | 10 nm |

The glass having the cured film thereon was evaluated for water repellency, slipperiness, stain wipe-off, and wear resistance by the following tests. It is noted that glass (Gorilla Glass by Corning) which had been coated on its outermost surface with SiO₂ of 10 nm thick, without any surface treatment, was similarly evaluated as Comparative Example 3.

### Evaluation of water repellency

Using a contact angle meter Drop Master DMo-701SA (Kyowa Interface Science Co., Ltd.), the cured film on glass was measured for contact angle with water as an index of water repellency (droplet 2 µL, temperature 25°C, relative humidity 40%). Measurement was carried out by dropping a water droplet, taking a picture of the steady droplet after 1 second by a CCD camera coupled to the contact angle meter, analyzing the droplet image by contact angle analysis software FAMAS built in the meter. The contact angle of the film on glass with water droplet was measured and computed by the θ/2 method. Analysis conditions are shown below. The results (initial contact angle with water) are shown in Table 1.

At the initial, the films of Examples and Comparative Examples all showed satisfactory water repellency.

### [Analysis conditions]

| | |
|---|---|
| Mode: | droplet mode (θ/2 method) |
| Deposition recognition: | automatic |
| Deposition recognition line (distance from needle): | 50 dots |
| Algorithm: | automatic |
| Image mode: | frame |
| Threshold level: | automatic |

### Evaluation of slipperiness

The cured film on glass was measured for a coefficient of dynamic friction relative to non-woven fabric by the following method as an index of slipperiness. The coefficient of dynamic friction of the cured film on glass relative to non-woven fabric was measured according to ASTM D1894, using a surface property tester Type 14FW (Shinto Scientific Co., Ltd.) under conditions: load 100 gf and pulling rate 500 mm/min. The results (coefficient of dynamic friction) are shown in Table 1.

### [Slipperiness evaluation conditions]

| | |
|---|---|
| Load: | 100 gf |
| Stroke: | 100 mm |
| Contact area: | 1×3 cm² |
| Non-woven fabric: | BEMCOT (Asahi Kasei Corp.) |

### Evaluation of stain wipe-off

The cured film on glass was examined by drawing a straight line of 2 cm wide with a marker (Hi! Mckee by Zebra Co., Ltd.), allowing the ink to dry, and wiping with tissue paper. The number of rubs taken until the ink was wiped off was counted and rated according to the following criteria. The results are shown in Table 1.

### [Stain wipe-off evaluation criteria]

| | |
|---|---|
| A: | up to 4 rubs |
| B: | 5 or more rubs |
| C: | ink could not be wiped off |

### Evaluation of wear resistance

The cured film on glass was examined by using a rubbing tester (Shinto Scientific Co., Ltd.), rubbing the film under the following conditions, and measuring a contact angle with water as above after every 500 rubs as an index of water repellency. The number of rubs repeated until the contact angle was below 80° was counted as an index of wear resistance. The test environment conditions included 25°C and relative humidity 40%. The results (contact angle with water after wear) are shown in Table 1.

### [Steel wool wear test conditions]

| | |
|---|---|
| Steel wool: | Bonster #0000 |
| Contact area: | 1 cm² |
| Travel distance (one way): | 40 mm |
| Travel speed: | 4,800 mm/min |
| Load: | 500 gf/cm² |

The number of rubs repeated until the contact angle was below 80° was counted.

The cured films of the surface treating agents of Examples 1 to 23 exhibit water repellency because the compound contains two hydrocarbon chains of specific carbon count at one end of its molecular chain and excellent slipperiness, stain wipe-off, and wear resistance due to improved molecular mobility. The cured film of the surface treating agent of Comparative Example 1 wherein the compound has only one hydrocarbon chain exhibits water repellency, but inferior stain wipe-off and wear resistance. The cured film of the surface treating agent of Comparative Example 2 wherein the compound has a fluorinated hydrocarbon chain exhibits high water repellency, but inferior durability. Comparative Example 3 is the glass substrate without the surface treating agent and lacks all properties because of no surface treatment, attesting the benefits of the invention. As described above, the surface treating agents within the scope of the invention form cured coatings that meet all of water repellency, slipperiness, stain wipe-off, and wear resistance after evaporative deposition.

**[Table 1]**

| | Water repellency (°) | Slipperiness | Stain wipe-off | Wear resistance (rubs) |
|---|---|---|---|---|
| Example 1 | 101 | 0.09 | A | 4,000 |
| Example 2 | 102 | 0.12 | A | 4,000 |
| Example 3 | 104 | 0.11 | A | 5,000 |
| Example 4 | 103 | 0.10 | A | 5,000 |
| Example 5 | 103 | 0.13 | A | 3,000 |
| Example 6 | 104 | 0.11 | A | 5,000 |
| Example 7 | 102 | 0.14 | A | 4,000 |
| Example 8 | 98 | 0.13 | A | 3,000 |
| Example 9 | 105 | 0.12 | A | 6,000 |
| Example 10 | 101 | 0.11 | A | 4,000 |
| Example 11 | 101 | 0.12 | A | 4,000 |
| Example 12 | 103 | 0.09 | A | 7,000 |
| Example 13 | 102 | 0.10 | A | 6,000 |
| Example 14 | 99 | 0.12 | A | 3,000 |
| Example 15 | 100 | 0.13 | A | 6,000 |
| Example 16 | 102 | 0.11 | A | 5,000 |
| Example 17 | 101 | 0.14 | A | 4,000 |
| Example 18 | 100 | 0.13 | A | 4,000 |
| Example 19 | 104 | 0.11 | A | 5,000 |
| Example 20 | 101 | 0.12 | A | 4,000 |
| Example 21 | 99 | 0.15 | A | 3,000 |
| Example 22 | 100 | 0.15 | A | 4,000 |
| Example 23 | 103 | 0.14 | A | 5,000 |
| Comparative Example 1 | 100 | 0.37 | B | 500 |
| Comparative Example 2 | 107 | 0.40 | A | 1,000 |
| Comparative Example 3 | 34 | 0.50 | C | - |

### [Example 24]

A surface treating agent was prepared by dissolving the compound of Synthesis Example 1 in dibutyl ether in a concentration of 0.1% by weight.

### [Example 25]

A surface treating agent was prepared by dissolving the compound of Synthesis Example 3 in toluene in a concentration of 0.1% by weight.

### [Example 26]

A surface treating agent was prepared by dissolving the compound of Synthesis Example 17 in isooctane in a concentration of 0.1% by weight.

### [Example 27]

A surface treating agent was prepared by dissolving the compound of Synthesis Example 22 in a *50*/*50* hexane/isooctane mixture in a concentration of 0.1% by weight.

### [Example 28]

A surface treating agent was prepared by dissolving the compound of Synthesis Example 30 in toluene in a concentration of 0.1% by weight.

### [Comparative Example 4]

A surface treating agent was prepared by dissolving the compound having formula (A') in dibutyl ether in a concentration of 0.1% by weight.

### [Comparative Example 5]

A surface treating agent was prepared by dissolving the compound having formula (B') in toluene in a concentration of 0.1% by weight.

### [Comparative Example 6]

No surface treating agent.

### Preparation of surface treating agent and formation of cured film

Surface treating agents were prepared as in the foregoing Examples and Comparative Examples. On glass (Gorilla Glass by Corning, tradename Gorilla III, size 100 mm × 50 mm × 0.7 mm), each surface treating agent was spray coated. The coating was cured in an atmosphere at 80°C and relative humidity 80% for 1 hour and further in an atmosphere at 25°C and relative humidity 50% for 12 hours, forming a cured film having a thickness of 3 to 5 nm.

The glass having the cured film thereon was evaluated for water repellency, slipperiness, stain wipe-off, and wear resistance by the following tests. It is noted that glass (Gorilla Glass by Coming) which had been coated on its outermost surface with SiO₂ of 10 nm thick, without any surface treatment, was similarly evaluated as Comparative Example 6.

### Evaluation of water repellency

Using a contact angle meter Drop Master DMo-701SA (Kyowa Interface Science Co., Ltd.), the cured film on glass was measured for contact angle with water as an index of water repellency (droplet 2 µL, temperature 25°C, relative humidity 40%). Measurement was carried out by dropping a water droplet, taking a picture of the steady droplet after 1 second by a CCD camera coupled to the contact angle meter, analyzing the droplet image by contact angle analysis software FAMAS built in the meter. The contact angle of the film on glass with water droplet was measured and computed by the θ/2 method. Analysis conditions are shown below. The results (initial contact angle with water) are shown in Table 2.

At the initial, the films of Examples and Comparative Examples all showed satisfactory water repellency.

### [Analysis conditions]

| | |
|---|---|
| Mode: | droplet mode (θ/2 method) |
| Deposition recognition: | automatic |
| Deposition recognition line (distance from needle): | 50 dots |
| Algorithm: | automatic |
| Image mode: | frame |
| Threshold level: | automatic |

### Evaluation of slipperiness

The cured film on glass was measured for a coefficient of dynamic friction relative to non-woven fabric by the following method as an index of slipperiness. The coefficient of dynamic friction of the cured film on glass relative to non-woven fabric was measured according to ASTM D1894, using a surface property tester Type 14FW (Shinto Scientific Co., Ltd.) under conditions: load 100 gf and pulling rate 500 mm/min. The results (coefficient of dynamic friction) are shown in Table 2.

### [Slipperiness evaluation conditions]

| | |
|---|---|
| Load: | 100 gf |
| Stroke: | 100 mm |
| Contact area: | 1×3 cm² |
| Non-woven fabric: | BEMCOT (Asahi Kasei Corp.) |

### Evaluation of stain wipe-off

The cured film on glass was examined by drawing a straight line of 2 cm wide with a marker (Hi! Mckee by Zebra Co., Ltd.), allowing the ink to dry, and wiping with tissue paper. The number of rubs taken until the ink was wiped off was counted and rated according to the following criteria. The results are shown in Table 2.

### [Stain wipe-off evaluation criteria]

| | |
|---|---|
| A: | up to 4 rubs |
| B: | 5 or more rubs |
| C: | ink could not be wiped off |

### Evaluation of wear resistance

The cured film on glass was examined by using a rubbing tester (Shinto Scientific Co., Ltd.), rubbing the film under the following conditions, and measuring a contact angle with water as above after every 500 rubs as an index of water repellency. The number of rubs repeated until the contact angle was below 80° was counted as an index of wear resistance. The test environment conditions included 25°C and relative humidity 40%. The results (contact angle with water after wear) are shown in Table 2.

### [Steel wool wear test conditions]

| | |
|---|---|
| Steel wool: | Bonster #0000 |
| Contact area: | 1 cm² |
| Travel distance (one way): | 40 mm |
| Travel speed: | 4,800 mm/min |
| Load: | 500 gf/cm² |

The number of rubs repeated until the contact angle was below 80° was counted.

The cured films of the surface treating agents of Examples 24 to 28 exhibit water repellency because the compound contains two hydrocarbon chains of specific carbon count at one end of its molecular chain and excellent slipperiness, stain wipe-off, and wear resistance due to improved molecular mobility. The cured film of the surface treating agent of Comparative Example 4 wherein the compound has only one hydrocarbon chain exhibits water repellency, but inferior stain wipe-off and wear resistance. The cured film of the surface treating agent of Comparative Example 5 wherein the compound has a fluorinated hydrocarbon chain exhibits high water repellency, but inferior durability. Comparative Example 6 is the glass substrate without the surface treating agent and lacks all properties because of no surface treatment, attesting the benefits of the invention. As described above, the surface treating agents within the scope of the invention form cured coatings that meet all of water repellency, slipperiness, stain wipe-off, and wear resistance after spray coating which is one example of wet coating.

**[Table 2]**

| | Water repellency (°) | Slipperiness | Stain wipe-off | Wear resistance (rubs) |
|---|---|---|---|---|
| Example 24 | 102 | 0.15 | A | 1,500 |
| Example 25 | 104 | 0.16 | A | 2,500 |
| Example 26 | 105 | 0.12 | A | 2,500 |
| Example 27 | 102 | 0.15 | A | 2,000 |
| Example 28 | 101 | 0.17 | A | 1,500 |
| Comparative Example 4 | 103 | 0.35 | B | 0 |
| Comparative Example 5 | 106 | 0.43 | A | 500 |
| Comparative Example 6 | 34 | 0.50 | C | - |

### [Example 29]

A surface treating agent was prepared by dissolving the compound of Synthesis Example 39 in ethylcyclohexane in a concentration of 10% by weight.

### [Example 30]

A surface treating agent was prepared by dissolving the compound of Synthesis Example 40 in dibutyl ether in a concentration of 10% by weight.

### [Example 31]

A surface treating agent was prepared by dissolving the compound of Synthesis Example 42 in toluene in a concentration of 20% by weight.

### [Example 32]

A surface treating agent was prepared by dissolving the compound of Synthesis Example 43 in butyl acetate in a concentration of 20% by weight.

### Preparation of surface treating agent and formation of cured film

Surface treating agents were prepared as in the foregoing Examples and Comparative Examples. On glass (Gorilla Glass by Corning, tradename Gorilla III, size 100 mm × 50 mm × 0.7 mm) which had been coated on its outermost surface with SiO₂ of 10 nm thick under the conditions shown below, each surface treating agent was deposited by a vacuum evaporation system (model VTR-350M by Ulvac Inc.) under conditions: pressure 2.0×10⁻² Pa and heating temperature 700°C. The coating was cured in an atmosphere at 80°C and relative humidity 80% for 1 hour and further in an atmosphere at 25°C and relative humidity 50% for 12 hours, forming a cured film having a thickness of 3 to 5 nm.

### [SiO₂ layer deposition conditions]

| | |
|---|---|
| Deposition system: | OTFC-1300 by Optorun Co., Ltd. |
| Depositing material: | SiO₂ |
| Depositing chamber pressure: | 0.015 Pa |
| Depositing rate: | 0.8 nm/s |
| Deposition thickness: | 10 nm |

The glass having the cured film thereon was evaluated for water repellency, slipperiness, stain wipe-off, and wear resistance by the following tests. It is noted that glass (Gorilla Glass by Coming) which had been coated on its outermost surface with SiO₂ of 10 nm thick, without any surface treatment, was similarly evaluated as Comparative Example 3.

### Evaluation of water repellency

Using a contact angle meter Drop Master DMo-701SA (Kyowa Interface Science Co., Ltd.), the cured film on glass was measured for contact angle with water as an index of water repellency (droplet 2 µL, temperature 25°C, relative humidity 40%). Measurement was carried out by dropping a water droplet, taking a picture of the steady droplet after 1 second by a CCD camera coupled to the contact angle meter, analyzing the droplet image by contact angle analysis software FAMAS built in the meter. The contact angle of the film on glass with water droplet was measured and computed by the θ/2 method. Analysis conditions are shown below. The results (initial contact angle with water) are shown in Table 3.

### [Analysis conditions]

| | |
|---|---|
| Mode: | droplet mode (θ/2 method) |
| Deposition recognition: | automatic |
| Deposition recognition line (distance from needle): | 50 dots |
| Algorithm: | automatic |
| Image mode: | frame |
| Threshold level: | automatic |

### Evaluation of slipperiness

The cured film on glass was measured for a coefficient of dynamic friction relative to non-woven fabric by the following method as an index of slipperiness. The coefficient of dynamic friction of the cured film on glass relative to non-woven fabric was measured according to ASTM D1894, using a surface property tester Type 14FW (Shinto Scientific Co., Ltd.) under conditions: load 100 gf and pulling rate 500 mm/min. The results (coefficient of dynamic friction) are shown in Table 3.

### [Slipperiness evaluation conditions]

| | |
|---|---|
| Load: | 100 gf |
| Stroke: | 100 mm |
| Contact area: | 1×3 cm² |
| Non-woven fabric: | BEMCOT (Asahi Kasei Corp.) |

### Evaluation of stain wipe-off

The cured film on glass was examined by drawing a straight line of 2 cm wide with a marker (Hi! Mckee by Zebra Co., Ltd.), allowing the ink to dry, and wiping with tissue paper. The number of rubs taken until the ink was wiped off was counted and rated according to the following criteria. The results are shown in Table 3.

### [Stain wipe-off evaluation criteria]

| | |
|---|---|
| A: | up to 4 rubs |
| B: | 5 or more rubs |
| C: | ink could not be wiped off |

### Evaluation of wear resistance

The cured film on glass was examined by using a rubbing tester (Shinto Scientific Co., Ltd.), rubbing the film under the following conditions, and measuring a contact angle with water as above after every 500 rubs as an index of water repellency. The number of rubs repeated until the contact angle was below 80° was counted as an index of wear resistance. The test environment conditions included 25°C and relative humidity 40%. The results (contact angle with water after wear) are shown in Table 3.

### [Steel wool wear test conditions]

| | |
|---|---|
| Steel wool: | Bonster #0000 |
| Contact area: | 1 cm² |
| Travel distance (one way): | 40 mm |
| Travel speed: | 4,800 mm/min |
| Load: | 500 gf/cm² |

The number of rubs repeated until the contact angle was below 80° was counted.

The cured films of the surface treating agents of Examples 29 to 32 exhibit water repellency because the compound contains two hydrocarbon chains of specific carbon count at one end of its molecular chain and excellent slipperiness, stain wipe-off, and wear resistance due to improved molecular mobility.

**[Table 3]**

| | Water repellency (°) | Slipperiness | Stain wipe-off | Wear resistance (rubs) |
|---|---|---|---|---|
| Example 29 | 104 | 0.09 | A | 7,000 |
| Example 30 | 100 | 0.10 | A | 6,000 |
| Example 31 | 98 | 0.14 | A | 3,000 |
| Example 32 | 99 | 0.11 | A | 4,000 |

### Evaluation of chemical resistance

The glass having the cured film formed thereon was immersed in alkaline solution in a vessel, which was held in a thermostat oven. The glass was taken out of the alkaline solution every 1 hour, washed over its entire surface with deionized water, and dried with dry air. The cured film on its area which had been immersed in the alkaline solution was measured for a contact angle with water as above, as an index of water repellency. While this operation was repeated, the immersion time until the contact angle with water became below 80° was determined. The results (chemical resistance in terms of the immersion time until the contact angle with water became below 80°) are shown in Table 4. The chemical resistance test conditions are shown below.

In the above chemical resistance test, satisfactory chemical resistance is given when the immersion time until the contact angle with water becomes below 80° is 2 hours or more, more preferably 4 hours or more, most preferably 5 hours or more.

### [Chemical resistance test conditions]

| | |
|---|---|
| Chemical: | 30 wt% sodium hydroxide aqueous solution |
| Temperature: | 55°C |

**[Table 4]**

| | Chemical resistance (hr) |
|---|---|
| Example 3 | 2 |
| Example 4 | 2 |
| Example 5 | 2 |
| Example 6 | 4 |
| Example 9 | 4 |
| Example 11 | 2 |
| Example 13 | 4 |
| Example 15 | 2 |
| Example 16 | 5 |
| Example 19 | 5 |
| Example 21 | 2 |
| Example 29 | 5 |
| Example 30 | 6 |
| Example 31 | 2 |
| Example 32 | 2 |
| Comparative Example 1 | 1 |
| Comparative Example 2 | 2 |
| Comparative Example 3 | 0 |

The cured films of the surface treating agents of Examples exhibit water repellency and chemical resistance because the compound contains two hydrocarbon chains of specific carbon count at one end of its molecular chain. Particularly, the compounds of Examples 6, 9, 13, 16, 19, 29 and 30 which have a long hydrocarbon chain of at least 13 carbon atoms at one end of their molecular chain, but do not contain an ester structure, urethane structure or -C(=O)N= structure are effective for preventing chemicals from penetrating into portions in close contact with substrates, marking a long durability time.

As viewed from different standpoints, the invention provides the following embodiments <1> to <3>.
<1> A hydrocarbon end group-containing compound having the general formula (1): 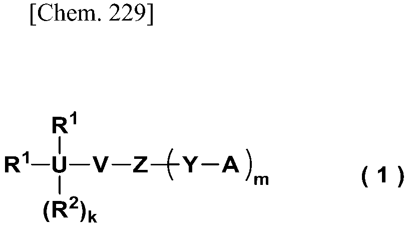 wherein R¹ is independently a straight, branched and/or cyclic C₃-C₃₂ monovalent hydrocarbon group which may contain at least one element selected from oxygen, sulfur, nitrogen and silicon, R² is hydrogen, halogen, hydroxy, siloxy, amino, thiol, monovalent hydrocarbon group of 1 or 2 carbon atoms, R¹ or -Y-A, U is carbon, silicon, nitrogen or tri- or tetravalent organic group, V is a single bond or a divalent hydrocarbon group which may contain at least one element selected from oxygen, nitrogen and sulfur, Z is a single bond, carbon, silicon, nitrogen, sulfur or a trivalent to octavalent organic group, Y is independently a divalent hydrocarbon group which may contain at least one element selected from oxygen, nitrogen, sulfur and silicon, A is independently a monovalent reactive group, k is 0 or 1, and m is an integer of 1 to 7, a cured film formed from a surface treating agent comprising the hydrocarbon end group-containing compound having chemical resistance.
<2> The hydrocarbon end group-containing compound of <1> wherein a cured film formed from a surface treating agent comprising the hydrocarbon end group-containing compound exhibits an immersion time of at least 2 hours when examined by a chemical resistance test under the following conditions.
[Chemical resistance test conditions]

| | |
|---|---|
| Chemical: | 30 wt% sodium hydroxide aqueous solution |
| Temperature: | 55°C |

Immersion time passed until the contact angle with water becomes below 80° is measured.
<3> The hydrocarbon end group-containing compound of <1>, which has the general formula (Z): 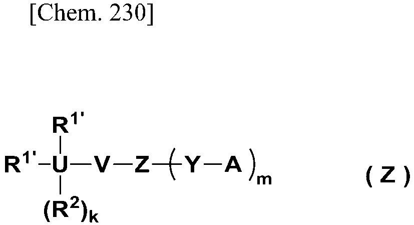 wherein R^{1'} is a straight, branched and/or cyclic, C₁₃-C₃₂ monovalent hydrocarbon group, R² is hydrogen, halogen, hydroxy, siloxy, amino, thiol, monovalent hydrocarbon group of 1 or 2 carbon atoms, R¹ or -Y-A, U is carbon, silicon, nitrogen or tri- or tetravalent organic group, V is a single bond or a divalent hydrocarbon group which may contain at least one element selected from oxygen, nitrogen and sulfur, Z is a single bond, carbon, silicon, nitrogen, sulfur or a trivalent to octavalent organic group, Y is independently a divalent hydrocarbon group which may contain at least one element selected from oxygen, nitrogen, sulfur and silicon, A is independently a monovalent reactive group, k is 0 or 1, and m is an integer of 1 to 7.

## Claims

1. A hydrocarbon end group-containing compound having the general formula (1): 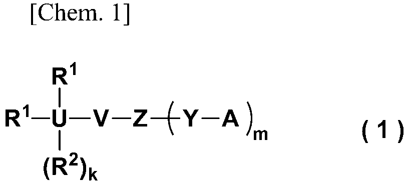 wherein R¹ is independently a straight, branched and/or cyclic C₃-C₃₂ monovalent hydrocarbon group which may contain at least one element selected from oxygen, sulfur, nitrogen and silicon, R² is hydrogen, halogen, hydroxy, siloxy, amino, thiol, monovalent hydrocarbon group of 1 or 2 carbon atoms, R¹ or -Y-A, U is carbon, silicon, nitrogen or tri- or tetravalent organic group, V is a single bond or a divalent hydrocarbon group which may contain at least one element selected from oxygen, nitrogen and sulfur, Z is a single bond, carbon, silicon, nitrogen, sulfur or a trivalent to octavalent organic group, Y is independently a divalent hydrocarbon group which may contain at least one element selected from oxygen, nitrogen, sulfur and silicon, A is independently a monovalent reactive group, k is 0 or 1, and m is an integer of 1 to 7.

2. The hydrocarbon end group-containing compound of claim 1 wherein A in formula (1) is a group having the general formula (2): 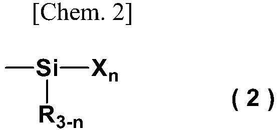
wherein R is independently a C₁-C₄ alkyl group or phenyl group, X is independently a hydroxy or hydrolyzable group, and n is an integer of 1 to 3, or the general formula (3): 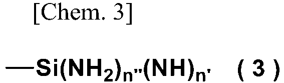
wherein n" is a number of 0 to 3, and n' is (3-n")/2.

3. The hydrocarbon end group-containing compound of claim 1 wherein R¹ in formula (1) is a group having any one of the following formulae: 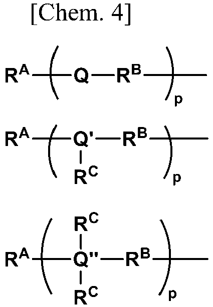 wherein R^{A} is independently a straight, branched and/or cyclic C₃-C₃₂ monovalent hydrocarbon group,
Q is independently a divalent group selected from the class consisting of oxygen, sulfur, C₆-C₈ divalent cyclic hydrocarbon group, diorganosilylene group, silalkylene structure or silarylene structure, a straight divalent organopolysiloxane residue of 2 to 10 silicon atoms, branched or cyclic divalent organopolysiloxane residue of 3 to 10 silicon atoms, carbonyl (or ketone) group, ester group, carbonate group, sulfinyl group, sulfonyl group, thioester group, thiocarbonate group, thiocarbamate group, amino group, amide group, carbamate group, urea group, and divalent nitrogen-containing heterocyclic group,
Q' is independently a trivalent group selected from the class consisting of nitrogen, C₆-C₈ trivalent cyclic hydrocarbon group, straight trivalent organopolysiloxane residue of 2 to 10 silicon atoms, branched or cyclic trivalent organopolysiloxane residue of 3 to 10 silicon atoms, trivalent amide group, and trivalent nitrogen-containing heterocyclic group,
Q" is independently a tetravalent group selected from the class consisting of carbon, silicon, C₆-C₈ tetravalent cyclic hydrocarbon group, straight tetravalent organopolysiloxane residue of 2 to 10 silicon atoms, and branched or cyclic tetravalent organopolysiloxane residue of 3 to 10 silicon atoms,
R⁸ is independently a single bond or a C₁-C₃₂ straight, branched or cyclic divalent hydrocarbon group,
R^{C} is independently R^{A} or hydrogen,
p is an integer of 0 to 10, and
the total number of carbon atoms in the structures is up to 32.

4. The hydrocarbon end group-containing compound of claim 1 wherein Y in formula (1) is a group selected from the class consisting of a C₁-C₂₀ alkylene group which may contain at least one element selected from oxygen, nitrogen and sulfur, a C₁-C₁₀ alkylene group containing a C₆-C₈ arylene moiety, a divalent group having C₁-C₈ alkylene groups bonded via a diorganosilylene moiety, silalkylene structure, silarylene structure or nitrogen-containing heterocyclic moiety, and a divalent group having a C₁-C₁₀ alkylene moiety bonded to the valence bond of a straight organopolysiloxane residue of 2 to 10 silicon atoms, or branched or cyclic organopolysiloxane residue of 3 to 10 silicon atoms.

5. The hydrocarbon end group-containing compound of claim 1 wherein Z in formula (1) is a single bond, or a trivalent to octavalent group selected from the class consisting of carbon, silicon, nitrogen, C₆-C₈ tri- or tetravalent cyclic hydrocarbon group, a trivalent group: -SiR³= wherein R³ is hydroxy, C₁-C₃ alkyl moiety or C₁-C₃ alkoxy moiety, a trivalent group: -CR⁴= wherein R⁴ is hydrogen, hydroxy, or C₁-C₃ alkyl moiety, a straight trivalent to octavalent organopolysiloxane residue of 2 to 10 silicon atoms, branched or cyclic trivalent to octavalent organopolysiloxane residue of 3 to 10 silicon atoms, trivalent amide group, trivalent carbamate group, tri- or tetravalent urea group, and trivalent to octavalent nitrogen-containing heterocyclic group.

6. The hydrocarbon end group-containing compound of claim 1 wherein U in formula (1) is a tri- or tetravalent group selected from the class consisting of carbon, silicon, nitrogen, a C₆-C₈ tri- or tetravalent cyclic hydrocarbon group, straight tri- or tetravalent organopolysiloxane residue of 2 to 10 silicon atoms, branched or cyclic tri- or tetravalent organopolysiloxane residue of 3 to 10 silicon atoms, trivalent amide group, trivalent carbamate group, tri- or tetravalent urea group, and tri- or tetravalent nitrogen-containing heterocyclic group.

7. The hydrocarbon end group-containing compound of claim 2 wherein X in formula (2) is selected from the class consisting of hydroxy, C₁-C₁₀ alkoxy group, C₂-C₁₀ alkoxyalkoxy group, C₁-C₁₀ acyloxy group, C₂-C₂₀ alkenyloxy group, halogen, and C₂-C₁₀ dialkylamino group.

8. A surface treating agent comprising the hydrocarbon end group-containing compound of any one of claims 1 to 7.

9. An article treated on its surface with the surface treating agent of claim 8.
